# EUROPEAN PATENT APPLICATION

(11) **EP 4 592 284 A1**
(43) Date of publication of application: **30.07.2025**
(21) Application number: 23868679.4
(22) Date of filing: 25.09.2023
(51) Int. Cl.: C07D 403/14, C07D 405/14, H10K 85/60, H10K 50/11

(54) **COMPOUND FOR ORGANIC OPTOELECTRONIC DEVICE, COMPOSITION FOR ORGANIC OPTOELECTRONIC DEVICE, ORGANIC OPTOELECTRONIC DEVICE AND DISPLAY DEVICE**

(30) Priority: 23.09.2022 KR 20220120904
(71) Applicant: SAMSUNG SDI CO., LTD., Yongin-si, Gyeonggi-do 17084 (KR)
(72) Inventor: JANG, Kipo, Suwon-si, Gyeonggi-do 16678 (KR); LEE, Byoungkwan, Suwon-si, Gyeonggi-do 16678 (KR); KANG, Dong Min, Suwon-si, Gyeonggi-do 16678 (KR); KWON, Jiyun, Suwon-si, Gyeonggi-do 16678 (KR); SEO, Hansol, Suwon-si, Gyeonggi-do 16678 (KR); SEO, Minseok, Suwon-si, Gyeonggi-do 16678 (KR); JO, Youngkyoung, Suwon-si, Gyeonggi-do 16678 (KR); KIM, Hyung Sun, Suwon-si, Gyeonggi-do 16678 (KR); JUNG, Ho Kuk, Suwon-si, Gyeonggi-do 16678 (KR); JUNG, Sunghyun, Suwon-si, Gyeonggi-do 16678 (KR)
(74) Representative: Michalski Hüttermann & Partner Patentanwälte mbB
(86) International application number: PCT/KR2023/014647
(87) International publication number: WO 2024/063628

(57) **Abstract**

Disclosed are a compound for an organic optoelectronic device represented by Chemical Formula 1, a composition for an organic optoelectronic device, an organic optoelectronic device, and a display device. The details of Chemical Formula 1 are as defined in the specification.

## Description

### [Technical Field]

A compound for an organic optoelectronic device, a composition for an organic optoelectronic device, an organic optoelectronic device, and a display device are disclosed.

### [Background Art]

An organic optoelectronic device (organic optoelectronic diode) is a device capable of converting electrical energy and optical energy to each other.

Organic optoelectronic devices may be largely divided into two types according to a principle of operation. One is a photoelectric device that generates electrical energy by separating excitons formed by light energy into electrons and holes, and transferring the electrons and holes to different electrodes, respectively and the other is light emitting device that generates light energy from electrical energy by supplying voltage or current to the electrodes.

Examples of the organic optoelectronic device include an organic photoelectric device, an organic light emitting diode, an organic solar cell, and an organic photoconductor drum.

Among them, organic light emitting diodes (OLEDs) are attracting much attention in recent years due to increasing demands for flat panel display devices. The organic light emitting diode is a device that converts electrical energy into light, and the performance of the organic light emitting diode is greatly influenced by an organic material between electrodes.

### [Disclosure]

### [Technical Problem]

An embodiment provides a compound for an organic optoelectronic device capable of realizing a high-efficiency and low-driving organic optoelectronic device.

Another embodiment provides a composition for an organic optoelectronic device including the compound for the organic optoelectronic device.

Another embodiment provides an organic optoelectronic device including the compound for the organic optoelectronic device or composition for the organic optoelectronic device.

Another embodiment provides a display device including the organic optoelectronic device.

### [Technical Solution]

According to an embodiment, a compound represented by Chemical Formula 1 is provided.

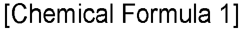

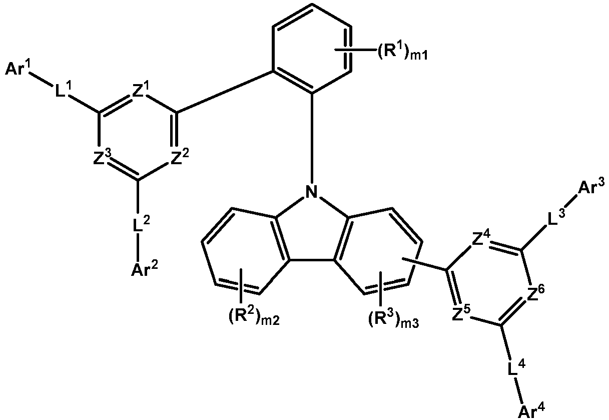

In Chemical Formula 1,
R¹ is hydrogen, deuterium, a substituted or unsubstituted C1 to C10 alkyl group, a substituted or unsubstituted C6 to C20 aryl group, or a substituted or unsubstituted C6 to C20 heterocyclic group,
Z¹ to Z³ are each independently N or CR^{a},
at least two of Z¹ to Z³ are N,
Z⁴ to Z⁶ are each independently N or CR^{b},
at least two of Z⁴ to Z⁶ are N,
R^{a}, R^{b}, R², and R³ are each independently hydrogen, deuterium, a cyano group, a halogen, a substituted or unsubstituted C1 to C30 alkyl group, a substituted or unsubstituted C6 to C30 aryl group, or a substituted or unsubstituted C2 to C30 heterocyclic group,
L¹ to L⁴ are each independently a single bond, a substituted or unsubstituted C6 to C20 arylene group, or a substituted or unsubstituted C2 to C20 heteroarylene group,
Ar¹ to Ar⁴ are each independently a substituted or unsubstituted C6 to C20 aryl group or a substituted or unsubstituted C2 to C30 heterocyclic group,
m1 and m2 are each independently one of integers of 1 to 4, and
m3 is one of integers of 1 to 3.

According to another embodiment, a composition for an organic optoelectronic device includes a first compound and a second compound.

The first compound may be the aforementioned compound for the organic optoelectronic device, and the second compound may be represented by Chemical Formula 2; a combination of Chemical Formula 3 and Chemical Formula 4; or Chemical Formula 5.

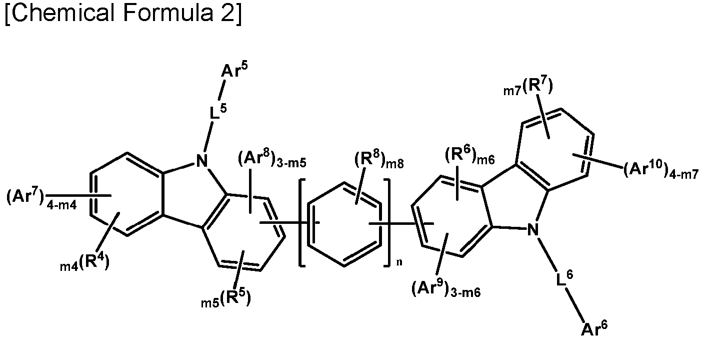

In Chemical Formula 2,
R⁴ to R⁸ and Ar⁷ to Ar¹⁰ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group,
L⁵ and L⁶ are each independently a single bond or a substituted or unsubstituted C6 to C20 arylene group,
m4, m7, and m8 are each independently one of integers of 1 to 4,
m5 and m6 are each independently one of integers of 1 to 3, and
n is one of integers of 0 to 2; 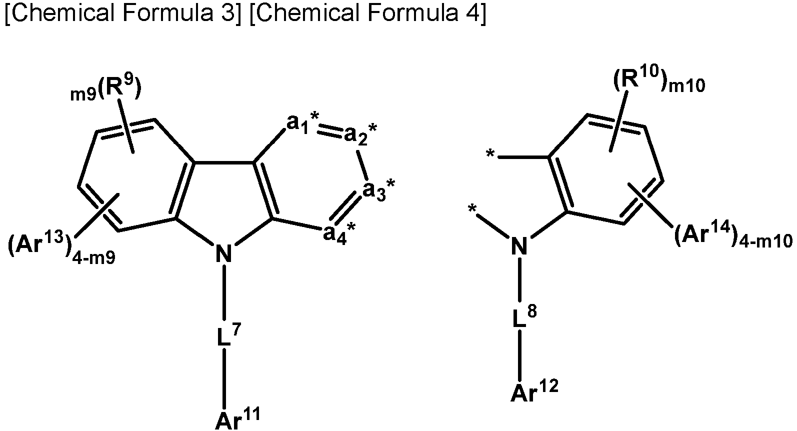
wherein, in Chemical Formula 3 and Chemical Formula 4,
a₁* to a₄* in Chemical Formula 3 are each independently a linking carbon (C) or C-L^{a}-R^{c},
among a₁* to a₄* in Chemical Formula 3, two adjacent ones are each linked to * in Chemical Formula 4,
L^{a}, L⁷, and L⁸ are each independently a single bond or a substituted or unsubstituted C6 to C20 arylene group,
R^{c}, Ar¹³, Ar¹⁴, R⁹, and R¹⁰ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar¹¹ and Ar¹² are each independently a substituted or unsubstituted C6 to C20 aryl group or a substituted or unsubstituted C2 to C30 heterocyclic group, and
m9 and m10 are each independently one of integers of 1 to 4; 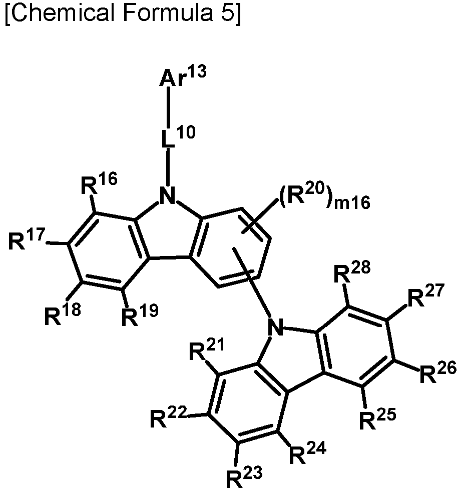
wherein, in Chemical Formula 5,
L¹⁰s are each independently a single bond or a substituted or unsubstituted C6 to C20 arylene group,
R¹⁶ to R²⁸ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar¹³ is a substituted or unsubstituted C6 to C20 aryl group or a substituted or unsubstituted C2 to C30 heterocyclic group, and
m16 is one of integers of 1 to 3.

According to another embodiment, an organic optoelectronic device includes an anode and a cathode facing each other, and at least one organic layer between the anode and the cathode, wherein the organic layer includes the aforementioned compound for the organic optoelectronic device or composition for the organic optoelectronic device.

According to another embodiment, a display device including the organic optoelectronic device is provided.

### [Advantageous Effects]

Low-driving and high-efficiency organic optoelectronic device may be realized.

### [Description of the Drawings]

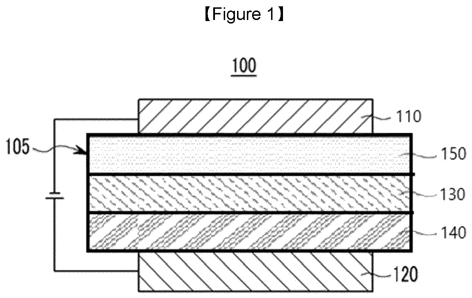

FIG. 1 is a cross-sectional view illustrating an organic light emitting diode according to an embodiment.

### <Description of Symbols>

100: organic light emitting diode
105: organic layer
110: cathode
120: anode
130: light emitting layer
140: hole transport region
150: electron transport region

### [Best Mode]

Hereinafter, embodiments of the present invention are described in detail. However, these embodiments are exemplary, and this disclosure is not limited thereto.

As used herein, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, or a cyano group. In specific example of the present invention, the "substituted" refers to replacement of at least one hydrogen of a substituent or a compound by deuterium, a C1 to C20 alkyl group, a C6 to C30 aryl group, or a cyano group. In specific example of the present invention, the "substituted" refers to replacement of at least one hydrogen of a substituent or a compound by deuterium, a C1 to C5 alkyl group, a C6 to C18 aryl group, or a cyano group. In specific example of the present invention, the "substituted" refers to replacement of at least one hydrogen of a substituent or a compound by deuterium, a cyano group, a methyl group, an ethyl group, a propyl group, a butyl group, a phenyl group, a biphenyl group, a terphenyl group, or a naphthyl group.

In the present specification, "unsubstituted" refers to non-replacement of a hydrogen atom by another substituent and remaining of the hydrogen atom.

In the present specification, "hydrogen substitution (-H)" may include "deuterium substitution (-D)" or "tritium substitution (-T)."

In the present specification, when a definition is not otherwise provided, "hetero" refers to one including one to three heteroatoms selected from N, O, S, P, and Si, and remaining carbons in one functional group.

In the present specification, "aryl group" refers to a group including at least one hydrocarbon aromatic moiety, and all elements of the hydrocarbon aromatic moiety have p-orbitals which form conjugation, for example a phenyl group, a naphthyl group, and the like, two or more hydrocarbon aromatic moieties may be linked by a sigma bond and may be, for example a biphenyl group, a terphenyl group, a quaterphenyl group, and the like, and two or more hydrocarbon aromatic moieties are fused directly or indirectly to provide a non-aromatic fused ring, for example a fluorenyl group.

The aryl group may include a monocyclic, polycyclic, or fused ring polycyclic (i.e., rings sharing adjacent pairs of carbon atoms) functional group.

As used herein, "heterocyclic group" is a generic concept of a heteroaryl group, and may include at least one heteroatom selected from N, O, S, P, and Si instead of carbon (C) in a cyclic compound such as aryl group, a cycloalkyl group, a fused ring thereof, or a combination thereof. When the heterocyclic group is a fused ring, the entire ring or each ring of the heterocyclic group may include one or more heteroatoms.

For example, "heteroaryl group" may refer to aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted benzophenanthrenyl group, a substituted or unsubstituted triphenylene group, a substituted or unsubstituted perylenyl group, a substituted or unsubstituted fluorenyl group, a substituted or unsubstituted indenyl group, or a combination thereof, but is not limited thereto.

More specifically, the substituted or unsubstituted C2 to C30 heterocyclic group may be a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, or a substituted or unsubstituted dibenzothiophenyl group, a substituted or unsubstituted benzonaphtofuranyl group, a substituted or unsubstituted benzonaphthothiophenyl group, a substituted or unsubstituted benzofuranofluorenyl group, a substituted or unsubstituted benzothiophenefluorenyl group, or a combination thereof, but is not limited thereto.

In the present specification, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to a lowest unoccupied molecular orbital (LUMO) level.

Hereinafter, a compound for an organic optoelectronic device according to an embodiment is described.

A compound for an organic optoelectronic device according to an embodiment is represented by Chemical Formula 1.

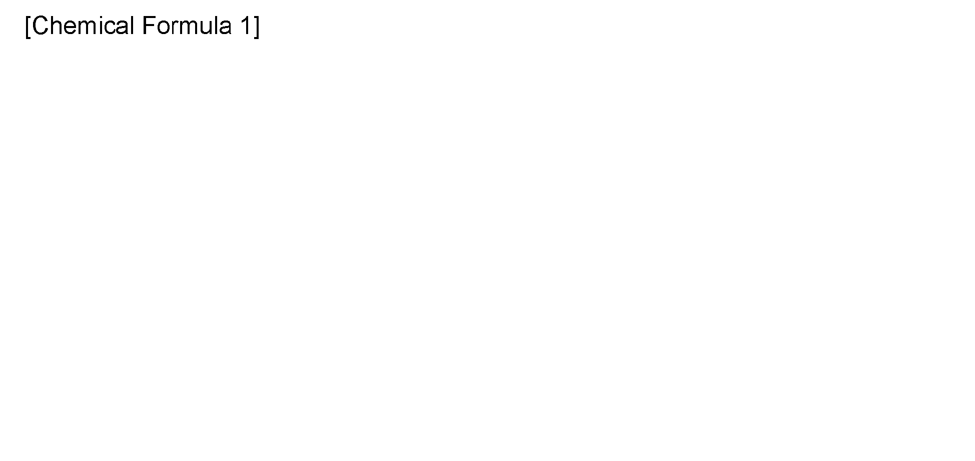

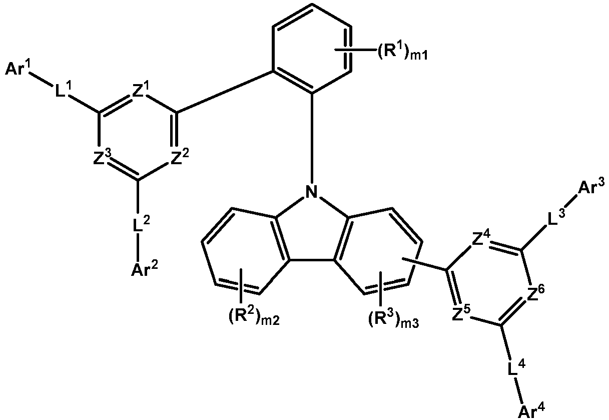

In Chemical Formula 1,
R¹ is hydrogen, deuterium, a substituted or unsubstituted C1 to C10 alkyl group, a substituted or unsubstituted C6 to C20 aryl group, or a substituted or unsubstituted C6 to C20 heterocyclic group,
Z¹ to Z³ are each independently N or CR^{a},
at least two of Z¹ to Z³ are N,
Z⁴ to Z⁶ are each independently N or CR^{b},
at least two of Z⁴ to Z⁶ are N,
R^{a}, R^{b}, R², and R³ are each independently hydrogen, deuterium, a cyano group, a halogen, a substituted or unsubstituted C1 to C30 alkyl group, a substituted or unsubstituted C6 to C30 aryl group, or a substituted or unsubstituted C2 to C30 heterocyclic group,
L ¹ to L⁴ are each independently a single bond, a substituted or unsubstituted C6 to C20 arylene group, or a substituted or unsubstituted C2 to C20 heteroarylene group,
Ar¹ to Ar⁴ are each independently a substituted or unsubstituted C6 to C20 aryl group or a substituted or unsubstituted C2 to C30 heterocyclic group,
m1 and m2 are each independently one of integers of 1 to 4, and
m3 is one of integers of 1 to 3.

The compound for an organic optoelectronic device represented by Chemical Formula 1 has a structure in which a nitrogen-containing 6-membered ring is simultaneously substituted in the N-direction (9-direction) of carbazole and the phenyl direction (1- to 8- directions) of carbazole with respect to the carbazole in the center.

The N-direction (9-direction) substituent of carbazole is linked through ortho-phenylene, and the luminescence efficiency of the device applied thereto may be improved by shallowly controlling a LUMO energy level.

In addition, as the nitrogen-containing 6-membered ring is additionally substituted in the phenyl direction of carbazole (1- to 8- directions), the electron mobility may be increased, and thus the operating voltage of the device to which it is applied may be lowered.

That is, a device to which the compound for organic optoelectronic devices represented by Chemical Formula 1 is applied can simultaneously implement low-driving and high-efficiency characteristics.

For example, R¹ may be a substituent other than an electron withdrawing group,

For example, R¹ may be hydrogen, deuterium, a substituted or unsubstituted C1 to C6 alkyl group, or a substituted or unsubstituted C6 to C12 aryl group.

The electron withdrawing group may be, for example, a halogen atom such as F, Cl, I, Br, and a halogenated alkyl compound composed of these atoms, a cyano group, and the like.

When R¹ is an electron withdrawing group, the LUMO energy level cannot be shallowly controlled, and in this case, the desired effect cannot be obtained.

In a specific embodiment, R¹ may be hydrogen, deuterium, or a substituted or unsubstituted C1 to C5 alkyl group.

For example, R² and R³ may each independently be hydrogen, deuterium, a substituted or unsubstituted C1 to C10 alkyl group, a substituted or unsubstituted C6 to C12 aryl group, or a substituted or unsubstituted C2 to C20 heterocyclic group.

As a specific example, R² and R³ may each independently be hydrogen, deuterium, a substituted or unsubstituted C1 to C5 alkyl group, or a substituted or unsubstituted C6 to C12 aryl group.

For example, R² and R³ may each independently be hydrogen, deuterium, a substituted or unsubstituted C1 to C5 alkyl group, a substituted or unsubstituted phenyl group, a substituted or unsubstituted biphenyl group, or a substituted or unsubstituted naphthyl group.

For example, L¹ to L⁴ may each independently be a single bond, or a substituted or unsubstituted C6 to C12 arylene group.

As a specific example, L¹ to L⁴ may each independently be a single bond, a substituted or unsubstituted phenylene group, or a substituted or unsubstituted biphenylene group.

For example, Ar¹ to Ar⁴ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylene group, a substituted or unsubstituted fluorenyl group, a substituted or unsubstituted dibenzofuranyl group, a substituted or unsubstituted dibenzothiophenyl group, or a substituted or unsubstituted dibenzosilolyl group.

As a specific example, Ar¹ to Ar⁴ may each independently be a substituted or unsubstituted phenyl group, a substituted or unsubstituted biphenyl group, a substituted or unsubstituted fluorenyl group, a substituted or unsubstituted dibenzofuranyl group, or a substituted or unsubstituted dibenzothiophenyl group.

For example, Chemical Formula 1 may be represented by any one of Chemical Formula 1A to Chemical Formula 1D depending on the specific substitution position of the nitrogen-containing 6-membered ring substituted in the phenyl direction of carbazole.

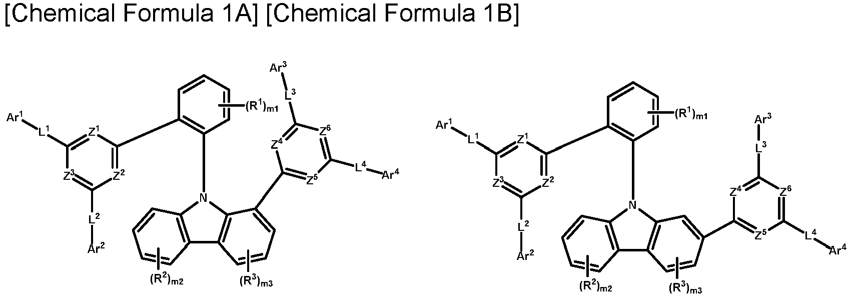

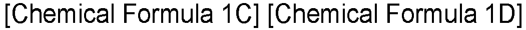

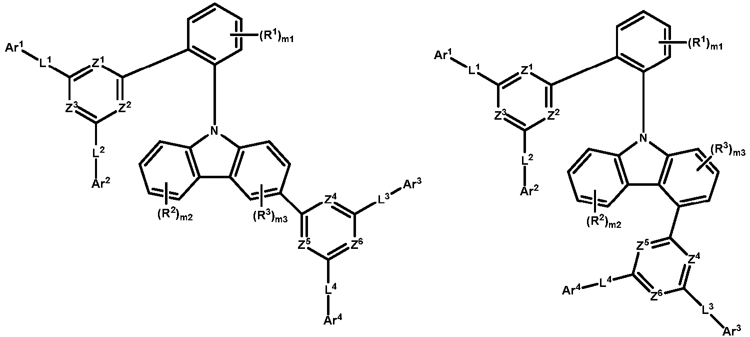

In Chemical Formula 1A to Chemical Formula 1D,
Z¹ to Z⁶, R¹ to R³, L¹ to L⁴, Ar¹ to Ar⁴, and m1 to m3 are the same as described above.

As a specific example, Chemical Formula 1 may be represented by any one of Chemical Formula 1B or Chemical Formula 1D.

For example, Chemical Formula 1 may be represented by any one of Chemical Formula 1-1 to Chemical Formula 1-9.

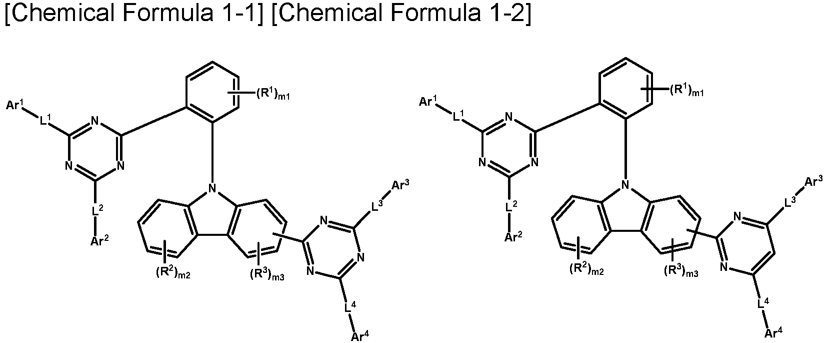

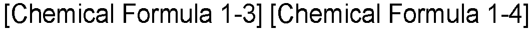

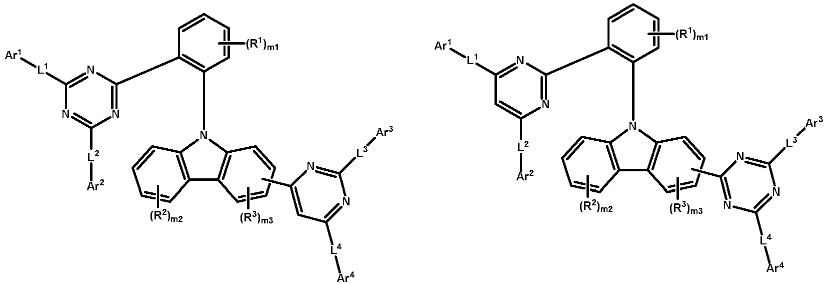

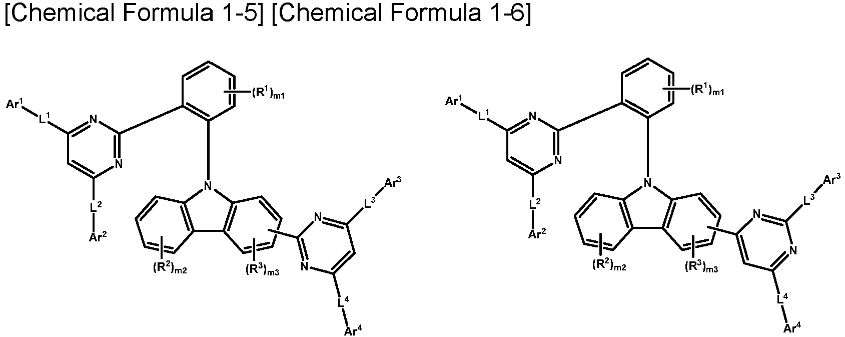

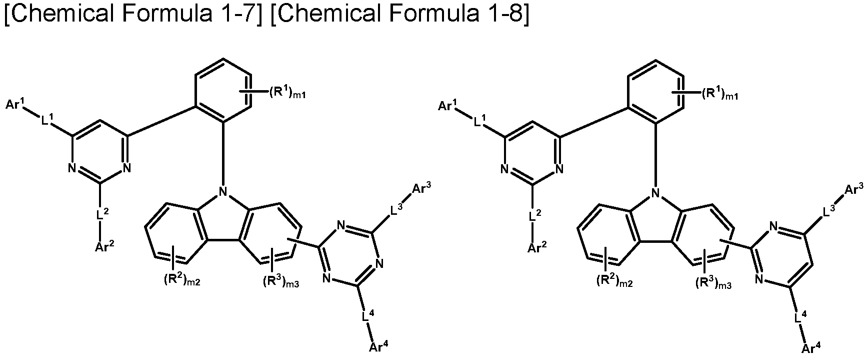

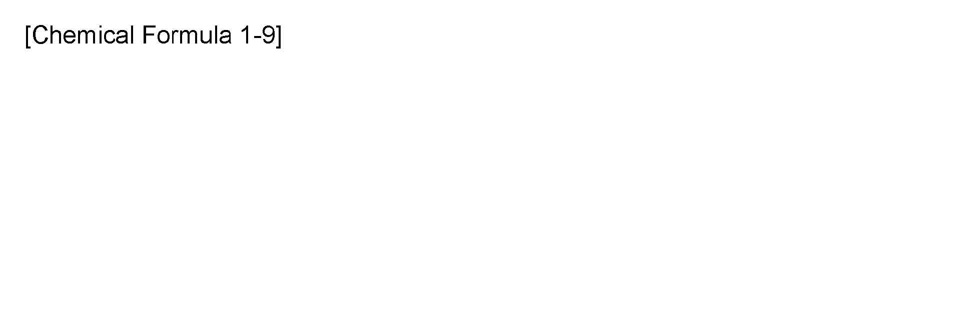

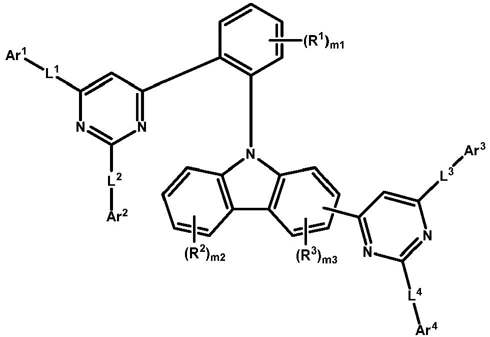

In Chemical Formula 1-1 to Chemical Formula 1-9,
R¹ to R³, L¹ to L⁴, Ar¹ to Ar⁴, and m1 to m3 are the same as described above.

As a specific example, Chemical Formula 1 may be represented by Chemical Formula 1-1.

As a more specific example, Chemical Formula 1 may be represented by Chemical Formula 1-B-1 or Chemical Formula 1-D-1.

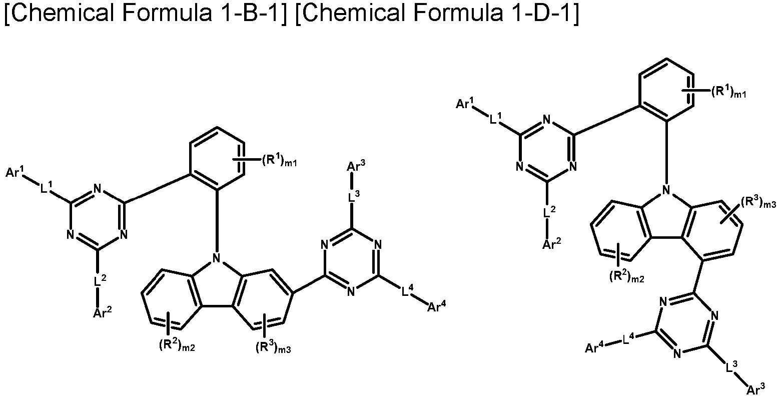

In Chemical Formula 1-B-1 and Chemical Formula 1-D-1,
R¹ to R³, L¹ to L⁴, Ar¹ to Ar⁴, and m1 to m3 are the same as described above.

For example, the compound for the organic optoelectronic device represented by Chemical Formula 1 may be one selected from the compounds listed in Group 1, but is not limited thereto.

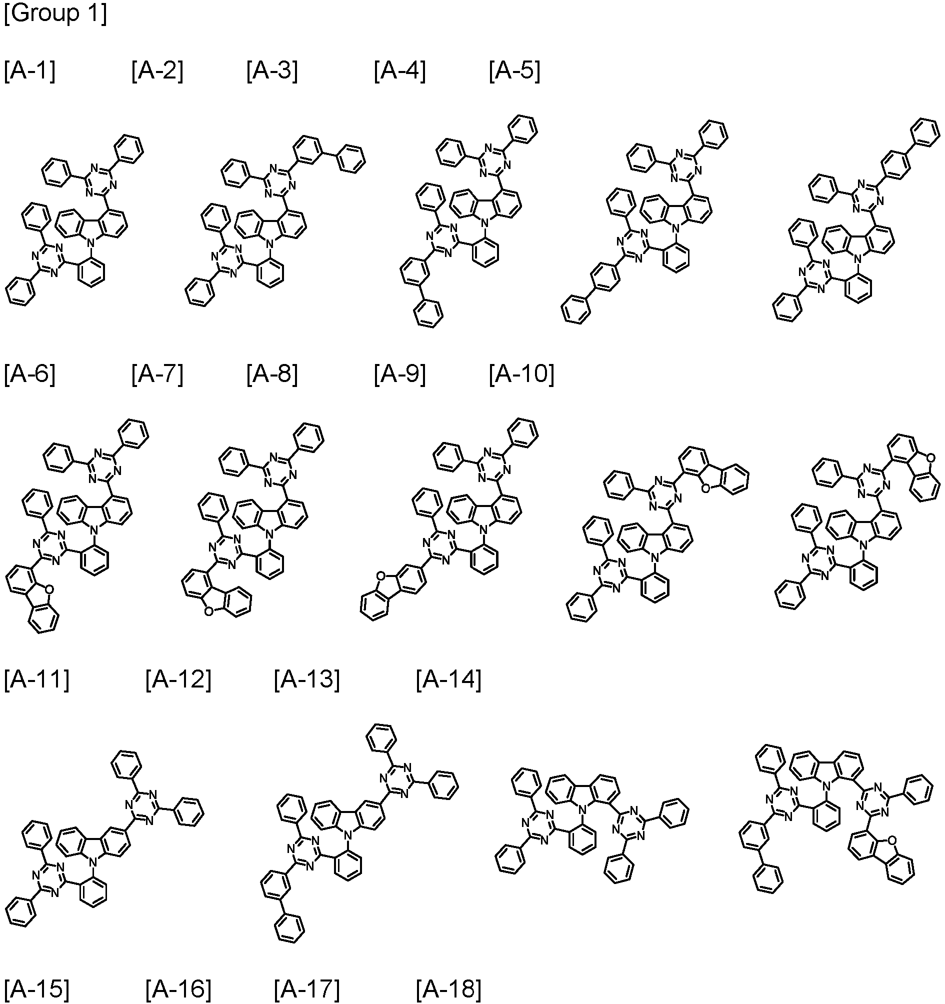

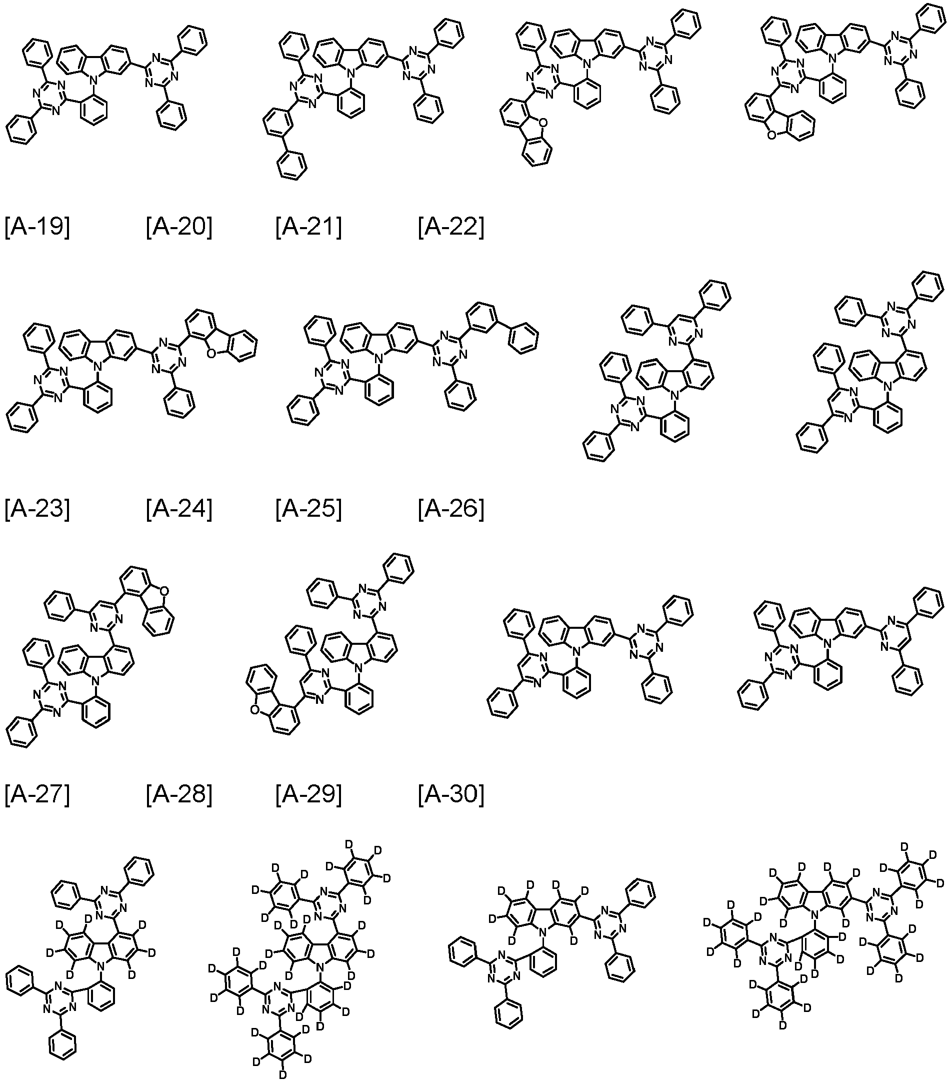

A composition for an organic optoelectronic device according to another embodiment includes a first compound and a second compound, wherein the first compound may be the aforementioned compound for an organic optoelectronic device, and the second compound may be represented by Chemical Formula 2; a combination of Chemical Formula 3 and Chemical Formula 4; or Chemical Formula 5.

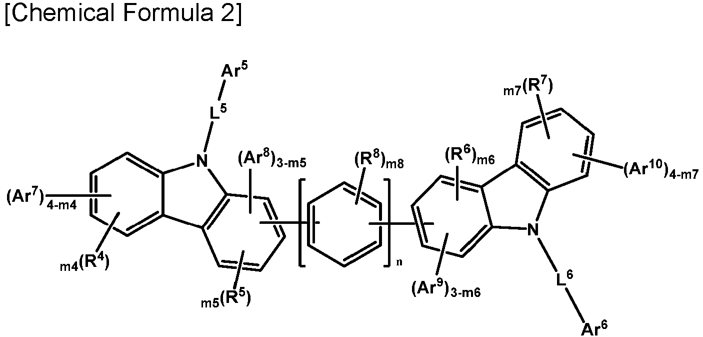

In Chemical Formula 2,
R⁴ to R⁸ and Ar⁷ to Ar¹⁰ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group,
L⁵ and L⁶ are each independently a single bond or a substituted or unsubstituted C6 to C20 arylene group,
m4, m7, and m8 are each independently one of integers of 1 to 4,
m5 and m6 are each independently one of integers of 1 to 3, and
n is an integer of 0 to 2.

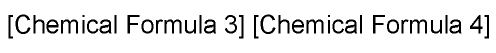

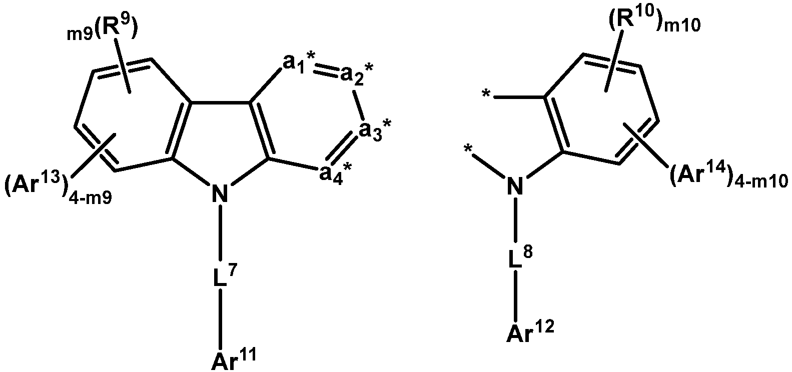

In Chemical Formula 3 and Chemical Formula 4,
a₁* to a₄* in Chemical Formula 3 are each independently a linking carbon (C) or C-L^{a}-R^{c},
among a₁* to a₄* in Chemical Formula 3, two adjacent ones are each linked to * in Chemical Formula 4,
L^{a}, L⁷, and L⁸ are each independently a single bond or a substituted or unsubstituted C6 to C20 arylene group,
R^{c}, Ar¹³, Ar¹⁴, R⁹, and R¹⁰ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar¹¹ and Ar¹² are each independently a substituted or unsubstituted C6 to C20 aryl group or a substituted or unsubstituted C2 to C30 heterocyclic group, and
m9 and m10 are each independently one of integers of 1 to 4.

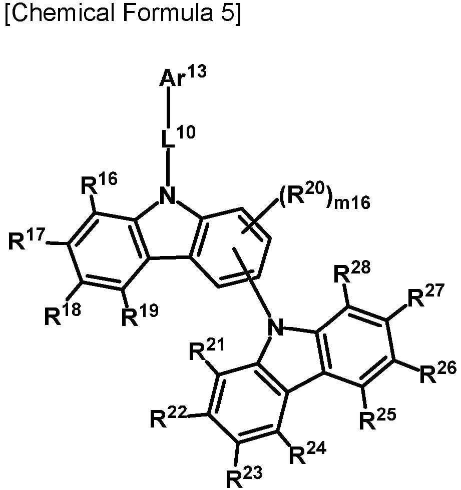

In Chemical Formula 5,
L¹⁰s are each independently a single bond or a substituted or unsubstituted C6 to C20 arylene group,
R¹⁶ to R²⁸ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar¹³ is a substituted or unsubstituted C6 to C20 aryl group or a substituted or unsubstituted C2 to C30 heterocyclic group, and
m16 is one of integers of 1 to 3.

The second compound can be used in the light emitting layer together with the first compound to improve luminous efficiency and life-span characteristics by increasing charge mobility and stability.

For example, in Chemical Formula 2, Ar⁵ and Ar⁶ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group,
in Chemical Formula 2, L⁵ and L⁶ may each independently be a single bond, a substituted or unsubstituted phenylene group, or a substituted or unsubstituted biphenylene group,
in Chemical Formula 2, R⁴ to R⁸ and Ar⁷ to Ar¹⁰ may each independently be hydrogen, deuterium, or a substituted or unsubstituted C6 to C12 aryl group, and
n may be 0 or 1.

As an example, in Chemical Formula 2, "substituted" refers to replacement of at least one hydrogen by deuterium, a C1 to C4 alkyl group, a C6 to C18 aryl group, or a C2 to C30 heteroaryl group.

For example, in Chemical Formula 2, Ar⁵ and Ar⁶ may each independently be a substituted or unsubstituted carbazolyl group, a substituted or unsubstituted dibenzothiophenyl group, a substituted or unsubstituted dibenzofuranyl group, or a substituted or unsubstituted fluorenyl group.

In a specific embodiment of the present invention, Chemical Formula 2 may be represented by one of Chemical Formula 2-1 to Chemical Formula 2-15.

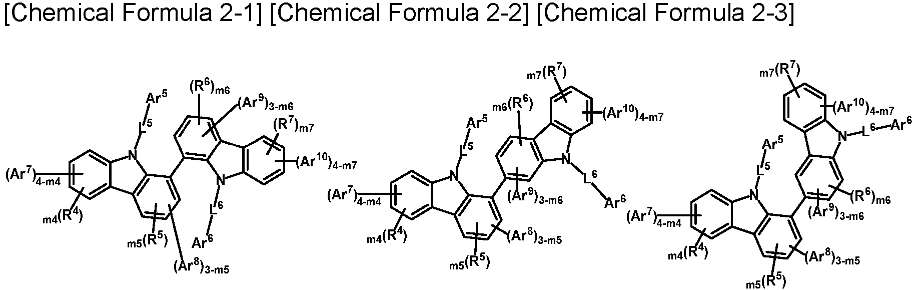

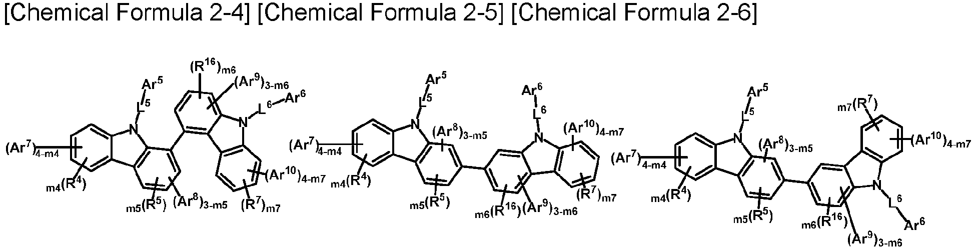

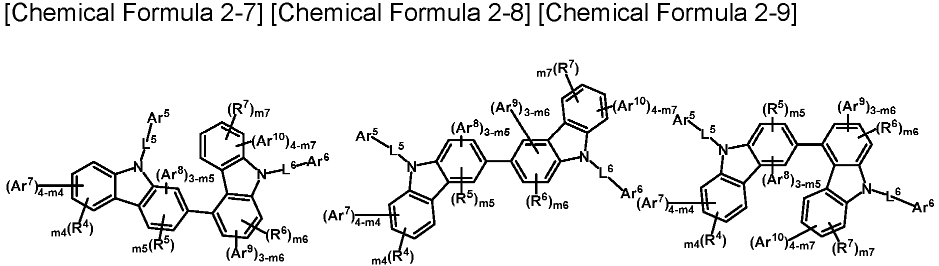

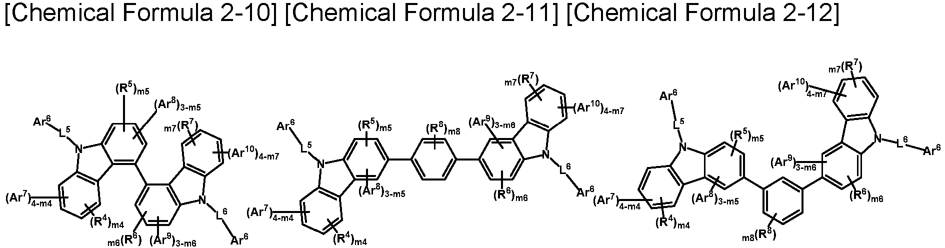

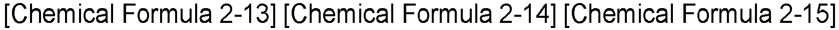

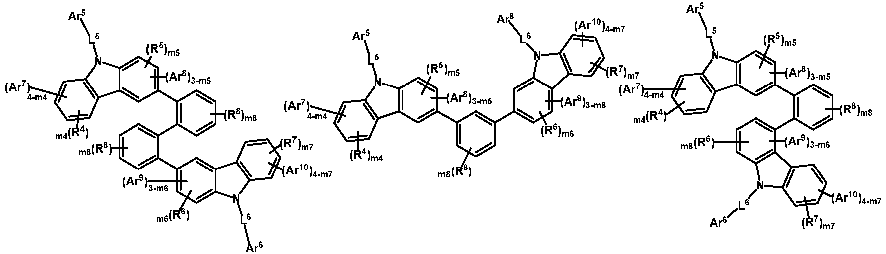

In Chemical Formula 2-1 to Chemical Formula 2-15, R⁴ to R⁸ and Ar⁷ to Ar¹⁰ may each independently be hydrogen, deuterium or a substituted or unsubstituted C6 to C12 aryl group, and *-L⁵-Ar⁵ and *-L⁶-Ar⁶ may each independently be one of the substituents listed in Group I.

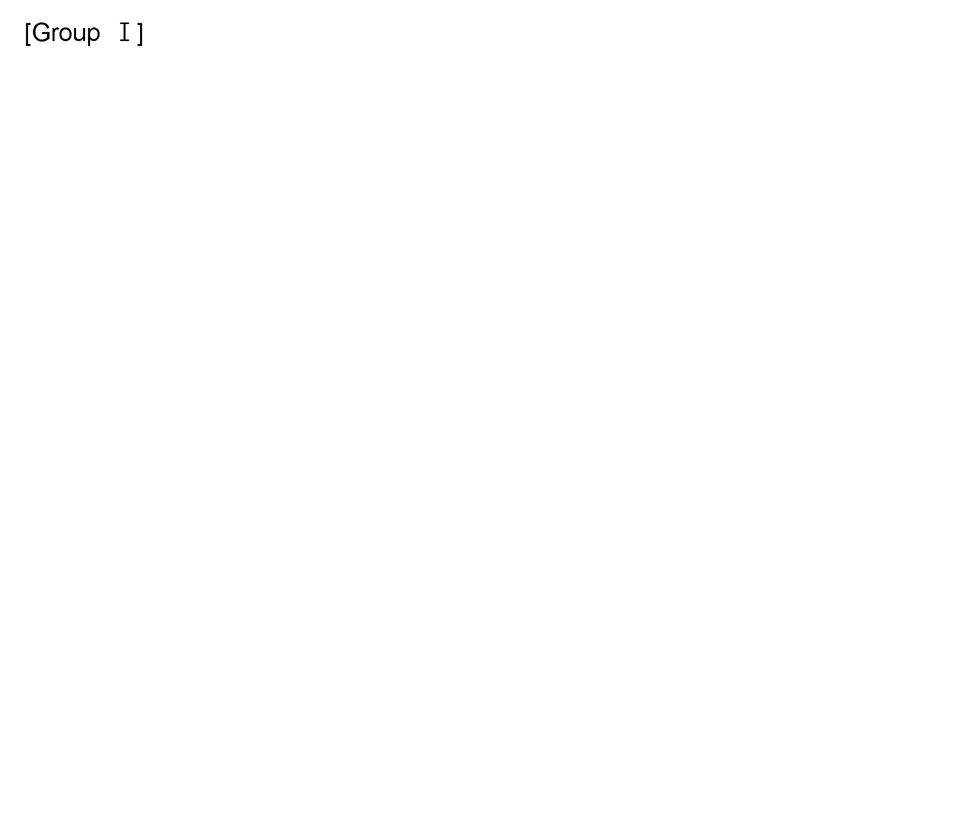

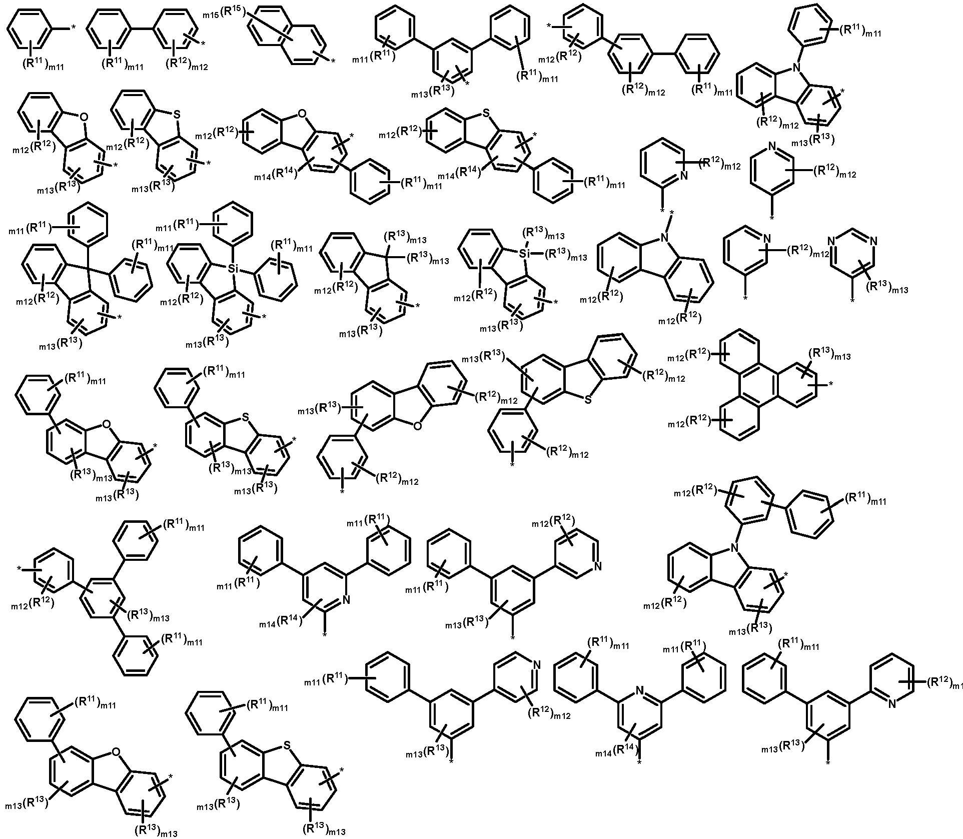

In Group I ,
R¹¹ to R¹⁵ are each independently hydrogen, deuterium, a cyano group, a C1 to C10 alkyl group, or a C6 to C12 aryl group,
m11 is one of integers of 1 to 5,
m12 is one of integers of 1 to 4,
m13 is one of integers of 1 to 3,
m14 is an integer of 1 or 2,
m15 is one of integers of 1 to 7, and
* is a linking point.

In a specific embodiment of the present invention, the combination of Chemical Formula 3 and Chemical Formula 4 may be represented by, for example, any one of Chemical Formula 3A, Chemical Formula 3B, Chemical Formula 3C, Chemical Formula 3D, and Chemical Formula 3E.

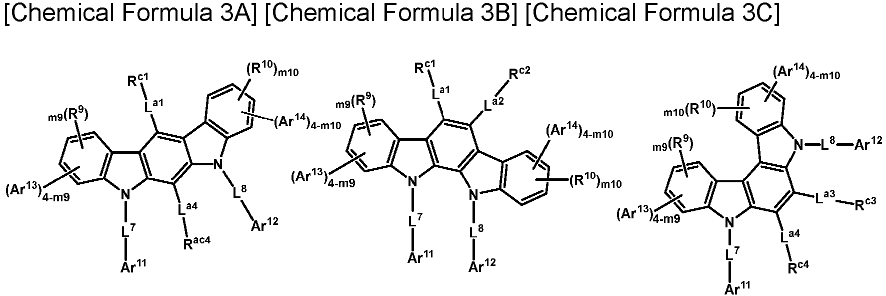

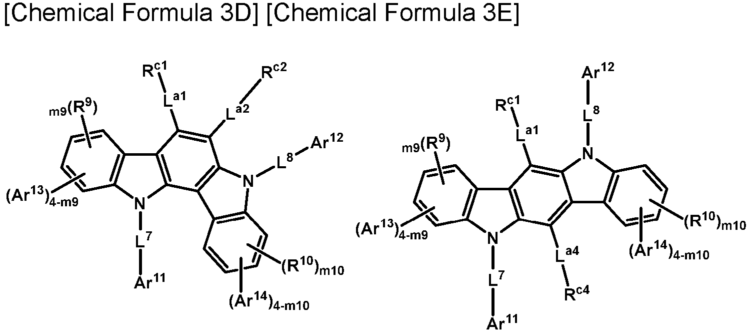

In Chemical Formula 3A to Chemical Formula 3E, Ar¹¹, Ar¹², L⁷, L⁸, Ar¹³, Ar¹⁴, R⁹, and R¹⁰ are the same as described above,
L^{a1} to L^{a4} are defined as L⁷ and L⁸ described above, and
R^{c1} to R^{c4} are as defined for Ar¹³, Ar¹⁴, R⁹, and R¹⁰ described above.

For example, in Chemical Formulas 3 and 4, Ar¹¹ and Ar¹² may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group, and

R^{c1} to R^{c4}, Ar¹³, Ar¹⁴, R⁹, and R¹⁰ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In a specific embodiment of the present invention, in Chemical Formula 3 and 4, *-L⁷-Ar¹¹ and *-L⁸-Ar¹² may each independently be selected from the substituents listed in Group I .

In an embodiment, R^{c1} to R^{c4}, Ar¹³, Ar¹⁴, R⁹, and R¹⁰ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, R^{c1} to R^{c4}, Ar¹³, Ar¹⁴, R⁹, and R¹⁰ may each independently be hydrogen, deuterium, a cyano group, or a substituted or unsubstituted phenyl group, and
in a specific embodiment, R^{c1} to R^{c4}, Ar¹³, Ar¹⁴, R⁹, and R¹⁰ may each independently be hydrogen, deuterium, or a substituted or unsubstituted phenyl group.

In a specific embodiment of the present invention, Chemical Formula 5 may be represented by, for example, any one of Chemical Formula 5-1 to Chemical Formula 5-4.

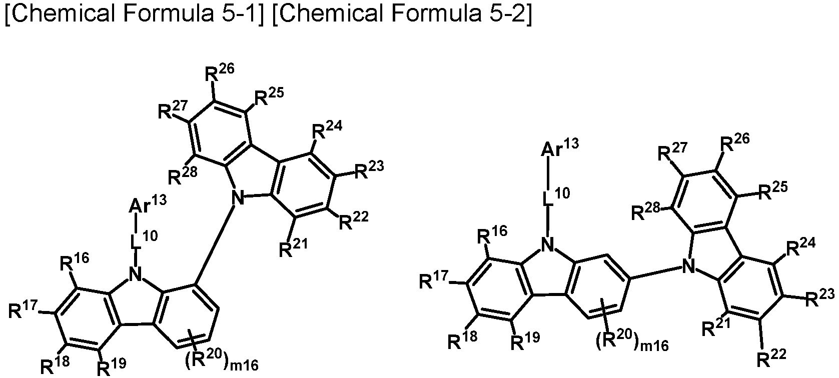

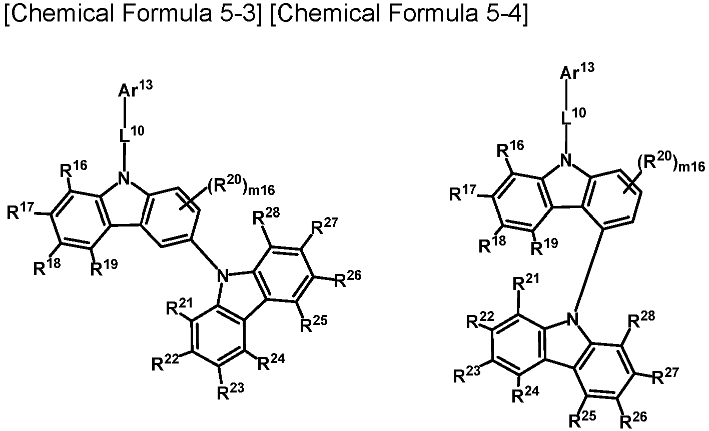

In Chemical Formula 5-1 to Chemical Formula 5-4,
L¹⁰, R¹⁶ to R²⁸, Ar¹³, and m16 are the same as described above.

For example, in Chemical Formula 5, Ar¹³ may be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group, and

R¹⁶ to R²⁸ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In a specific embodiment of the present invention, the second compound may be represented by Chemical Formula 2-8, wherein in Chemical Formula 2-8, Ar⁵ and Ar⁶ may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, L⁵ and L⁶ may each independently be a single bond, or a substituted or unsubstituted C6 to C20 arylene group, and Ar⁷ to Ar¹⁰ and R⁴ to R⁷ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, in Chemical Formula 2-8, Ar⁷ to Ar¹⁰ and R⁴ to R⁷ may each independently be hydrogen, deuterium or a substituted or unsubstituted C6 to C12 aryl group, and *-L⁵-Ar⁵ and *-L⁶-Ar⁶ may each independently be selected from the substituents listed in Group I .

In a specific embodiment of the present invention, the second compound may be represented by Chemical Formula 3C, wherein, in Chemical Formula Chemical Formula 3C, L^{a3} and L^{a4} may be a single bond, L⁷ and L⁸ may each independently be a single bond or a substituted or unsubstituted C6 to C12 arylene group, Ar¹³, Ar¹⁴, R⁹, R¹⁰, R^{c3}, and R^{c4} may each be hydrogen, deuterium or phenyl group, and Ar¹¹ and Ar¹² may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, in Chemical Formula 3C, L^{c3} and L^{c4} may be a single bond, Ar¹³, Ar¹⁴, R⁹, R¹⁰, R^{c3}, and R^{c4} may each independently be hydrogen, deuterium or a C6 to C12 aryl group, and *-L⁷-Ar¹¹ and *-L⁸-Ar¹² may each independently be one of the substituents listed in Group I .

In another specific embodiment of the present invention, the second compound may be represented by Chemical Formula 5-2 or Chemical Formula 5-3, and L¹⁰-Ar¹³ may be selected from the substituents listed in Group I.

For example, R¹⁶ to R²⁸ may each independently be hydrogen, deuterium, a cyano group, or a substituted or unsubstituted phenyl group.

For example, the compound for the second organic optoelectronic device may be one selected from the compounds listed in Group 2, but is not limited thereto.

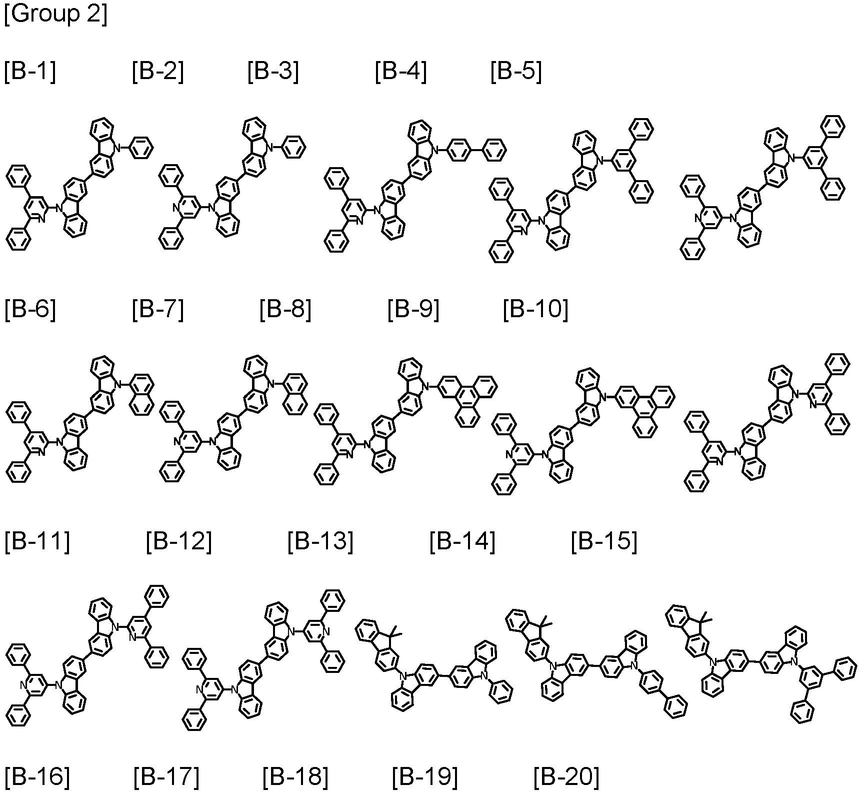

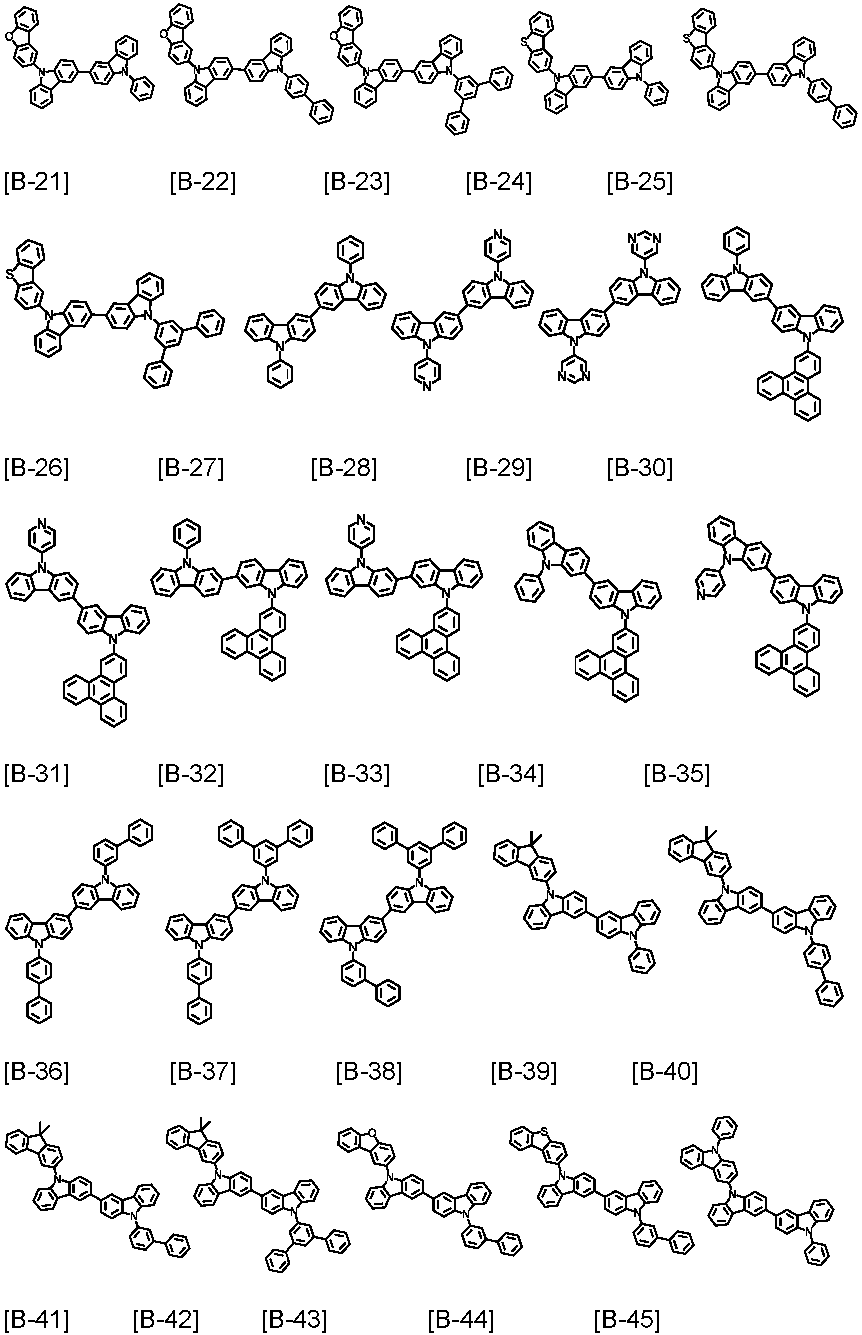

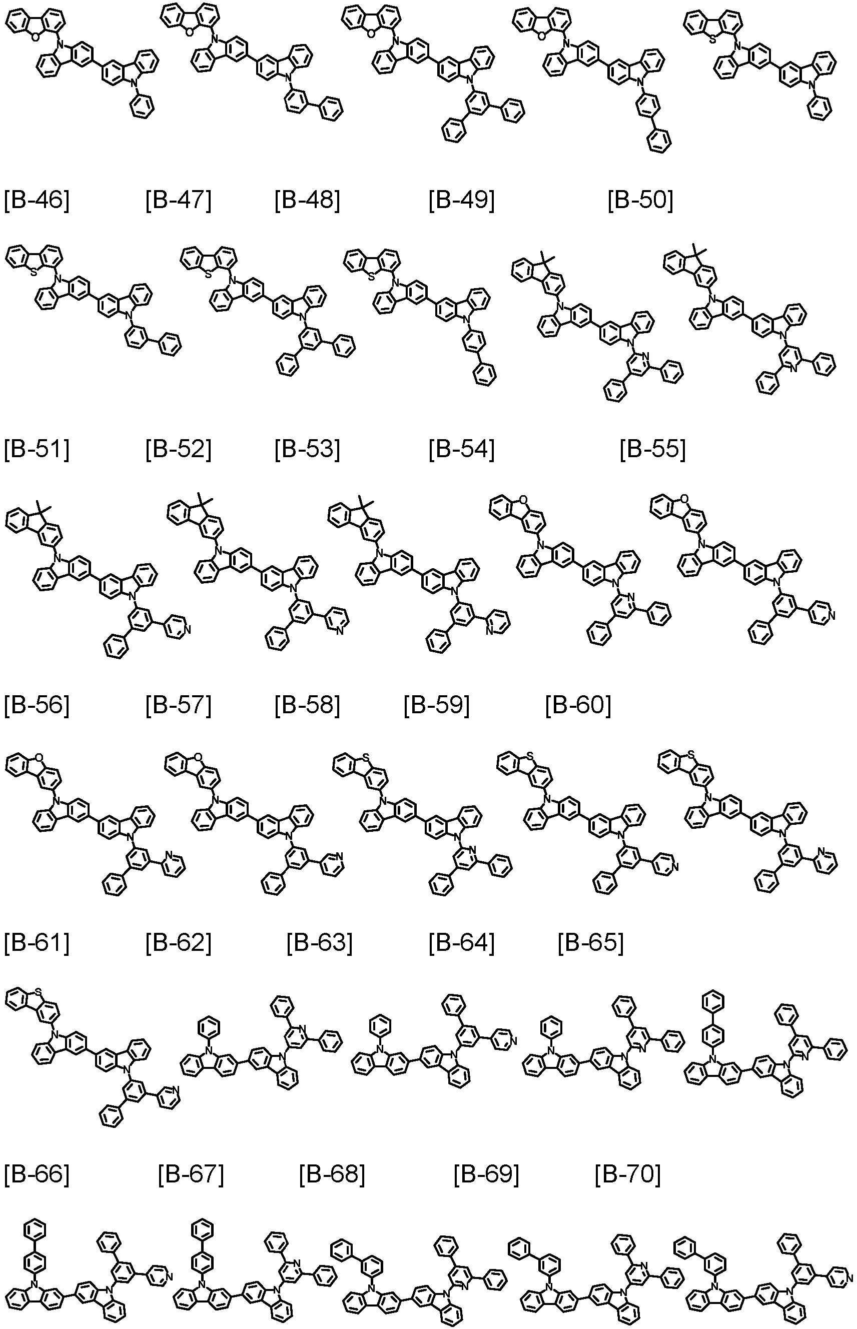

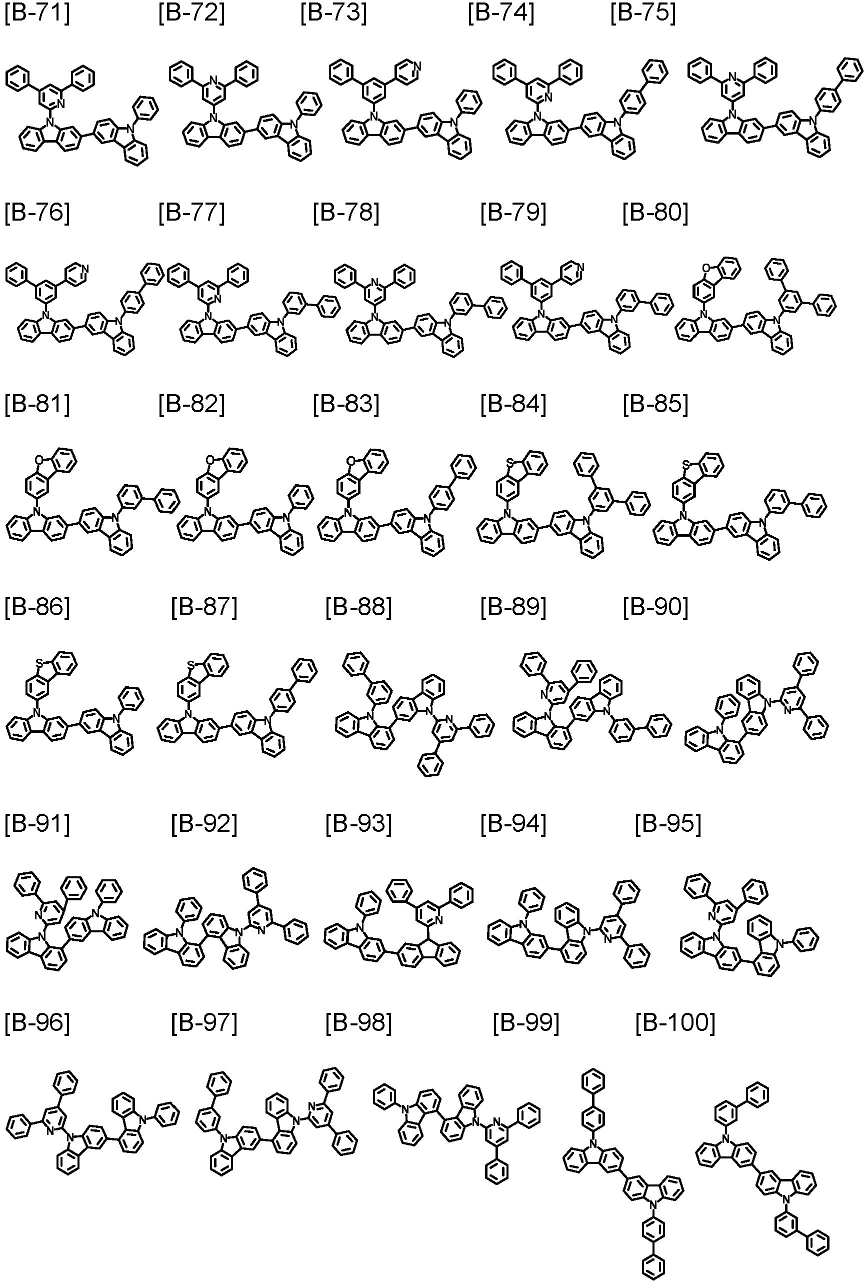

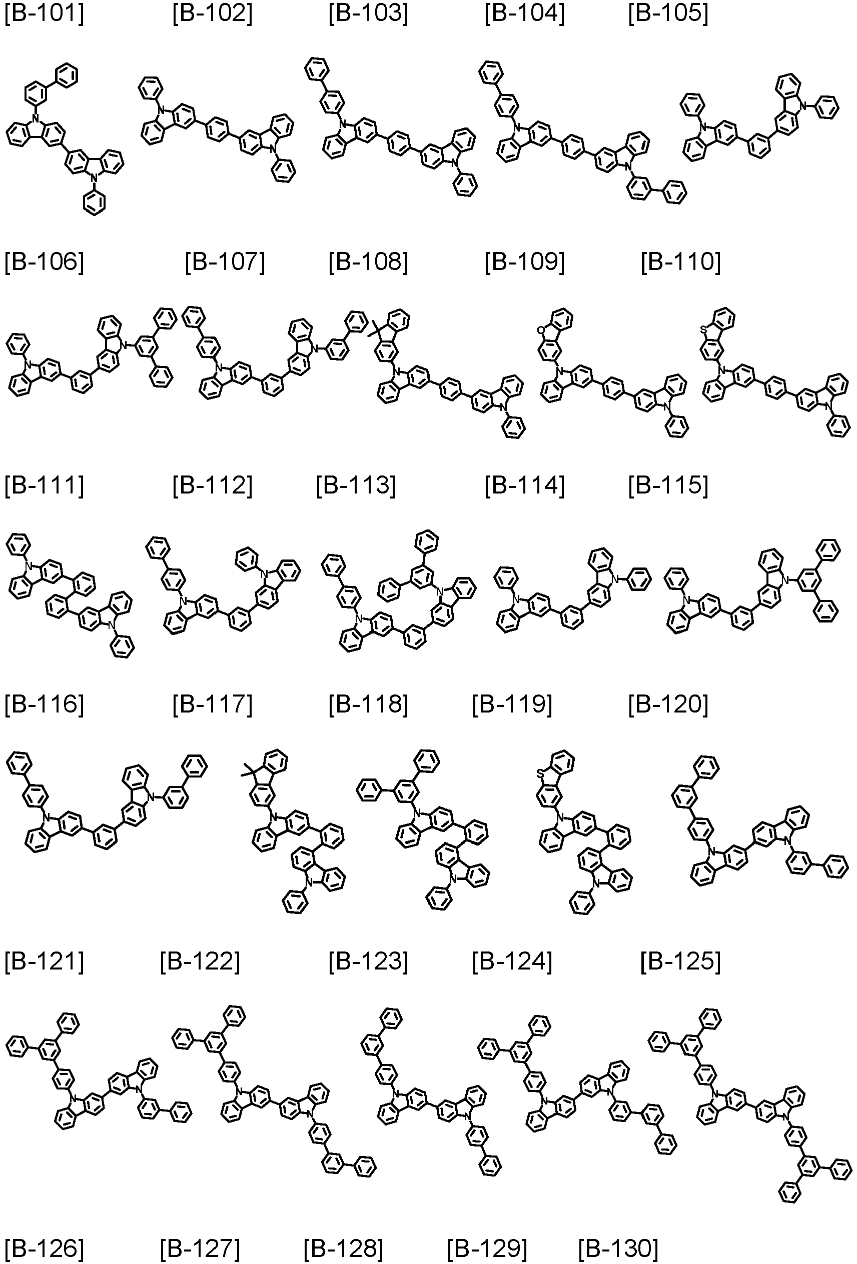

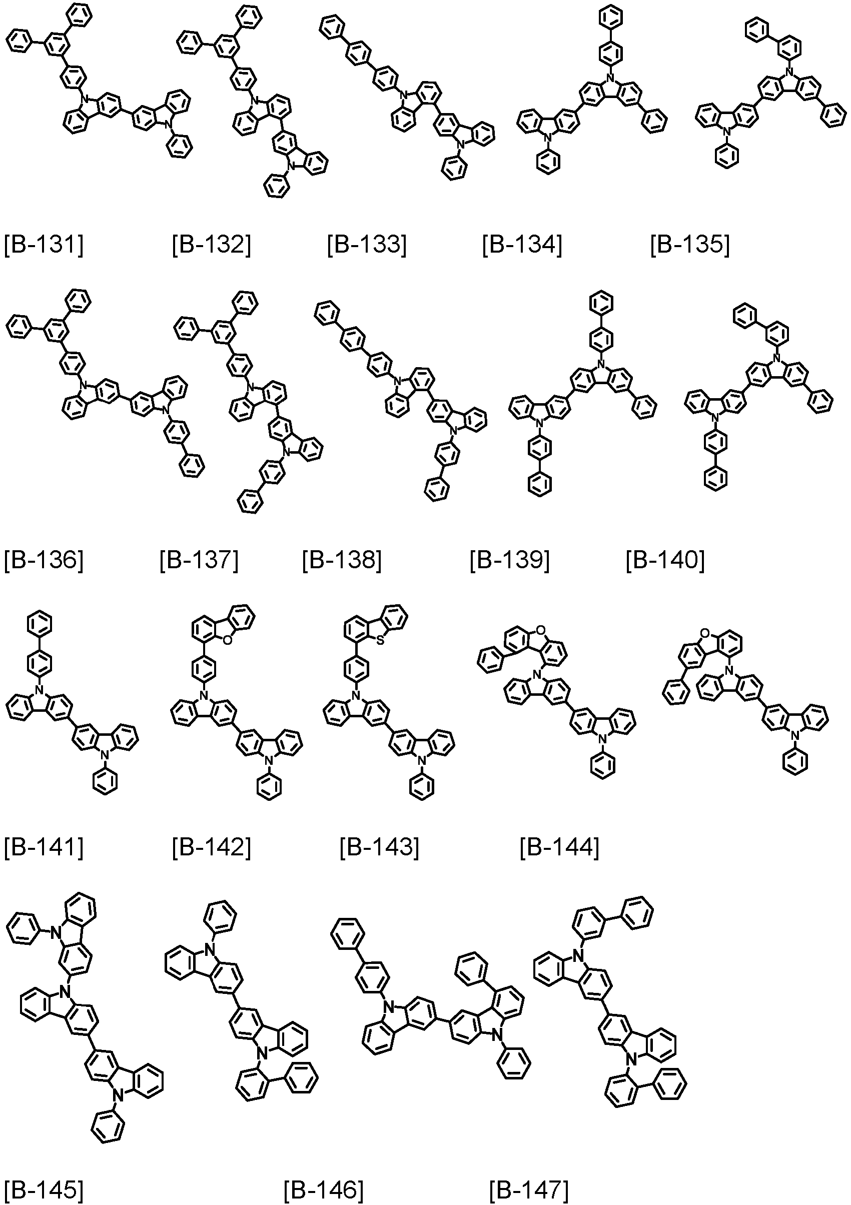

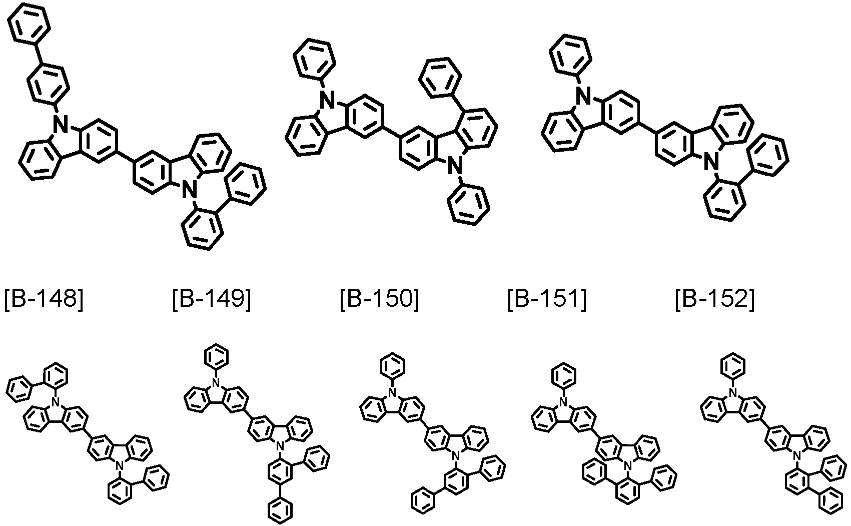

Additionally, examples of Compound B-1 to Compound B-152 listed in Group 2 in which at least one hydrogen is replaced with deuterium are given below, but are not limited thereto. 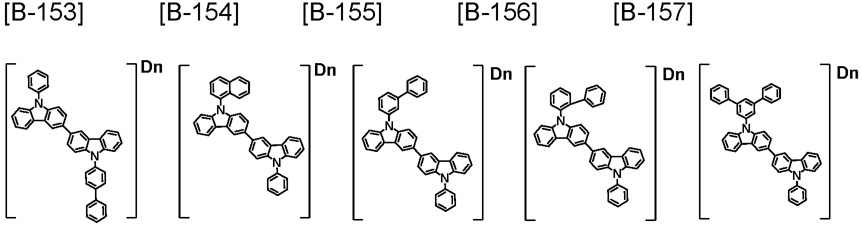 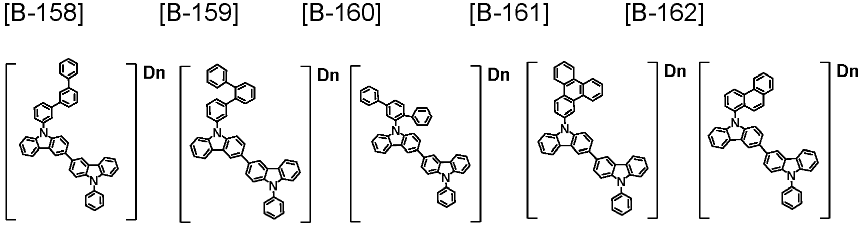 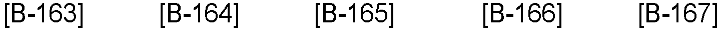 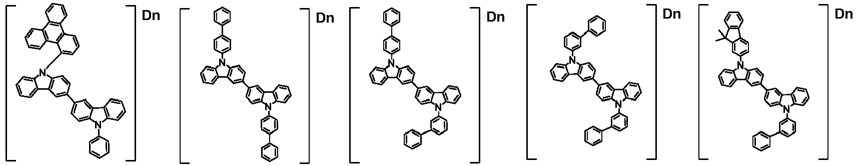 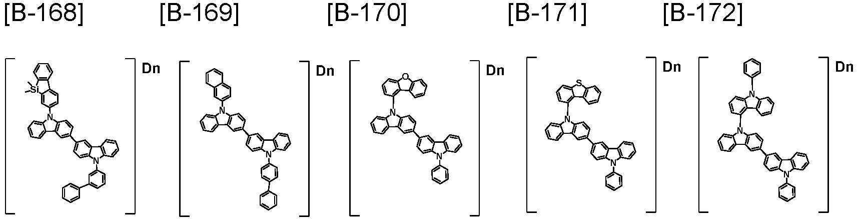 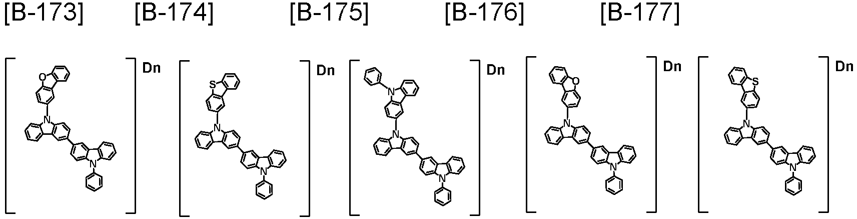 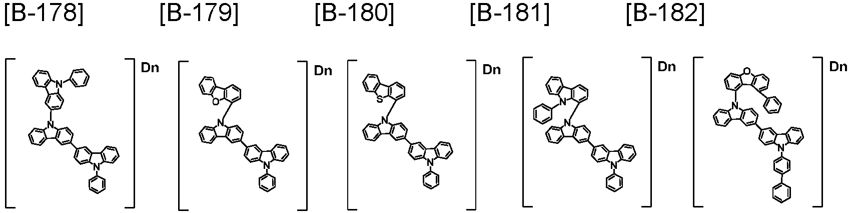 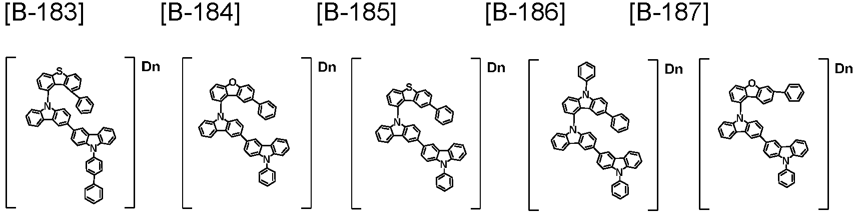 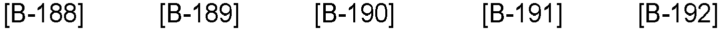 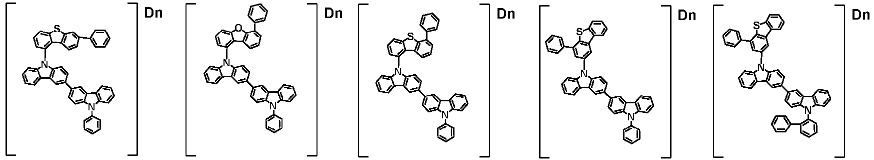 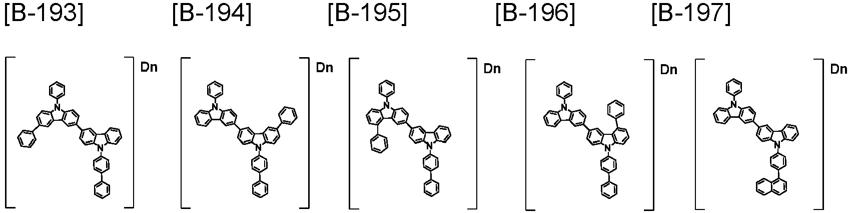 (Dn means the number of substituted deuterium and indicates a structure that one or more deuteriums are substituted)

For compounds B-153 to B-197 of Group 2, the most specific structures are presented below only as examples according to the deuterium substitution position and substitution rate, and are not intended to limit the scope of rights to compounds not presented below.

The scope of the present invention is determined by the claims, and if substituted with deuterium, the present invention is not limited to the compounds exemplified below but may include all changeable ranges within the range of Compound B-1 to Compound B-197 according to a deuterium substitution position and deuterium substitution rate, and the like.

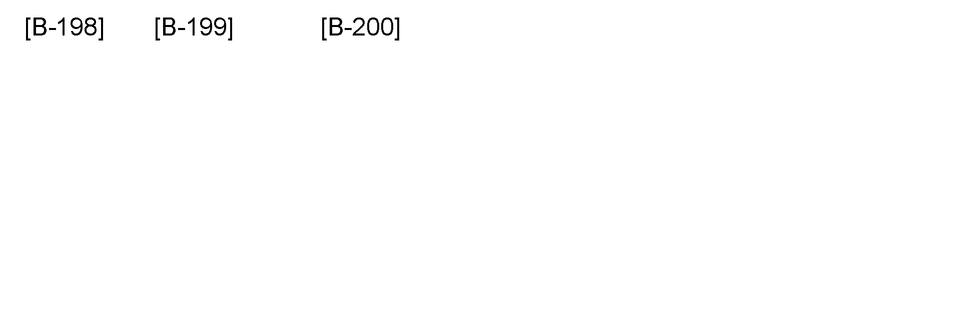

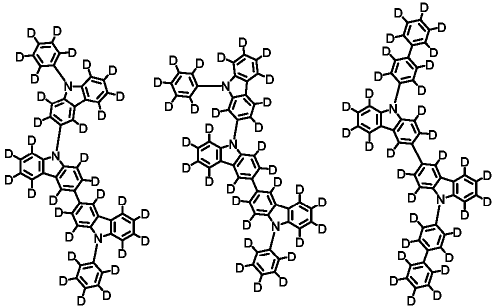

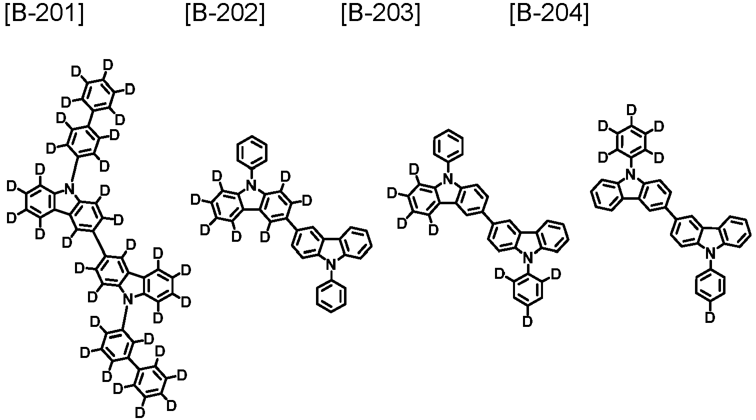

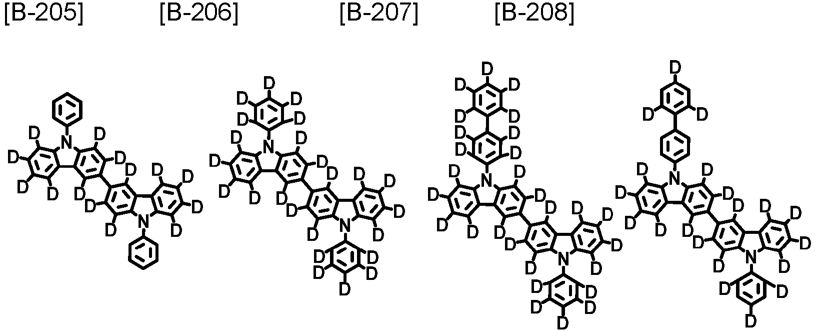

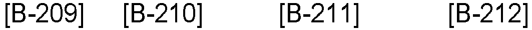

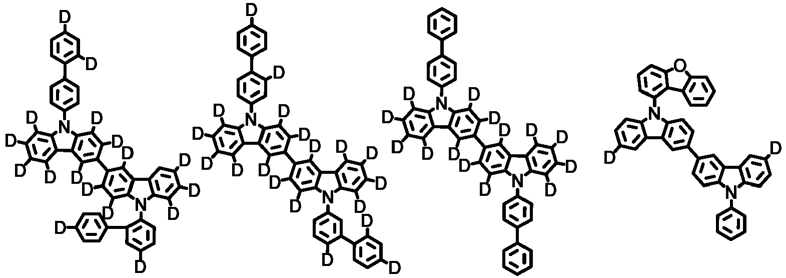

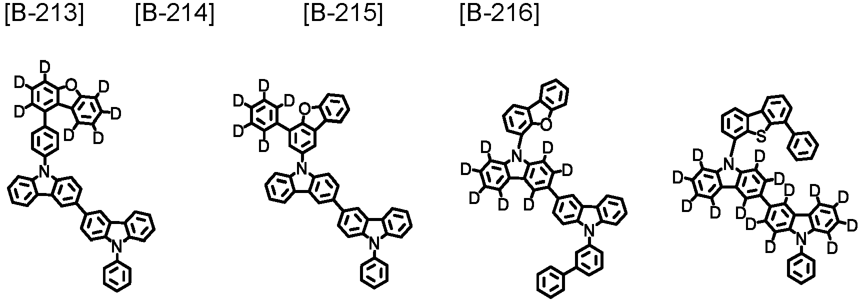

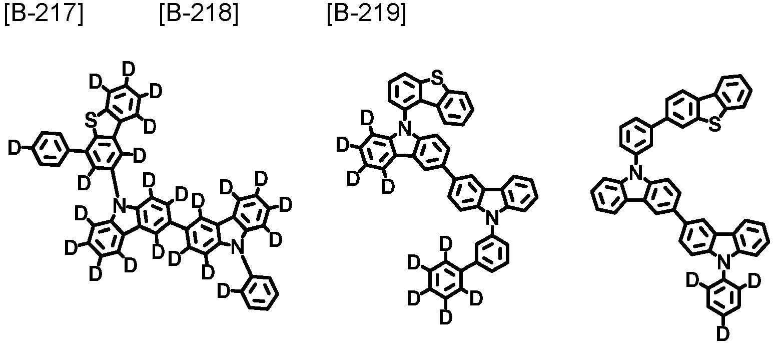

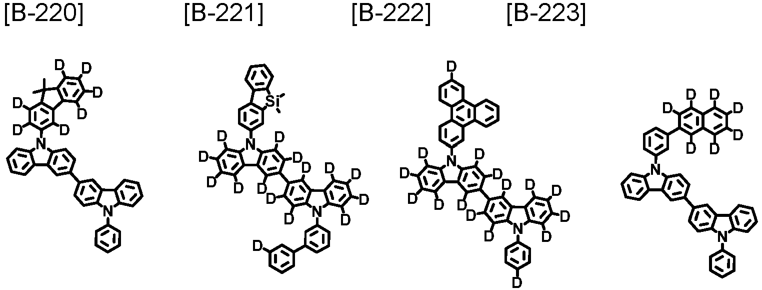

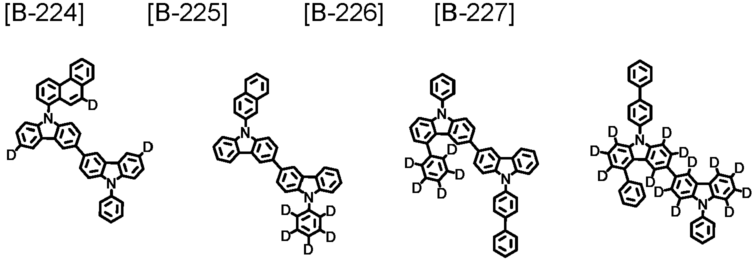

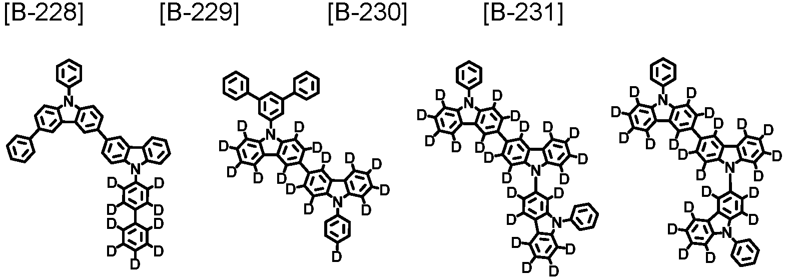

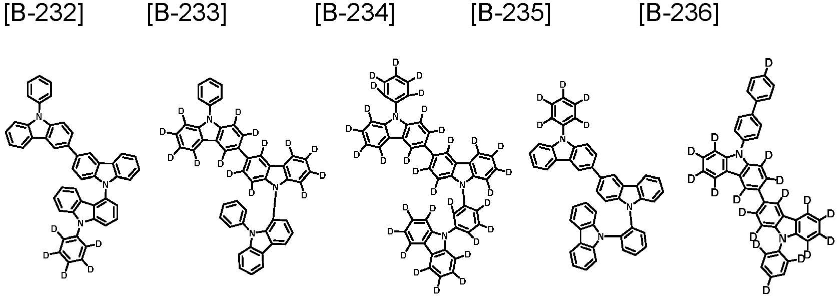

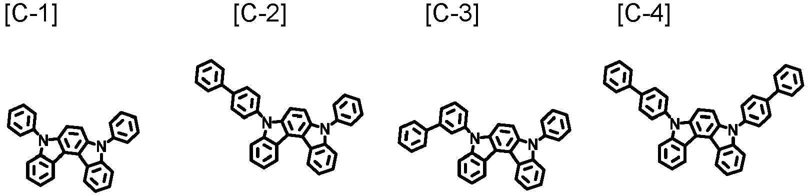

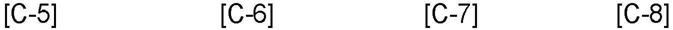

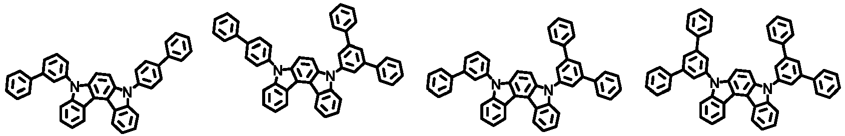

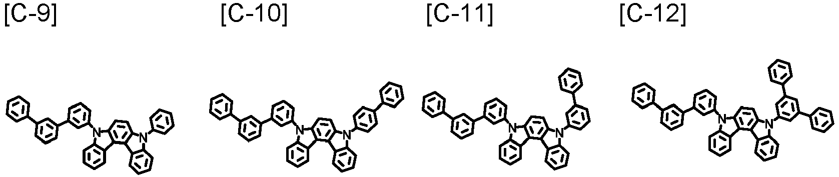

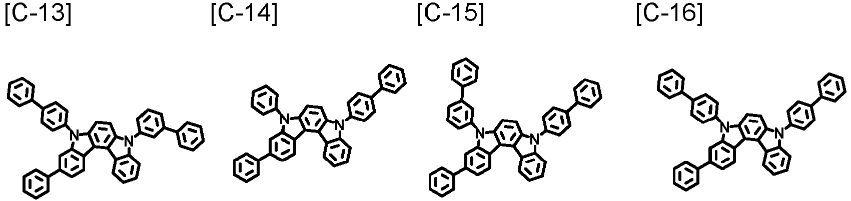

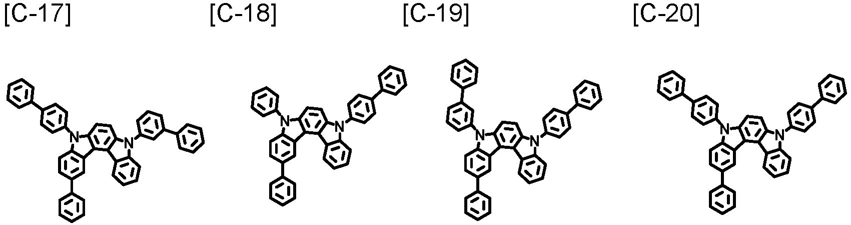

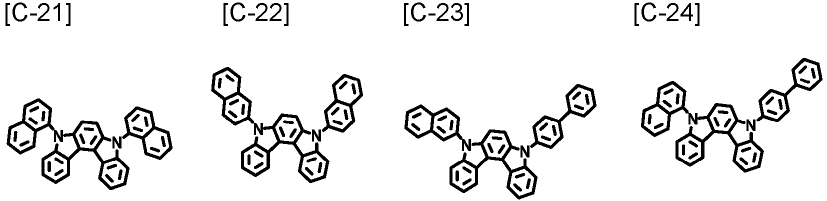

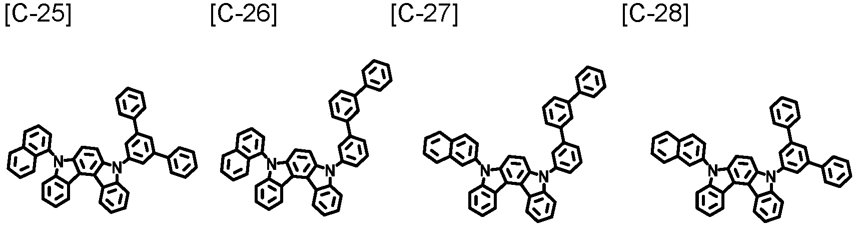

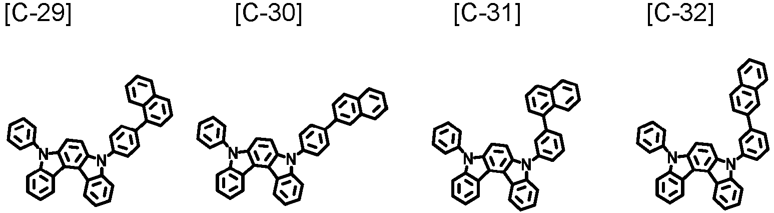

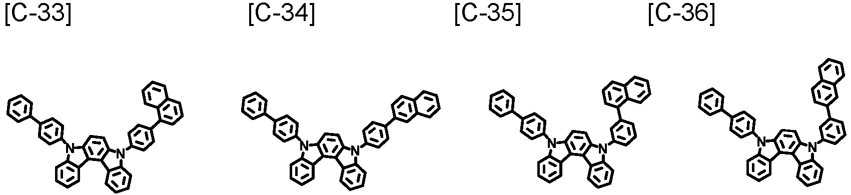

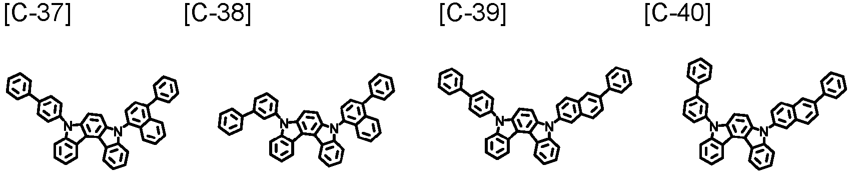

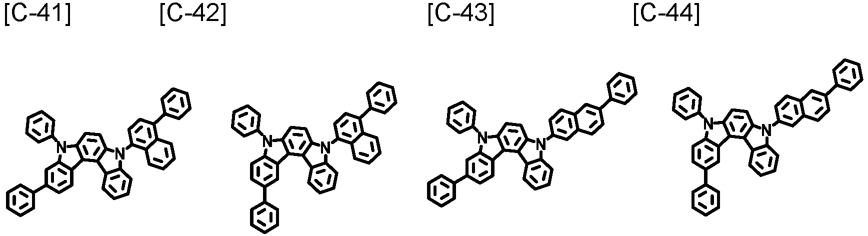

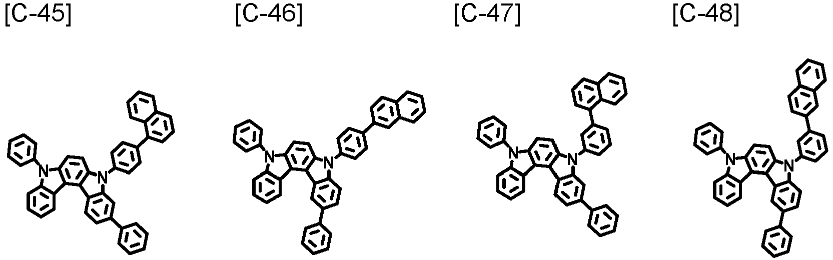

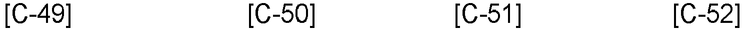

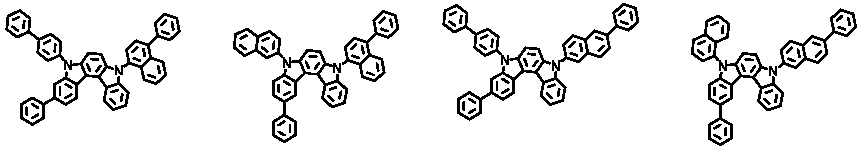

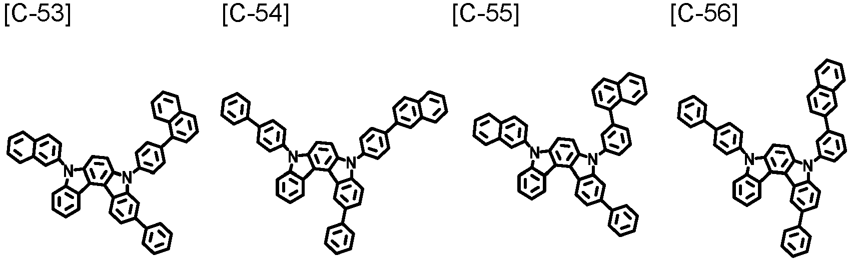

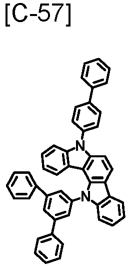

Additionally, examples of Compound C-1 to Compound C-57 listed in Group 2 in which at least one hydrogen is replaced with deuterium are given below, but are not limited thereto. 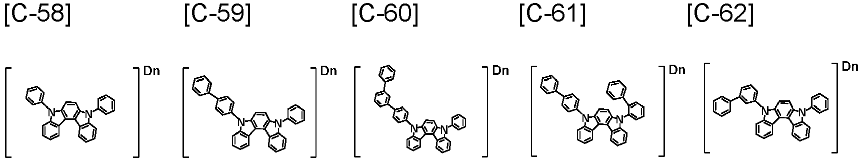 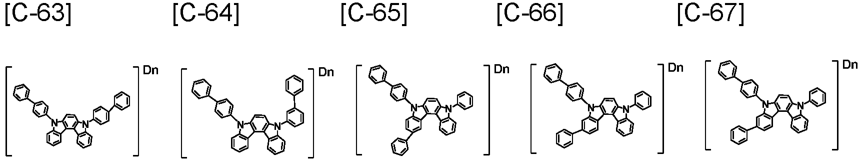 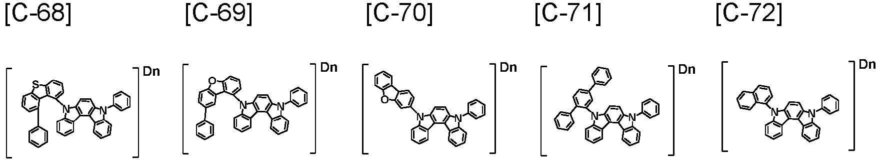 (Dn means the number of substituted deuterium and indicates a structure that one or more deuteriums are substituted)

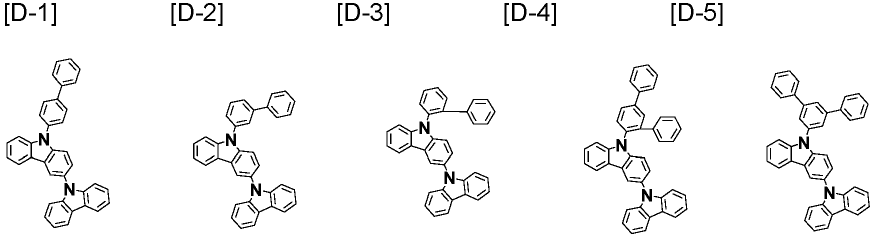

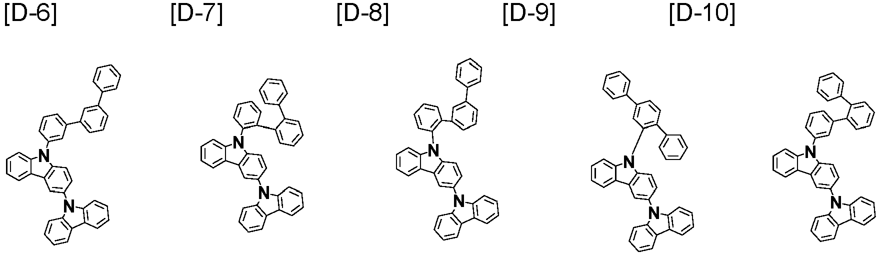

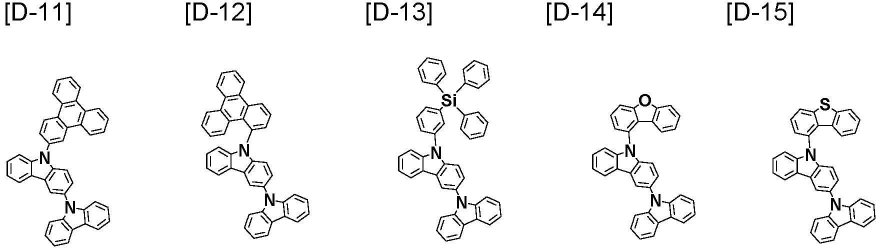

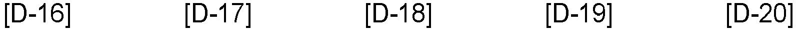

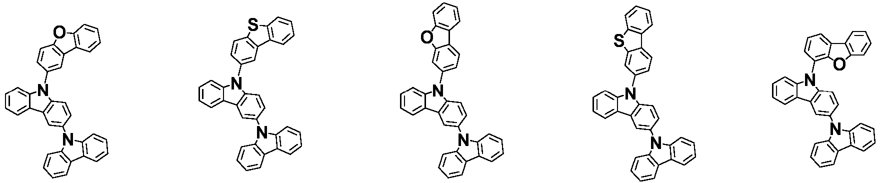

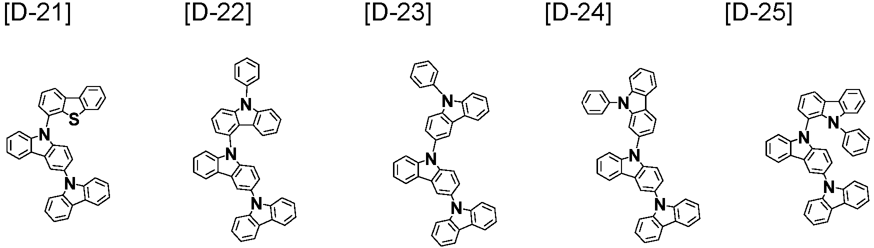

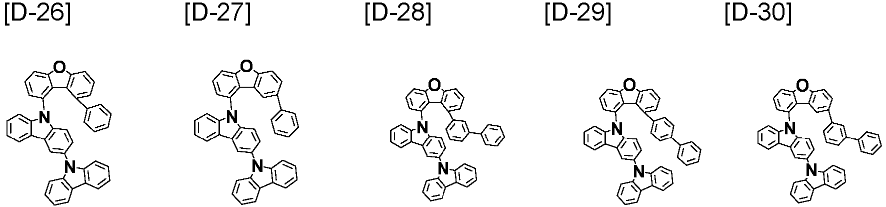

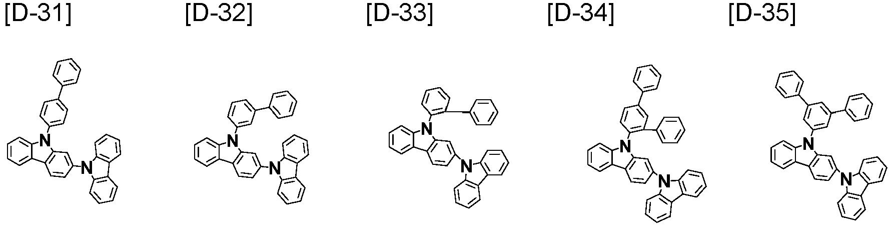

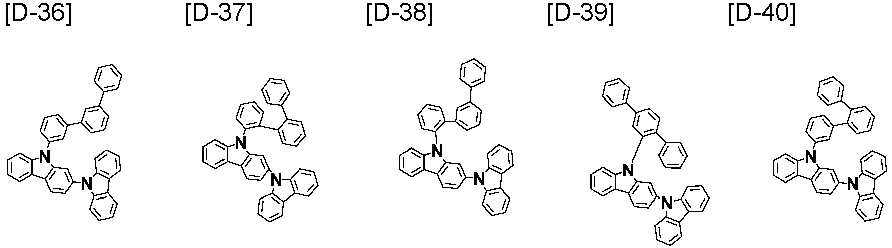

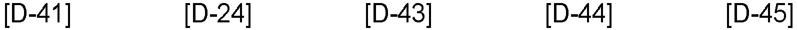

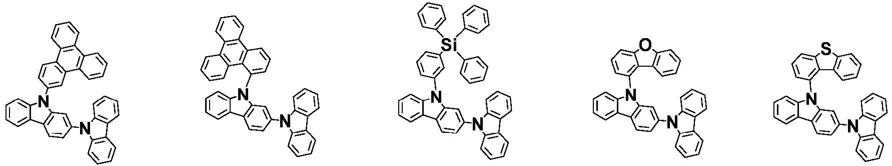

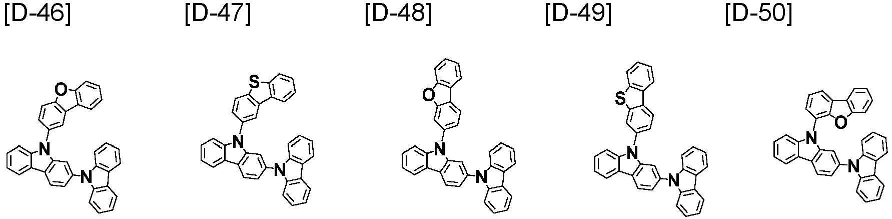

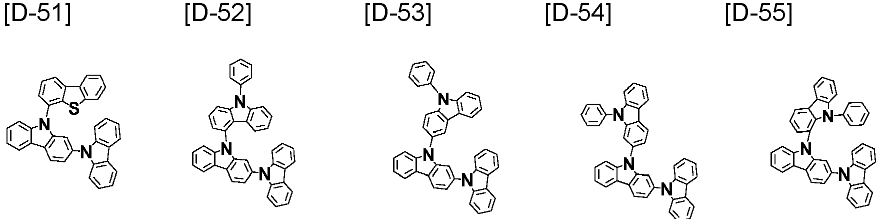

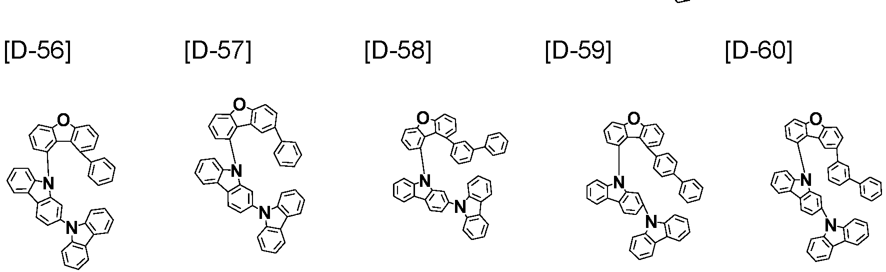

Additionally, examples of Compound D-1 to Compound D-60 listed in Group 2 in which at least one hydrogen is replaced with deuterium are given below, but are not limited thereto. 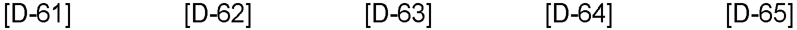 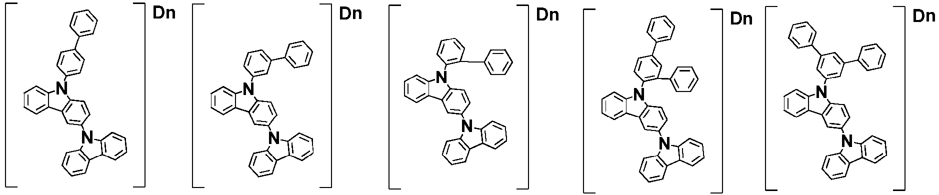 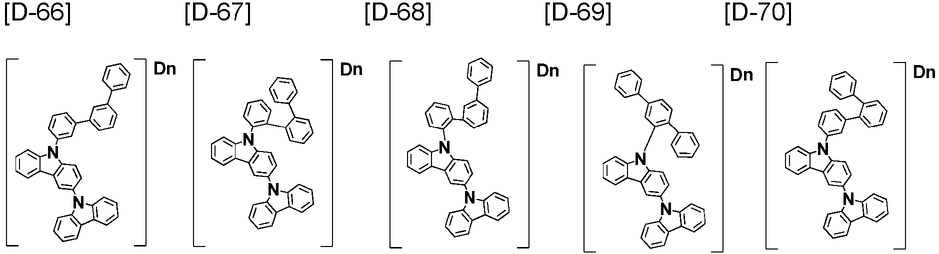 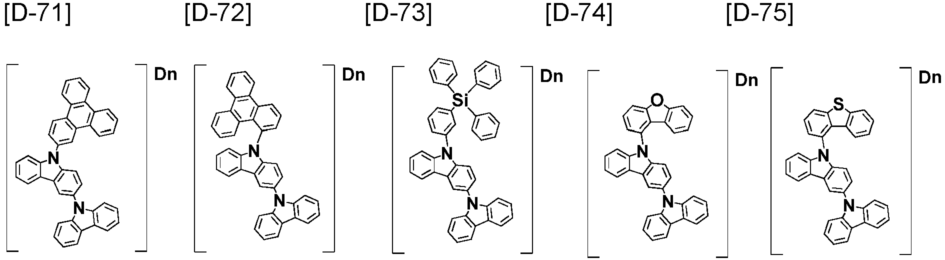 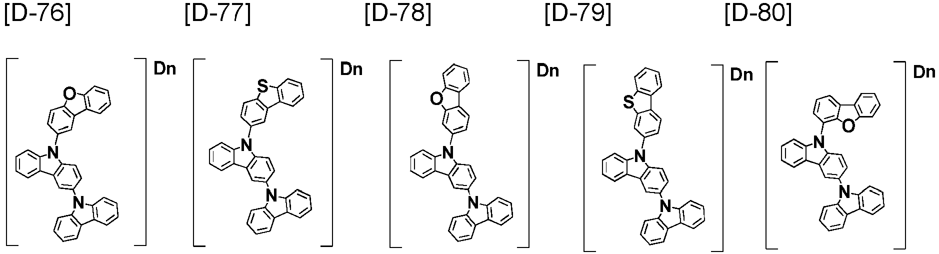 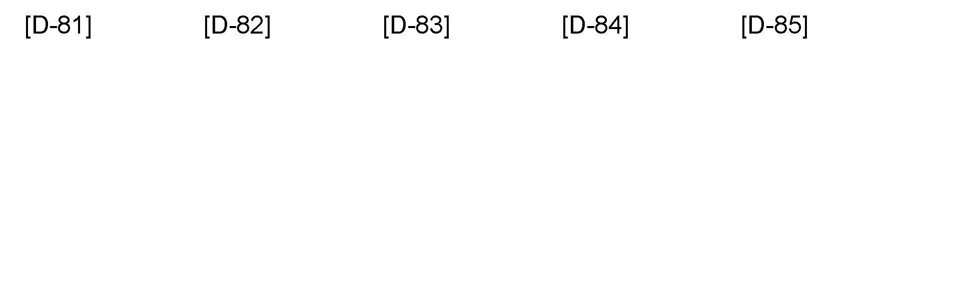 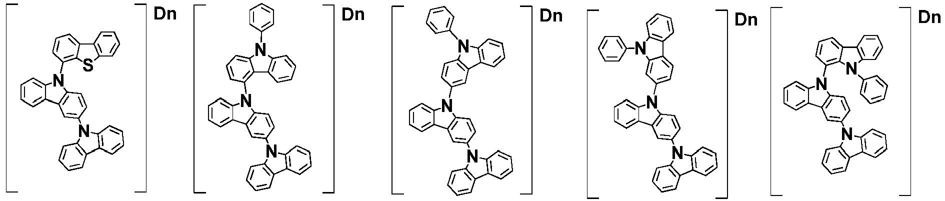 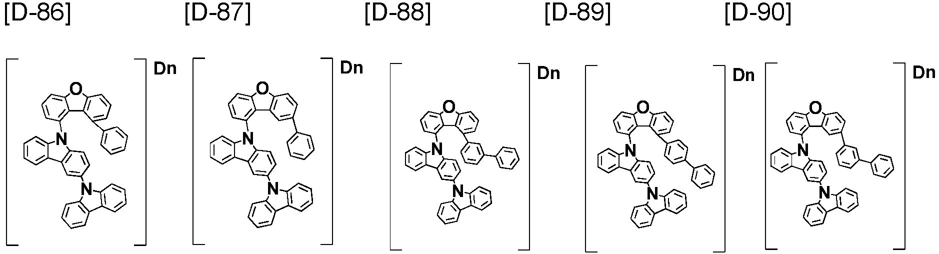 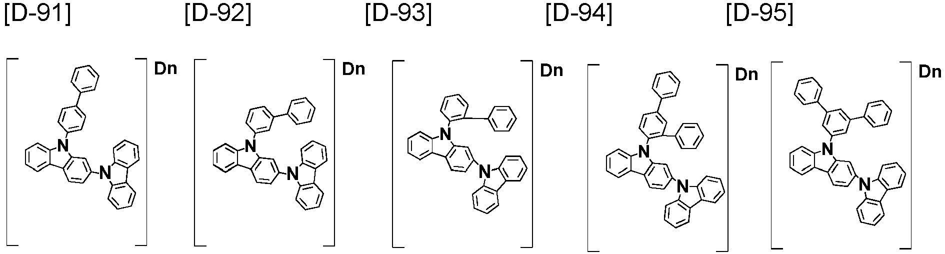 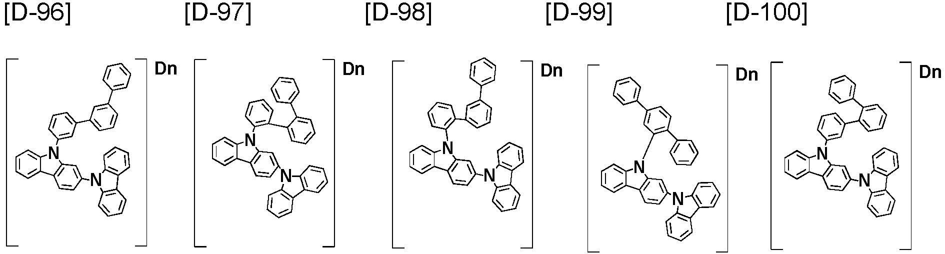 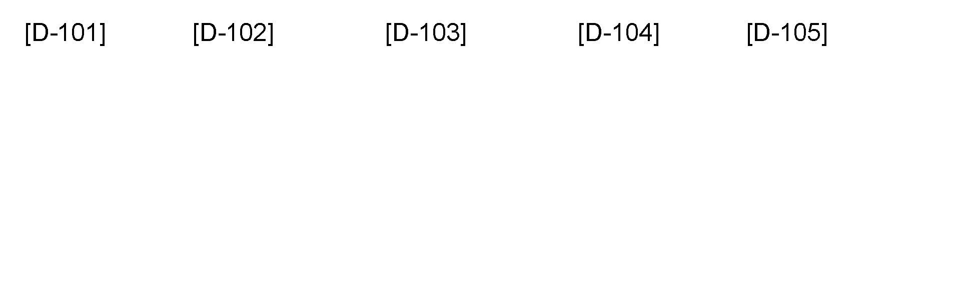 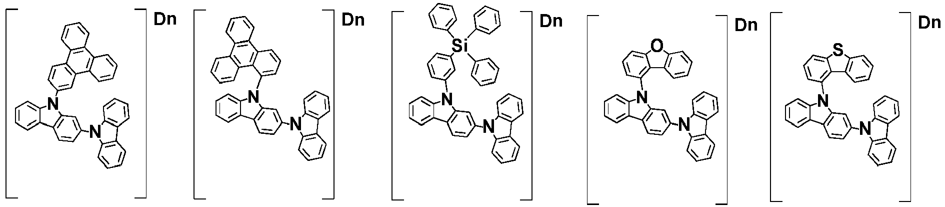 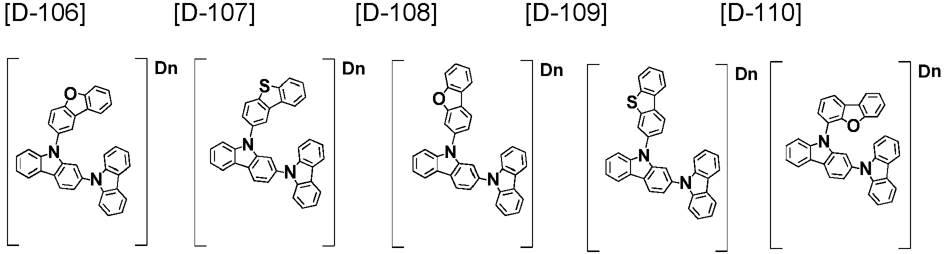 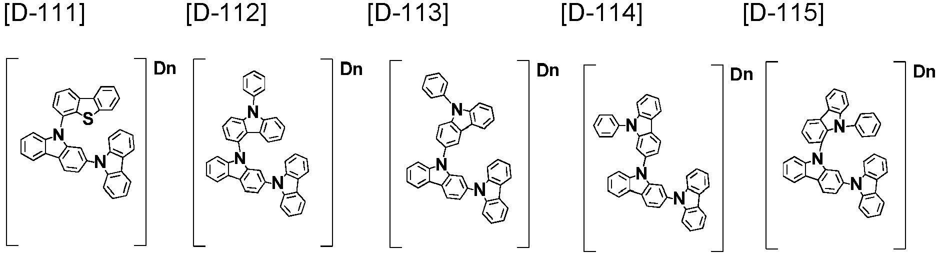 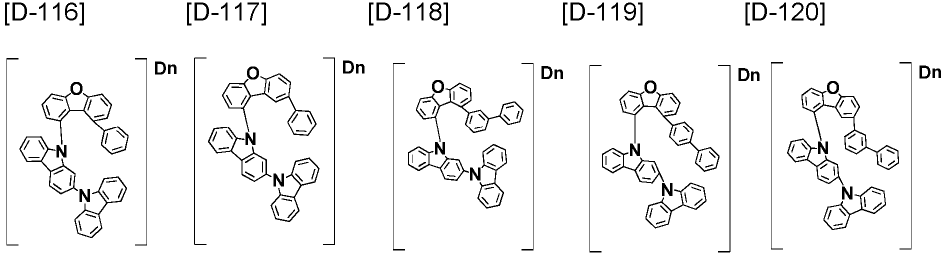 (Dn refers to the number of deuterium substitutions and indicates a structure substituted with one or more deuteriums)

The first compound and the second compound may be included in a weight ratio of, for example, 1:99 to 99:1. Within the above range, bipolar properties may be implemented by matching an appropriate weight ratio using electron transport capability of the first compound and the hole transport capability of the second compound, to improve efficiency and life-span. Within this range, for example, they may be included in a weight ratio of about 10:90 to 90:10, about 20:80 to 80:20, for example, about 20:80 to about 70: 30, about 20:80 to about 60:40, and about 30:70 to about 60:40. As a specific example, they may be included in a weight ratio of 40:60, 50:50, or 60:40.

In addition to the aforementioned first compound and second compound, one or more additional compounds may be included.

The aforementioned compound for the organic optoelectronic device or composition for the organic optoelectronic device may be a composition further including a dopant.

The dopant may be, for example, a phosphorescent dopant, for example, a red, green, or blue phosphorescent dopant, and may be, for example, a red or green phosphorescent dopant.

The dopant is a material mixed with the compound or composition for an organic optoelectronic device in a small amount to cause light emission, and may be generally a material such as a metal complex that emits light by multiple excitation into a triplet or more. The dopant may be, for example an inorganic, organic, or organic/inorganic compound, and one or more types thereof may be used.

Examples of the dopant may be a phosphorescent dopant and examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example a compound represented by Chemical Formula Z, but is not limited thereto.

[Chemical Formula Z] L⁹MX

In Chemical Formula Z, M is a metal, and L⁹ and X are the same or different and are a ligand to form a complex compound with M.

The M may be for example Ir, Pt, Os, Ti, Zr, Hf, Eu, Tb, Tm, Fe, Co, Ni, Ru, Rh, Pd, or a combination thereof and L⁹ and X may be for example a bidentate ligand.

Examples of ligands represented by L⁹ and X may be selected from the chemical formulas listed in Group A, but are not limited thereto.

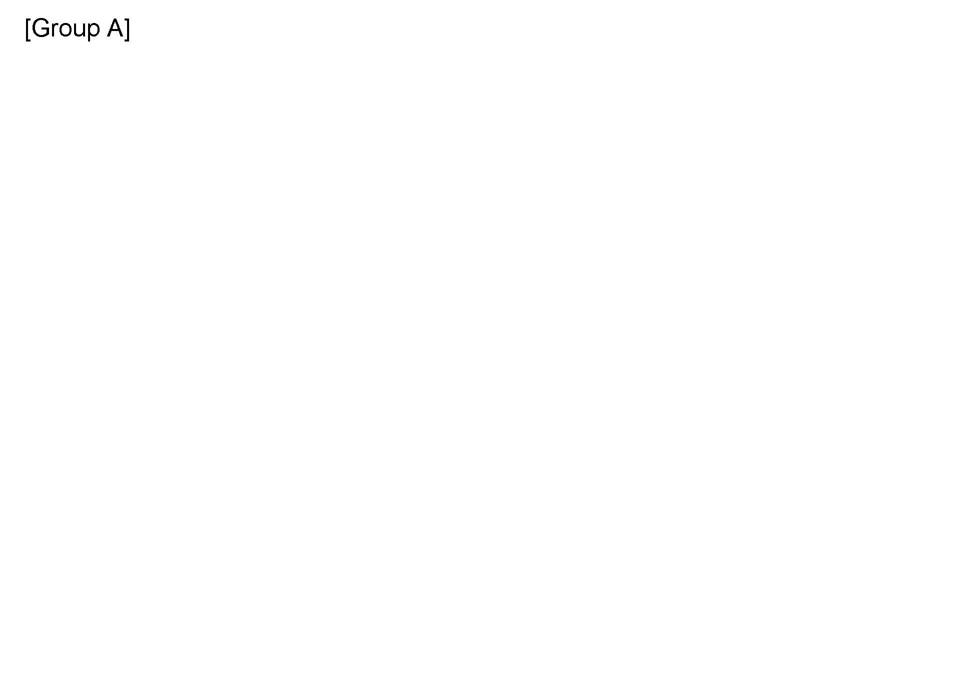

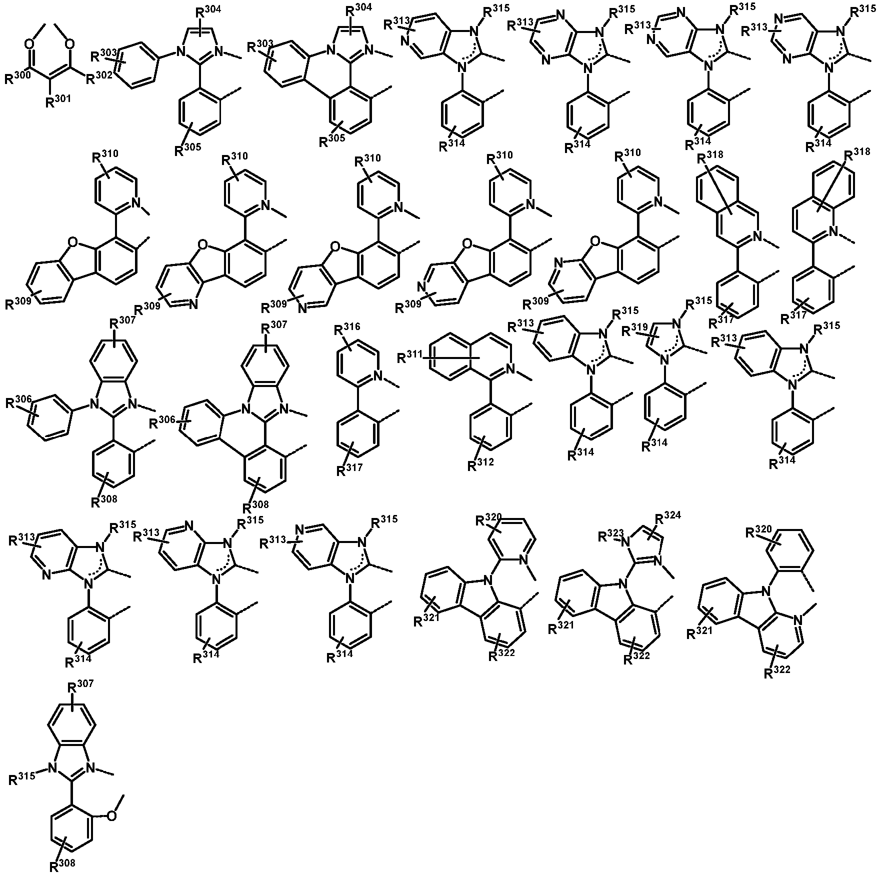

In Group A,
R³⁰⁰ to R³⁰² are each independently hydrogen, deuterium, a C1 to C30 alkyl group that is substituted or unsubstituted with a halogen, a C6 to C30 aryl group that is substituted or unsubstituted with a C1 to C30 alkyl, or a halogen, and
R³⁰³ to R³²⁴ are each independently hydrogen, deuterium, halogen, a substituted or unsubstituted C1 to C30 alkyl group, a substituted or unsubstituted C1 to C30 alkoxy group, a substituted or unsubstituted C3 to C30 cycloalkyl group, a substituted or unsubstituted C2 to C30 alkenyl group, a substituted or unsubstituted C6 to C30 aryl group, a substituted or unsubstituted C1 to C30 heteroaryl group, a substituted or unsubstituted C1 to C30 amino group, a substituted or unsubstituted C6 to C30 arylamino group, SFs, a trialkylsilyl group having a substituted or unsubstituted C1 to C30 alkyl group, a dialkylarylsilyl group having a substituted or unsubstituted C1 to C30 alkyl group and a C6 to C30 aryl group, or a triarylsilyl group having a substituted or unsubstituted C6 to C30 aryl group.

The dopant according to an embodiment may be an iridium complex, and may be, for example, represented by Chemical Formula 6-1 or Chemical Formula 6-2.

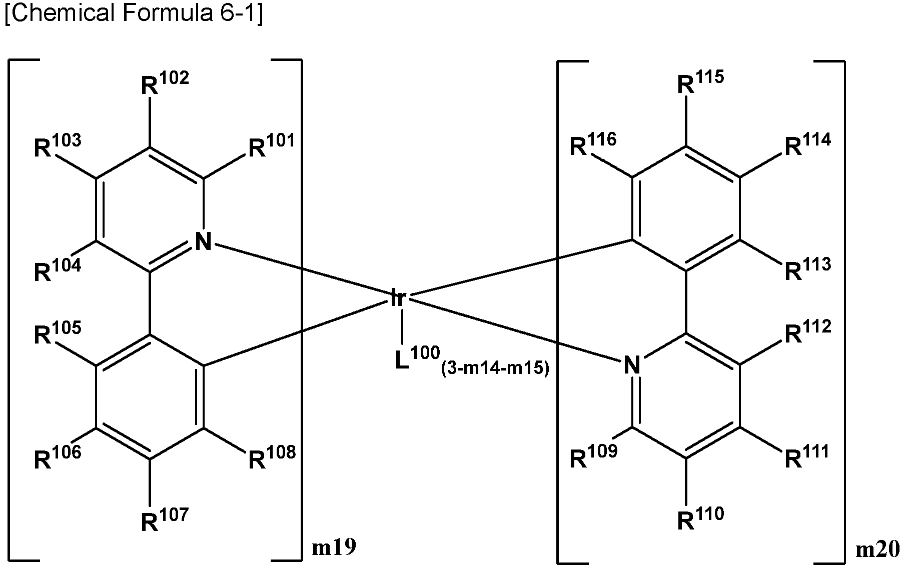

In Chemical Formula 6-1,
R¹⁰¹ to R¹¹⁶ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a C1 to C6 alkyl group,
at least one of R¹⁰¹ to R¹¹⁶ is a functional group represented by Chemical Formula V-1,
L¹⁰⁰ is a bidentate ligand of a monovalent anion, and is a ligand that coordinates to iridium through a lone pair of carbons or heteroatoms, and
m19 and m20 are each independently any one of integers of 0 to 3, and m19 + m20 is any one of integers of 1 to 3, 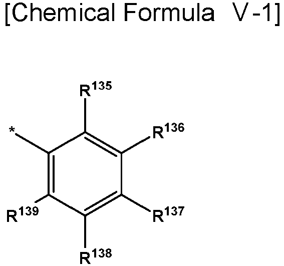
wherein, in Chemical Formula V-1,
R¹³⁵ to R¹³⁹ are each independently hydrogen, deuterium, a substituted or unsubstituted C1 to C10 alkyl group, a substituted or unsubstituted C6 to C20 aryl group, or -SiR¹³²R¹³³R¹³⁴, and
* means a portion linked to a carbon atom.

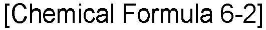

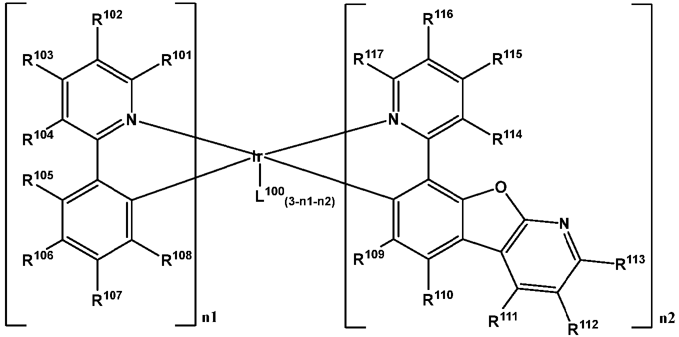

In Chemical Formula 6-2,
R¹⁰¹ to R¹¹⁷ are each independently hydrogen, deuterium, a substituted or unsubstituted C1 to C10 alkyl group, a substituted or unsubstituted C6 to C20 aryl group, or -SiR¹³³R¹³⁴R¹³⁵,
R¹³³ to R¹³⁵ are each independently a substituted or unsubstituted C1 to C6 alkyl group,
L¹⁰⁰ is a bidentate ligand of a monovalent anion, and is a ligand that coordinates to iridium through a lone pair of carbons or heteroatoms, and
n1 and n2 are each independently any one of integers of 0 to 3, and n1 + n2 is any one of integers of 1 to 3.

As an example, a dopant represented by Chemical Formula Z-1 may be included.

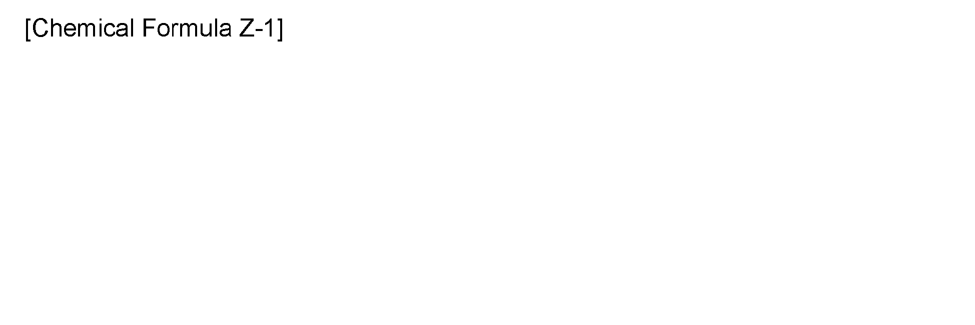

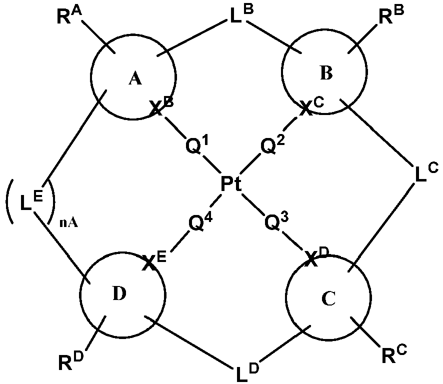

In Chemical Formula Z-1, rings A, B, C, and D independently represent a 5-membered or 6-membered carbocyclic or heterocyclic ring;
R^{A}, R^{B}, R^{C}, and R^{D} independently represent mono-, di-, tri-, or tetra-substitution, or unsubstitution;
L^{B}, L^{C}, and L^{D} are each independent selected from a direct bond, BR, NR, PR, O, S, Se, C=O, S=O, SO₂, CRR', SiRR', GeRR', and a combination thereof;
when nA is 1, L^{E} is selected from a direct bond, BR, NR, PR, O, S, Se, C=O, S=O, SO₂, CRR', SiRR', GeRR', and a combination thereof; when nA is 0, L^{E} does not exist; and
R^{A}, R^{B}, R^{C}, R^{D}, R, and R' are each independently selected from hydrogen, deuterium, a halogen, alkyl group, a cycloalkyl group, a heteroalkyl group, an arylalkyl group, an alkoxy group, an aryloxy group, an amino group, a silyl group, an alkenyl group, a cycloalkenyl group, a heteroalkenyl group, an alkynyl group, an aryl group, a heteroaryl group, an acyl group, a carbonyl group, a carboxylic acid group, an ester group, a nitrile group, an isonitrile group, a sulfanyl group, a sulfinyl group, a sulfonyl group, a phosphino group, and a combination thereof; any adjacent R^{A}, R^{B}, R^{C}, R^{D}, R, and R' are optionally linked to each other to provide a ring; X^{B}, X^{C}, X^{D}, and X^{E} are each independently selected from carbon and nitrogen; and Q¹, Q², Q³, and Q⁴ each represent oxygen or a direct bond.

The platinum complex may be represented, for example, by Chemical Formula 7-1 or Chemical Formula 7-2.

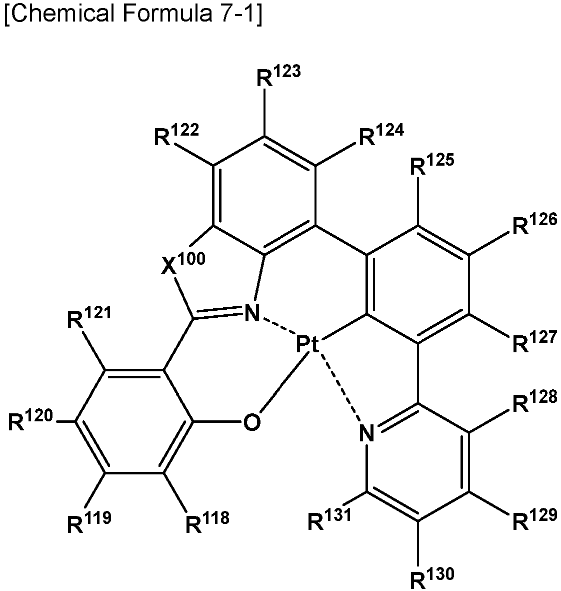

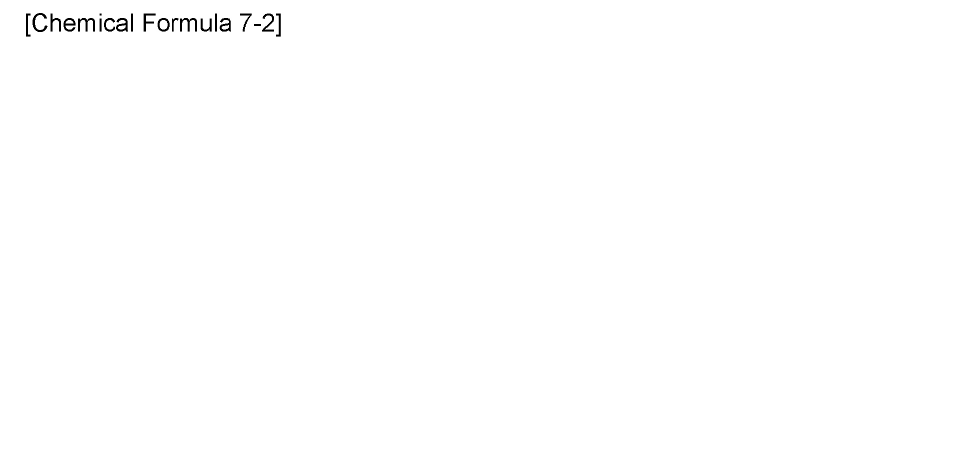

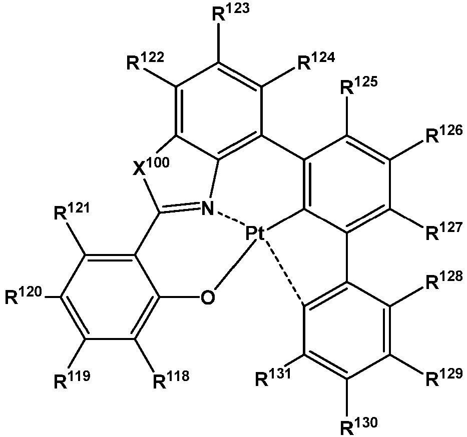

In Chemical Formula 7-1 and Chemical Formula 7-2,
X¹⁰⁰ is selected from O, S, and NR¹³²,
R¹¹⁸ to R¹³² are each independently hydrogen, deuterium, a substituted or unsubstituted C1 to C10 alkyl group, a substituted or unsubstituted C6 to C20 aryl group, or -SiR¹³³R¹³⁴R¹³⁵,
R¹³³ to R¹³⁵ are each independently a substituted or unsubstituted C1 to C6 alkyl group,
at least one of R¹¹⁸ to R¹³² is -SiR¹³³R¹³⁴R¹³⁵ or a tert-butyl group, and
R¹³³ to R¹³⁵ are each independently a substituted or unsubstituted C1 to C6 alkyl group.

Hereinafter, an organic optoelectronic device using the aforementioned compound for an organic optoelectronic device or composition for an organic optoelectronic device is described.

The organic optoelectronic device may be a suitable device to convert electrical energy into photoenergy and vice versa, e.g., an organic photoelectric device, an organic light emitting diode, an organic solar cell, or an organic photoconductor drum.

Here, an organic light emitting diode as one example of an organic optoelectronic device is described referring to drawings.

FIG. 1 is a cross-sectional view showing an organic light emitting diode according to an embodiment.

Referring to FIG. 1, an organic light emitting diode 100 according to an embodiment includes an anode 120 and a cathode 110 facing each other and an organic layer 105 disposed between the anode 120 and cathode 110.

The anode 120 may be made of a conductor having a large work function to help hole injection, and may be for example a metal, a metal oxide and/or a conductive polymer. The anode 120 may be, for example a metal such as nickel, platinum, vanadium, chromium, copper, zinc, gold, and the like or an alloy thereof; a metal oxide such as zinc oxide, indium oxide, indium tin oxide (ITO), indium zinc oxide (IZO), and the like; a combination of a metal and an oxide such as ZnO and Al or SnO₂ and Sb; a conductive polymer such as poly(3-methylthiophene), poly(3,4-(ethylene-1,2-dioxy)thiophene) (PEDOT), polypyrrole, and polyaniline, but is not limited thereto.

The cathode 110 may be made of a conductor having a small work function to help electron injection, and may be for example a metal, a metal oxide, and/or a conductive polymer. The cathode 110 may include a metal such as magnesium, calcium, sodium, potassium, titanium, indium, yttrium, lithium, gadolinium, aluminum, silver, tin, lead, cesium, barium, or an alloy thereof; a multilayer structure material such as LiF/Al, LiO₂/Al, LiF/Ca, LiF/Al, and BaF₂/Ca, but is not limited thereto.

The organic layer 105 may include the aforementioned compound for an organic optoelectronic device or composition for an organic optoelectronic device.

The organic layer 105 may include a light emitting layer 130 and the light emitting layer 130 may include the aforementioned compound for an organic optoelectronic device or composition for an organic optoelectronic device.

The composition for an organic optoelectronic device further including a dopant may be, for example, a green light emitting composition.

The light emitting layer 130 may include, for example, the aforementioned compound for an organic optoelectronic device or composition for an organic optoelectronic device, as a phosphorescent host.

The organic layer may further include a charge transport region in addition to the light emitting layer.

The charge transport region may be, for example, a hole transport region 140.

The hole transport region 140 may further increase hole injection and/or hole mobility between the anode 120 and the light emitting layer 130 and block electrons.

Specifically, the hole transport region 140 may include a hole transport layer between the anode 120 and the light emitting layer 130, and a hole transport auxiliary layer between the light emitting layer 130 and the hole transport layer, and at least one of the compounds of Group B may be included in at least one of the hole transport layer and the hole transport auxiliary layer. 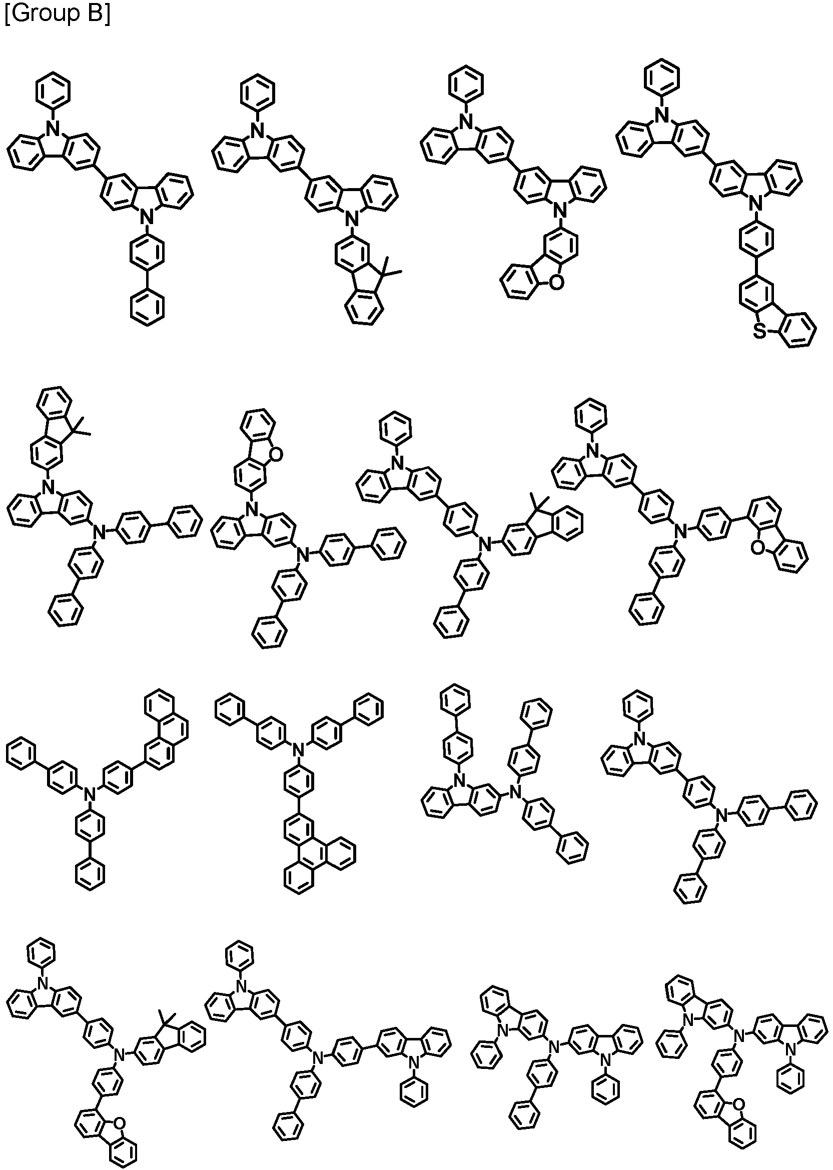 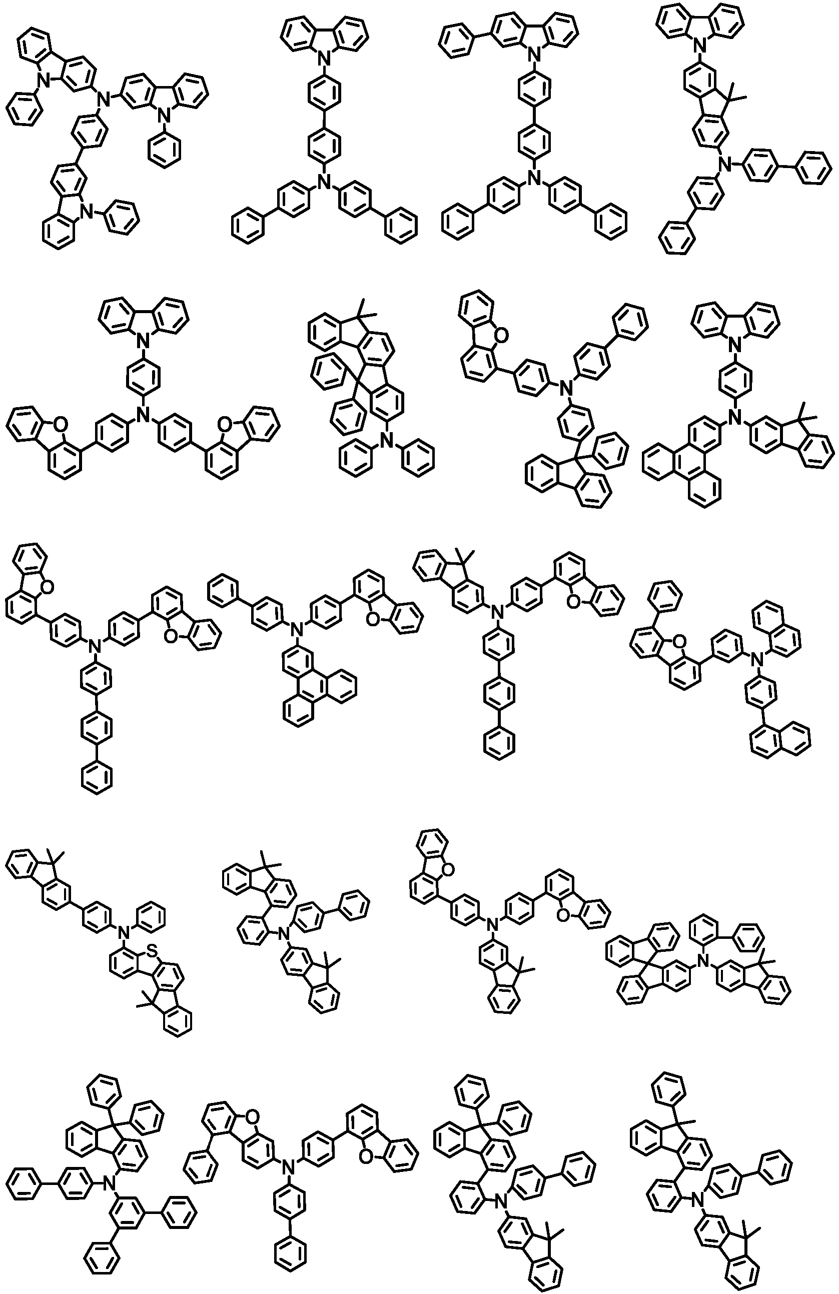 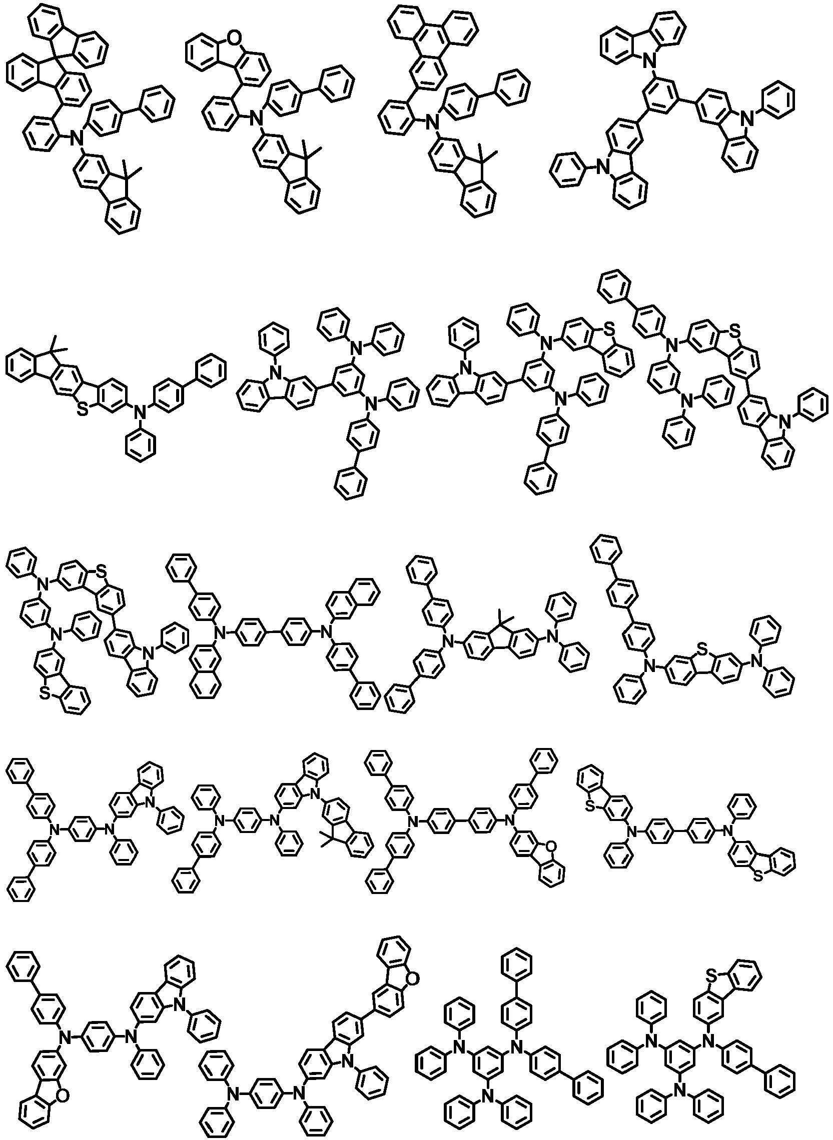 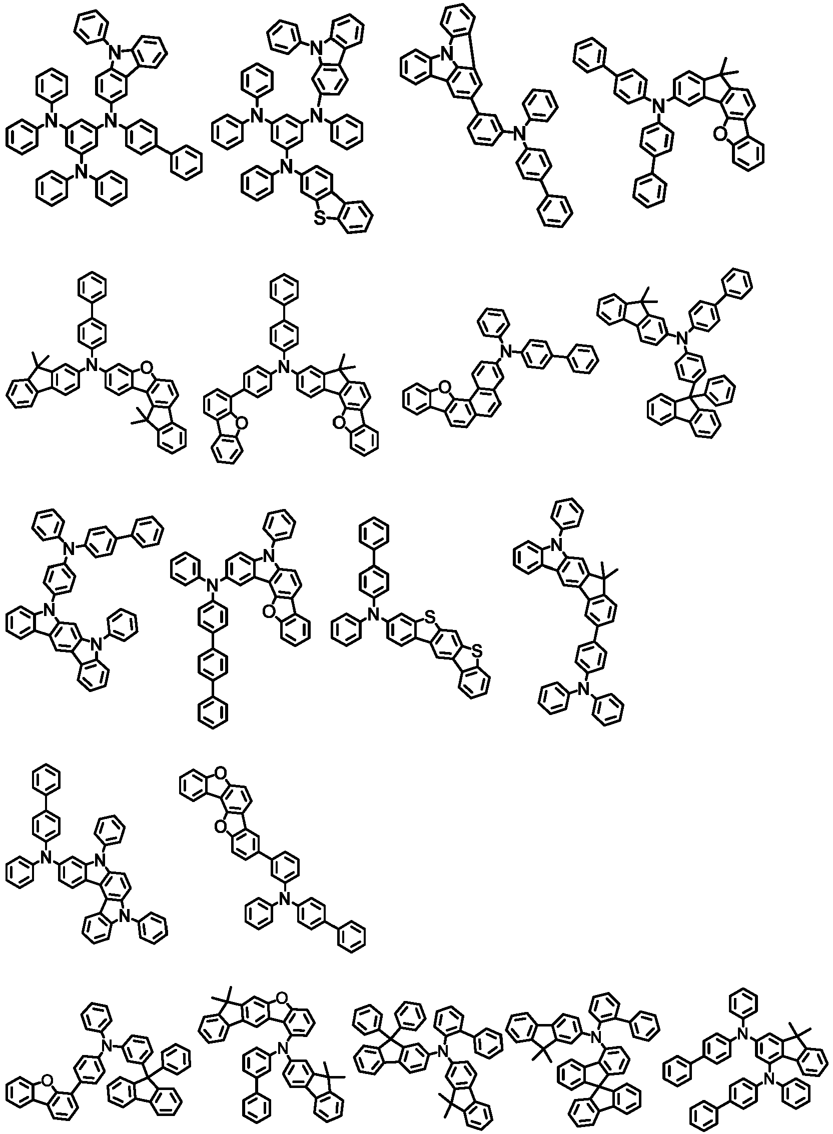 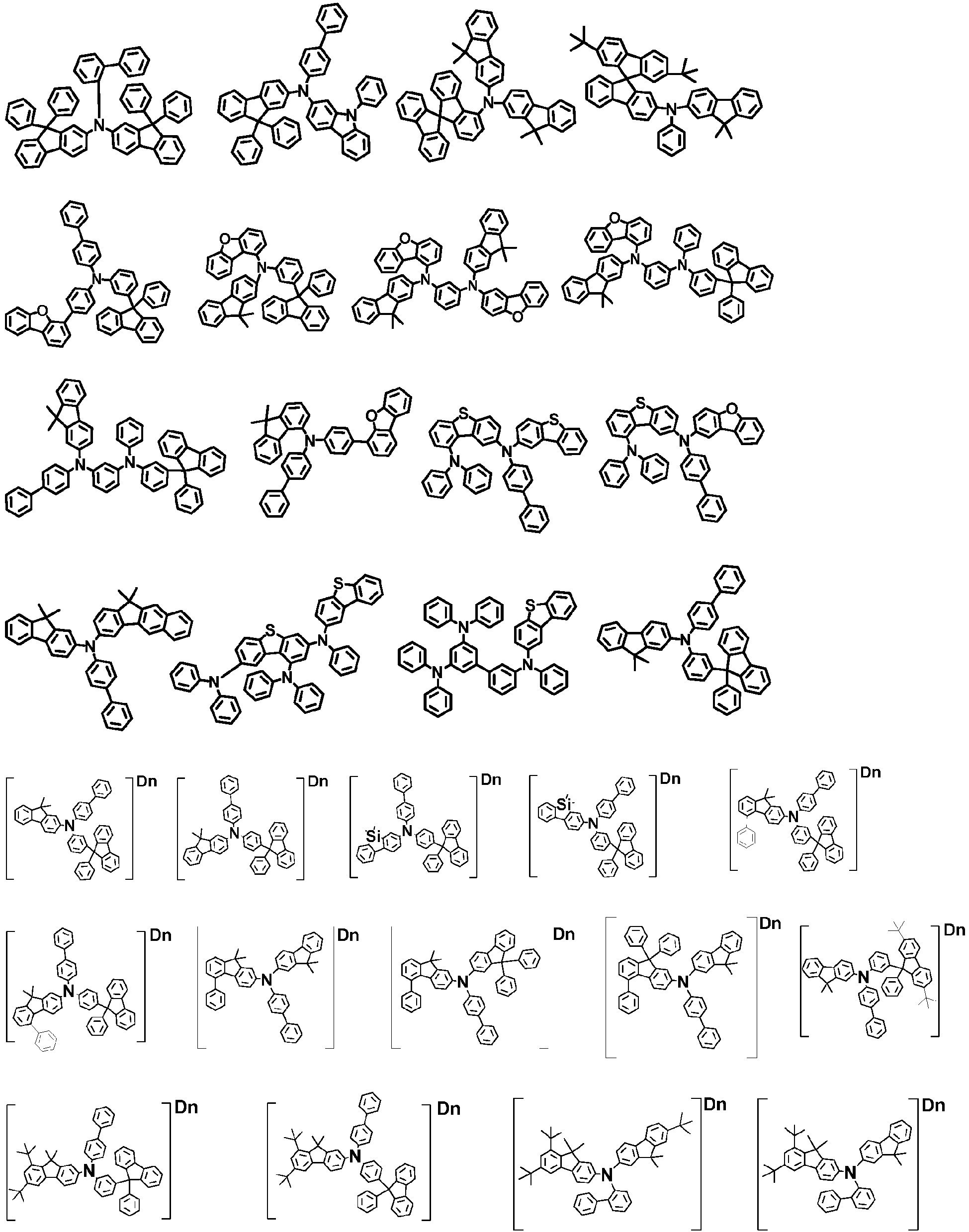 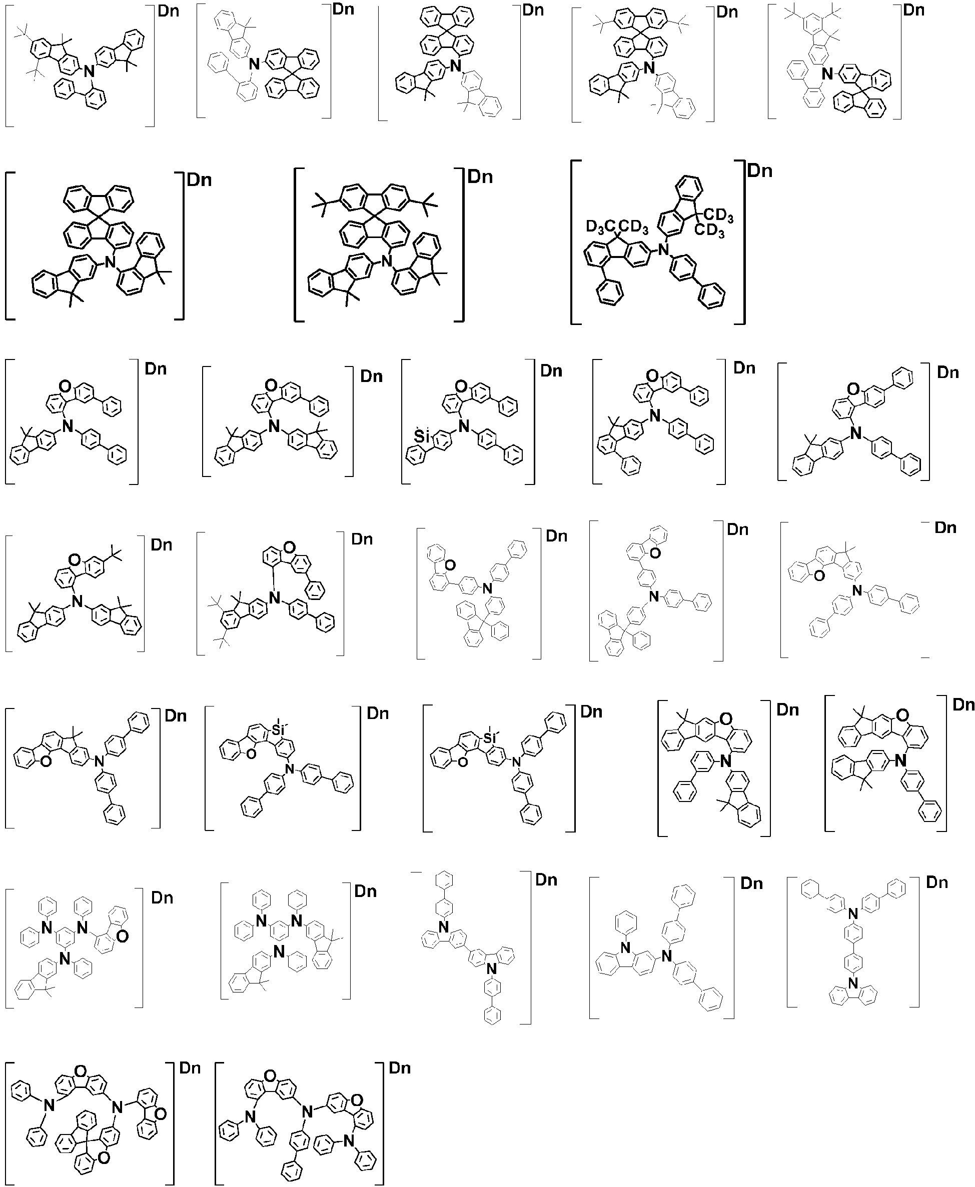 (Dn refers to the number of deuterium substitutions and indicates a structure substituted with one or more deuteriums)

In the hole transport region, in addition to the compounds described above, known compounds disclosed in US5061569A, JP1993-009471A, WO1995-009147A1, JP1995-126615A, JP1998-095973A, etc. and compounds having a similar structure may also be used.

Also, the charge transport region may be, for example, the electron transport region 150.

The electron transport region 150 may further increase electron injection and/or electron mobility and block holes between the cathode 110 and the light emitting layer 130.

Specifically, the electron transport region 150 may include an electron transport layer between the cathode 110 and the light emitting layer 130, and an electron transport auxiliary layer between the light emitting layer 130 and the electron transport layer, and at least one of the compounds of Group C may be included in at least one of the electron transport layer and the electron transport auxiliary layer.

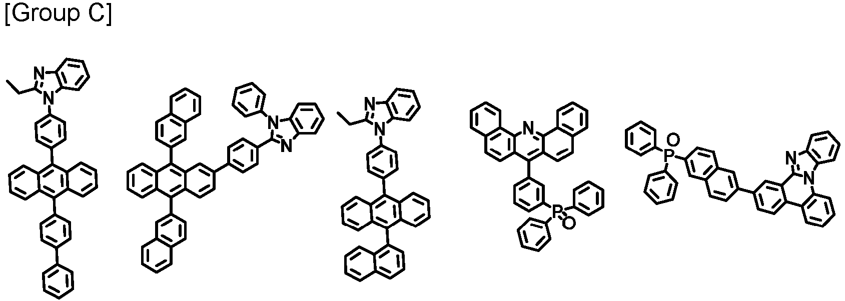

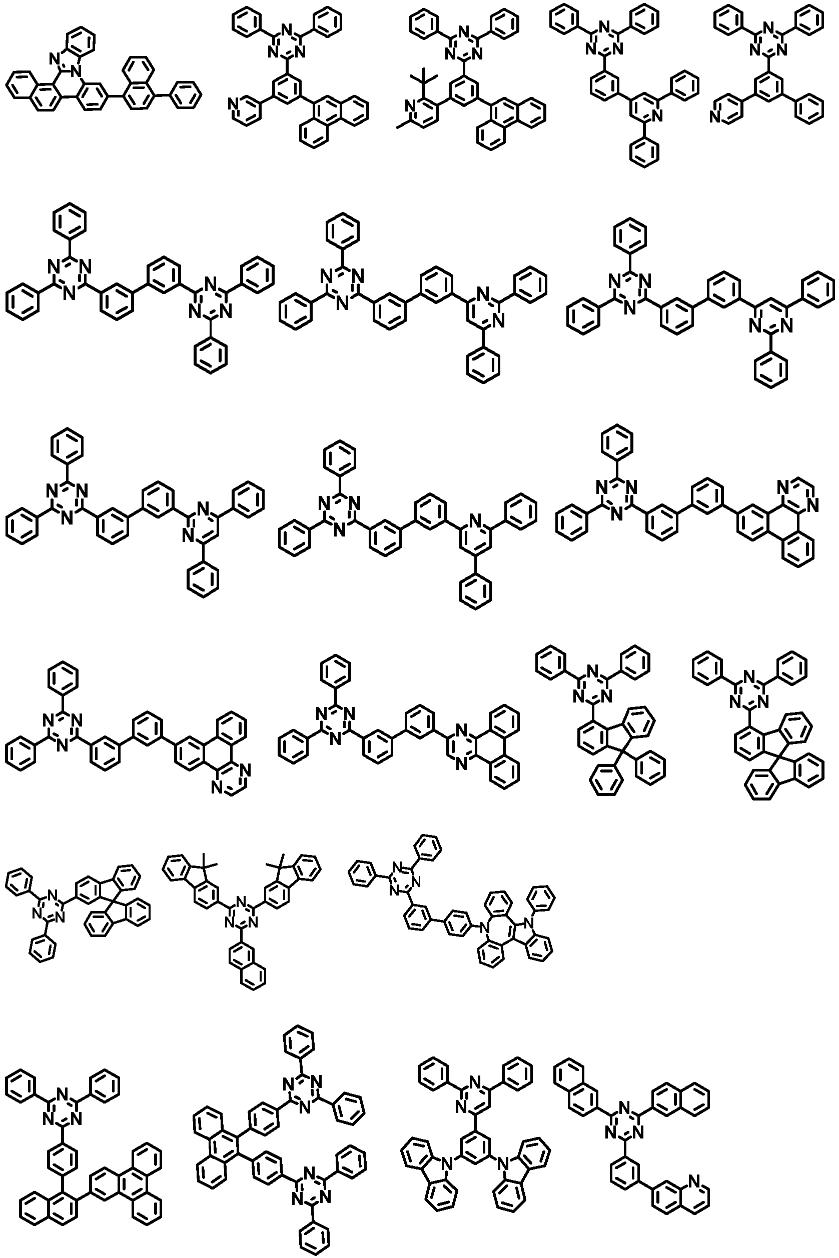

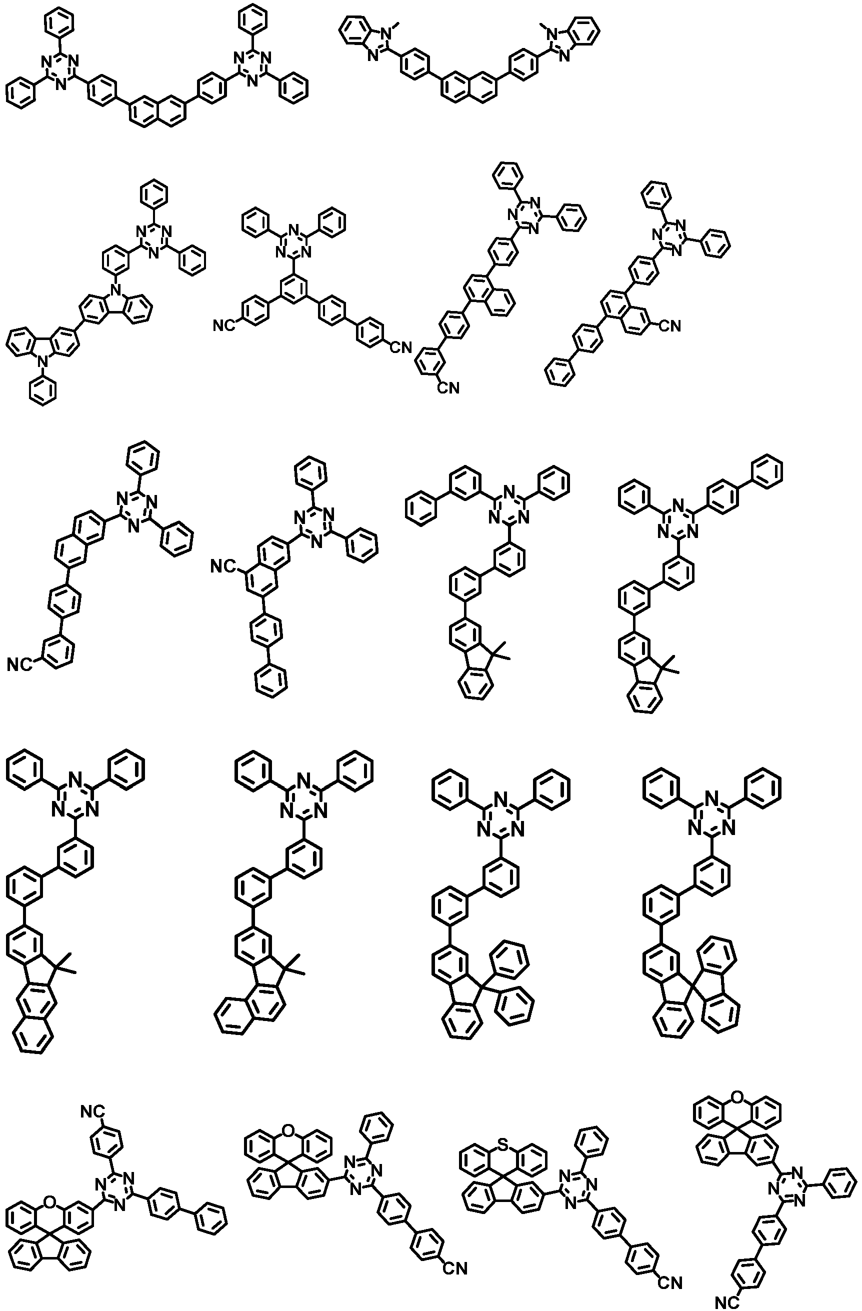

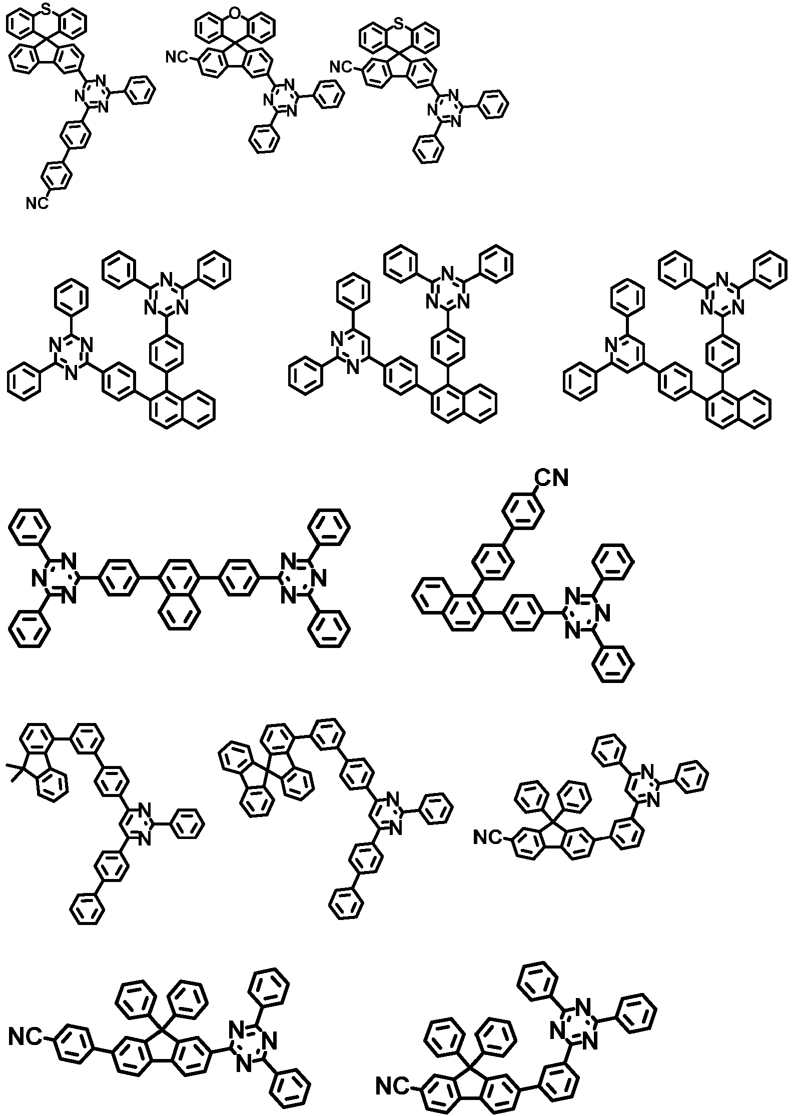

An embodiment may be an organic light emitting diode including the light emitting layer as the organic layer.

Another embodiment may be an organic light emitting diode including a light emitting layer and a hole transport region as the organic layer.

Another embodiment may be an organic light emitting diode including a light emitting layer and an electron transport region as the organic layer.

Another embodiment of the present invention may provide an organic light emitting diode including a hole transport region 140 and an electron transport region 150 in addition to the light emitting layer 130 as the organic layer 105, as shown in FIG. 1.

On the other hand, an organic light emitting diode may further include an electron injection layer (not shown), a hole injection layer (not shown), etc. in addition to the light emitting layer as the organic layer.

The organic light emitting diode 100 may be manufactured by forming an anode or a cathode on a substrate, and then forming an organic layer by a dry film method such as vacuum deposition, sputtering, plasma plating and ion plating, and forming a cathode or an anode thereon.

The organic light emitting diode may be applied to an organic light emitting display device.

### [Mode for Invention]

Hereinafter, the embodiments are illustrated in more detail with reference to examples. However, these examples are exemplary, and the scope of claims is not limited thereto.

Hereinafter, starting materials and reactants used in Examples and Synthesis Examples were purchased from Sigma-Aldrich Co. Ltd., TCI Inc., Tokyo chemical industry, or P&H tech as far as there in no particular comment or were synthesized by known methods.

### (Preparation of Compound for Organic Optoelectronic Device)

### Synthesis Example 1: Synthesis of Compound A-1

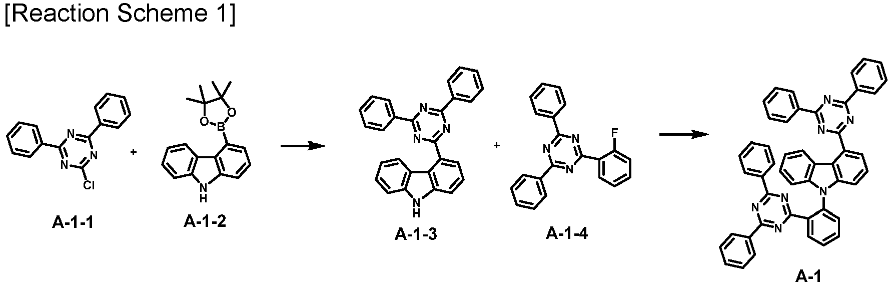

### 1st step: Synthesis of Compound A-1-3

23.4 g (87.3 mmol, A-1-1) of 1 equivalent of 2-chloro-4,6-diphenyl-1,3,5-triazine was added to 200 mL of toluene and 100 mL of distilled water, and 1 equivalent of 9H-carbazole-4-pinacol boron ester (A-1-2, cas: 1255309-13-7), 0.03 equivalent of Pd(PPh₃)₄, and 2 equivalents of K₂CO₃ were added thereto and then, stirred at 80 °C for 6 hours under a nitrogen atmosphere. After removing an aqueous layer, an organic layer therefrom was dried under a reduced pressure. A solid obtained therefrom was washed with water and hexane and recrystallized with 300 mL of toluene to obtain Compound A-1-3 with a yield of 60%.

### 2nd step: Synthesis of Compound A-1

12 g (30.1 mmol) of 1 equivalent of Compound A-1-3 was added to 100 mL of DMF, and 1 equivalent of Intermediate A-1-4 (cas: 1818371-42-4), and 3 equivalent of K₃PO₄ were added thereto and then, stirred at 140 °C for 6 hours under a nitrogen atmosphere. After drying an organic layer under a reduced pressure, a solid obtained therefrom was washed with water and hexane and recrystallized with 150 mL of toluene to obtain a white solid of Compound A-1 with a yield of 70%.

(LC/MS : theoretical value 705.26 g/mol, measured value : 706.37 g/mol)

### Synthesis Example 2: Synthesis of Compound A-15

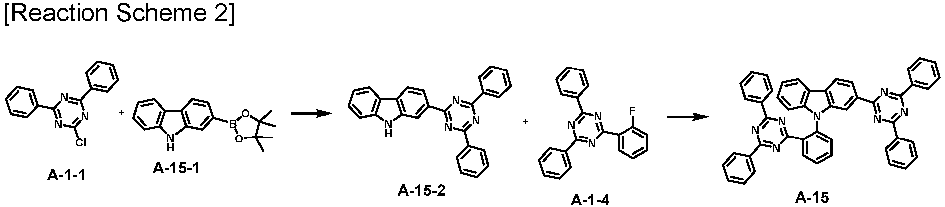

### 1st step: Synthesis of Compound A-15-2

23.4 g (87.3 mmol, A-1-1) of 1 equivalent of 2-chloro-4,6-diphenyl-1,3,5-triazine was added to 200 mL of toluene and 100 mL of distilled water, and 1 equivalent of 9H-carbazole-2-pinacol boron ester (A-15-1, cas: 1242412-60-7), 0.03 equivalents of Pd(PPh₃)₄, and 2 equivalents of K₂CO₃ were added thereto and then, stirred at 80 °C for 6 hours under a nitrogen atmosphere. After removing an aqueous layer, an organic layer therefrom was dried under a reduced pressure. A solid obtained therefrom was washed with water and hexane and recrystallized with 300 mL of toluene to obtain Intermediate A-15-2 with a yield of 60%.

### 2nd step: Synthesis of Compound A-15

Compound A-15 was synthesized at a yield of 75% in the same manner as in the 2^{nd} step of Synthesis Example 1 except that 1 equivalent of Compound A-15-2 and 1 equivalent of Compound A-1-4 were used.

### (LC/MS : theoretical value 705.26 g/mol, measured value : 706.35 g/mol)

### Synthesis Example 3: Synthesis of Compound A-27

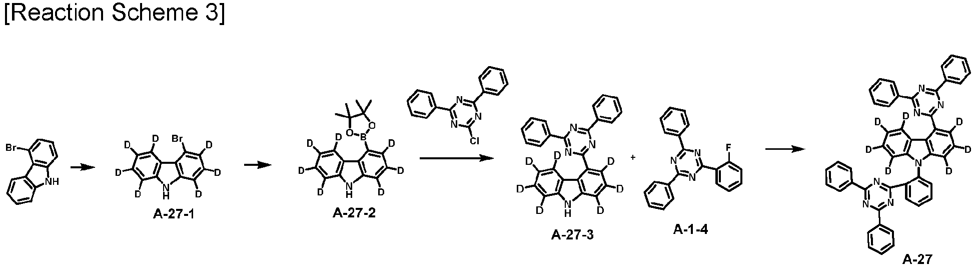

### 1st step: Synthesis of Compound A-27-1

4-bromo-9H-carbazole (16.4 g, 66.5 mmol), trifilc acid (29.9 g, 199.5 mmol), and benzene-D₆ (78.4 g, 931.0 mmol) were added to a round-bottomed flask under a nitrogen condition and then, stirred under reflux at 50 °C for 20 hours. When a reaction was completed, D₂O (10 ml) was slowly poured thereinto and then, quenched and sufficiently stirred. The resultant was neutralized by titration with a K₃PO₄ (aq) saturated solution. When a reaction was completed, after removing an aqueous layer with a separatory funnel, an organic solvent was removed therefrom under a reduced pressure to obtain a solid. The obtained solid was dissolved in toluene and treated with MgSO₄ to remove moisture, and after filtering an organic solvent by using a silica gel pad, a filtrate therefrom was vacuum-dried to obtain 14.2 g (84%) of Compound A-27-1.

### 2nd step: Synthesis of Compound A-27-2

14.2 g (56.1 mmol) of Compound A-27-1 was added to 200 mL of toluene in a 500 mL round-bottomed flask, and 0.05 equivalent of dichlorodiphenyl phosphinoferrocene palladium, 1.2 equivalent of bispinacolato diboron, and 2 equivalents of potassium acetate were added thereto and then, heated under reflux under a nitrogen atmosphere for 4 hours. After adding 200 mL of heated toluene (80 °C) to the reaction solution and filtering the mixture through silica gel, a filtrate therefrom was concentrated. The concentrated solid (A-27-2) was dried and then, used in the subsequent reaction without additional purification process.

### 3rd step: Synthesis of Compound A-27-3

Compound A-27-3 was synthesized with a yield of 60% in the same manner as in the 1st step of Synthesis Example 1 by using 1 equivalent of Compound A-27-2.

### 4th step: Synthesis of Compound A-27

Compound A-27 with a yield of 70% was synthesized in the same manner as in the 2^{nd} step of Synthesis Example 1 by using 1 equivalent of Compound A-27-3 and 1 equivalent of Compound A-1-4.

### (LC/MS : theoretical value 712.31 g/mol, measured value : 713.29 g/mol)

### Comparative Synthesis Example 1: Synthesis of Compound C1

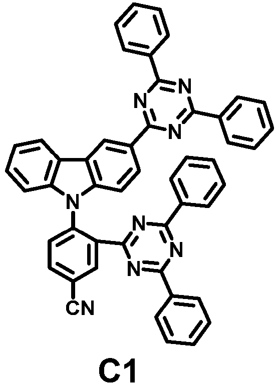

Compound C1 was synthesized with reference to patent EP3315581.

### Comparative Synthesis Example 2: Synthesis of Compound C2

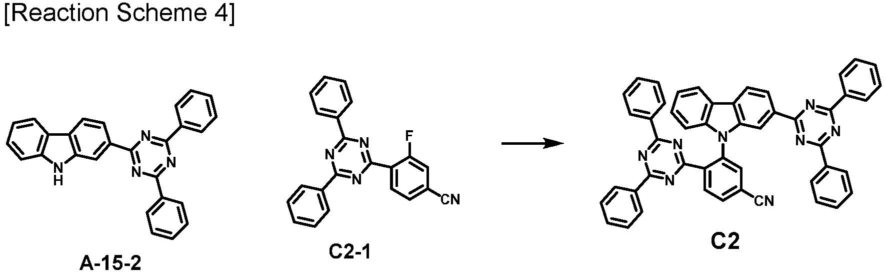

Compound C2 was synthesized by referring to the synthetic method of EP3315581 using Compound A-15-2 and Compound C2-1 (refer to EP3315581).

### Comparative Synthesis Example 3: Synthesis of Compound C3

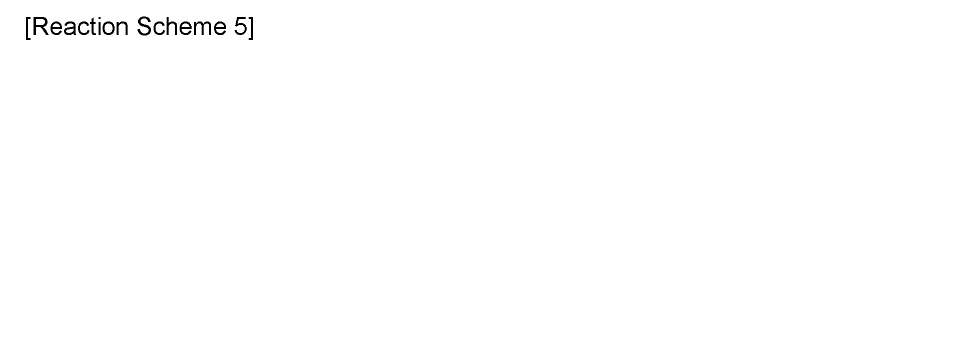

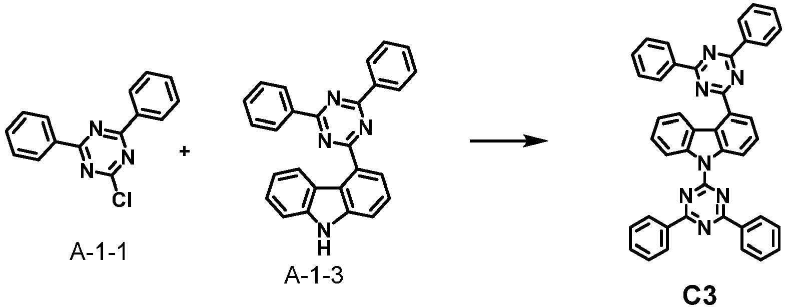

8 g (30 mmol) of 1 equivalent of Intermediate A-1-1, 12.0 g (30 mmol) of 1 equivalent of Compound A-1-3, 1.5 equivalent of NaOtBu, 0.03 equivalent of Pd₂(dba)₃, and 0.12 equivalent of P(t-Bu)₃ were added to 150 mL of xylene and then, stirred under reflux under a nitrogen flow for 12 hours. After removing the xylene, 200 mL of methanol was added to the obtained mixture to crystallize a solid, which was filtered and dissolved in MCB and then filtered, and Compound C3 with a yield of 60% was obtained by condensing an appropriate amount of the organic solvent.

(LC/MS theoretical value: 629.23 g/mol, measured value: M+1 = 630.32 g/mol)

### Comparative Synthesis Example 4: Synthesis of Compound C4

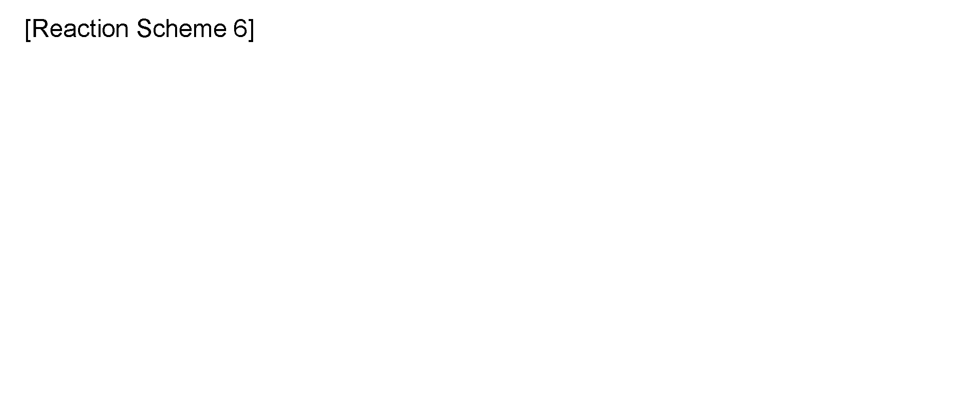

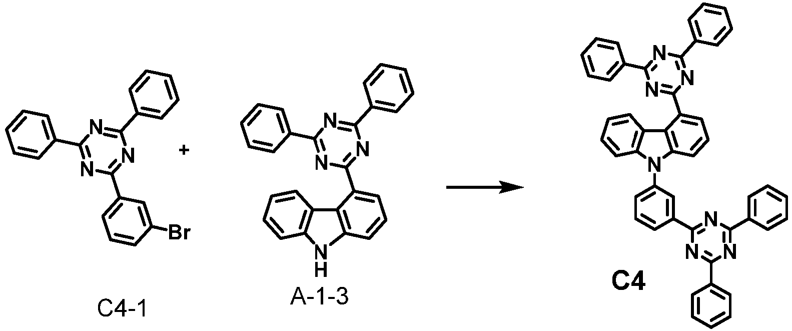

Compound C4 was synthesized with a yield of 80% in the same manner as in Comparative Synthesis Example 3 except that 1 equivalent of Intermediate C4-1 and 1 equivalent of Compound A-1-3 were used.

(LC/MS theoretical value: 705.26 g/mol, measured value: M+1 = 706.31 g/mol)

### Comparative Synthesis Example 5: Synthesis of Compound C5

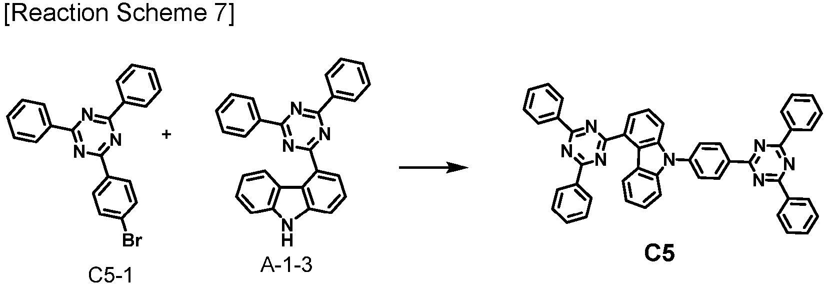

Compound C5 was synthesized with a yield of 75% in the same manner as in Comparative Synthesis Example 3 except that 1 equivalent of Intermediate C5-1 and 1 equivalent of Compound A-1-3 were used.

(LC/MS theoretical value: 705.26 g/mol, measured value: M+1 = 706.30 g/mol)

### (Preparation of Second Compound)

Compound B-99 was synthesized using the same method disclosed in US2017-0317293A1.

### Synthesis Example 4: Synthesis of Compound B-186

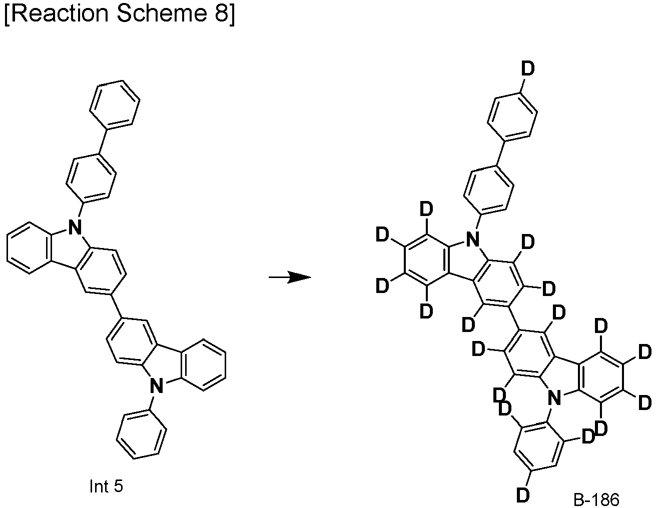

### 1st step: Synthesis of Compound Int 5

Compound Int 1 was synthesized with reference to the method disclosed in Korean Publication No. 2016-0049842.

### 2nd step: Synthesis of Compound B-186

30 g (0.0535 mol) of Compound Int 5, 40 g (0.267 mol) of trifluoromethanesulfonic acid, and 282 g (3.35 mol) of D₆-benzene were added thereto and then, stirred at 10 °C for 24 hours. After adding purified water thereto, the mixture was neutralized with a saturated K₃PO₄ solution. An organic layer therefrom was concentrated and column-purified, obtaining 18 g of Compound B-186 (a white solid, LC-Mass Mz 578.79, C₄₂H₁₀D₁₈N₂).

### Synthesis Example 5: Synthesis of Compound C-5

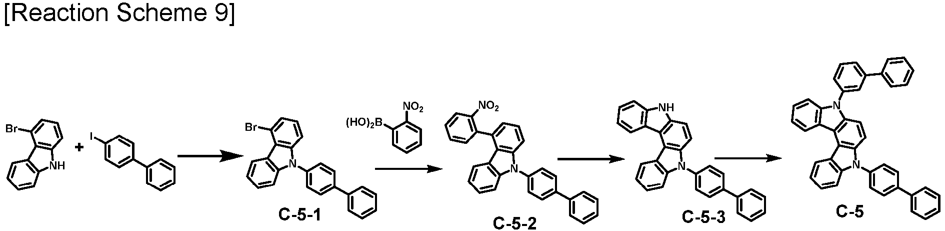

### 1st step: Synthesis of Intermediate C-5-1

In a round-bottomed flask, 10.44 g (42.41 mmol) of 4-bromo-9H-carbazole, 11.88 g (42.41 mmol) of 4-iodo-1,1'-biphenyl (Aldrich Sigma Co., Ltd.), 0.388 g (0.424 mmol) of Pd₂(dba)₃, 0.206 g (0.848 mmol) of P(t-Bu)₃, and 6.11 g (63.61 mmol) of NaO(t-Bu) were suspended in 420 ml of toluene and then, stirred at 60 °C for 12 hours. When a reaction was completed, distilled water was added thereto and then, stirred for 30 minutes for extraction, and an organic layer therefrom alone was columned through silica gel (hexane/dichloromethane = 9: 1 (v/v)) to obtain 14.70 g (a yield: 87%) of Intermediate C-5-1.

### 2nd step: Synthesis of Intermediate C-5-2

In a round-bottomed flask, 15.50 g (38.92 mmol) of Intermediate C-5-1, 7.15 g (42.81 mmol) of (2-nitrophenyl)-boronic acid, and 16.14 g (116.75 mmol) of potassium carbonate, and 1.35 g (1.17 mmmol) of tetrakis-(triphenylphosphine)palladium (0) (Pd(PPh₃)₄) were suspended in 150 ml of toluene and 70 ml of distilled water and then, stirred under reflux for 12 hours. Subsequently, an organic layer was extracted therefrom with dichloromethane and distilled water and then, silica gel-filtered. After removing the organic solution, a product solid therefrom was recrystallized with dichloromethane and n-hexane to obtain 13.72 g (a yield: 80%) of Intermediate C-5-2.

### 3rd step: Synthesis of Intermediate C-5-3

22.46 g (51.00 mmol) of Intermediate C-5-2 and 52.8 ml of triethyl phosphite were added to a round-bottomed flask and after substituting with nitrogen, stirred at 160 °C for 12 hours. When a reaction was completed, 3 L of MeOH was added thereto and then, stirred and filtered, and a filtrate therefrom was volatilized. 10.42 g (a yield of 50%) of Intermediate C-5-3 was obtained by purifying through column chromatography.

### 4th step: Synthesis of Compound C-5

Compound C-5 (a yield: 60%) was synthesized in the same manner as in the 1st step of Synthesis Example 5 except that Intermediate C-5-3 and 1-iodo-3-phenylbenzene were used.

(LC/MS : theoretical value 560.23 g/mol, measured value : 561.57 g/mol)

### (Manufacturing of Organic Light Emitting Diode)

### Example 1

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. This prepared ITO transparent electrode was used as an anode, Compound A doped with 3% NDP-9 (Novaled GmbH) was vacuum-deposited on the ITO substrate to form a 100 Å-thick hole injection layer, and Compound A was deposited on the hole injection layer to a thickness of 1350 Å to form a hole transport layer. Compound B was deposited on the hole transport layer to a thickness of 350 Å to form a hole transport auxiliary layer. On the hole transport auxiliary layer, Compound A-1 obtained in Synthesis Example 1 was used as a host, and 7 wt% of PhGD was doped as a dopant to form a 400 Å-thick light emitting layer by vacuum deposition. Then, Compound C was deposited on the light emitting layer to a thickness of 50 Å to form an electron transport auxiliary layer, and Compound D and LiQ were simultaneously vacuum deposited at a weight ratio of 1:1 to form an electron transport layer to a thickness of 300 Å. An organic light emitting diode was manufactured by sequentially vacuum-depositing 15 Å of LiQ and 1200 Å of Al on the electron transport layer to form a cathode.

ITO / Compound A (3% NDP-9 doping, 100 Å)/ Compound A (1350 Å) / Compound B (350 Å) / EML [93 wt% of host (Compound A-1) : 7 wt% of PhGD] (400 Å)/Compound C (50 Å) / Compound D : LiQ (300 Å) / LiQ (15 Å) / Al (1200 Å).
Compound A: N-(biphenyl-4-yl)-9,9-dimethyl-N-(4-(9-phenyl-9H-carbazol-3-yl)phenyl)-9H-fluoren-2-amine
Compound B: N-[4-(4-dibenzofuranyl)phenyl]-N-[4-(9-phenyl-9H-fluoren-9-yl)phenyl][1,1'-biphenyl]-4-amine
Compound C: 2,4-diphenyl-6-(4',5',6'-triphenyl[1,1':2',1":3",1‴:3‴,1ʺʺ-quinquephenyl]-3""-yl)-1,3,5-triazine
Compound D: 2-(1,1'-biphenyl-4-yl)-4-(9,9-diphenylfluoren-4-yl)-6-phenyl-1,3,5-triazine [PhGD]

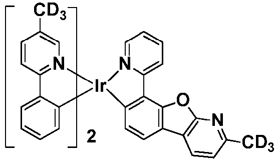

### Example 2

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. This prepared ITO transparent electrode was used as an anode, Compound A doped with 3% NDP-9 (Novaled GmbH) was vacuum-deposited on the ITO substrate to form a 100 Å-thick hole injection layer, and Compound A was deposited on the hole injection layer to a thickness of 1350 Å to form a hole transport layer. Compound E was deposited on the hole transport layer to a thickness of 350 Å to form a hole transport auxiliary layer. On the hole transport auxiliary layer, Compound A-1 obtained in Synthesis Example 1 and Compound B-186 obtained in Synthesis Example 4 were simultaneously used as hosts, and PhGD was doped at 10 wt% as a dopant to form a 330 Å thick light emitting layer by vacuum deposition. Subsequently, Compound F was deposited on the light emitting layer to a thickness of 50 Å to form an electron transport auxiliary layer, and Compound G and LiQ were simultaneously vacuum deposited at a weight ratio of 1:1 to form an electron transport layer to a thickness of 300 Å. An organic light emitting diode was manufactured by sequentially vacuum-depositing 15 Å of LiQ and 1200 Å of Al on the electron transport layer to form a cathode.

ITO/Compound A (3% NDP-9 doping, 100 Å)/ Compound A (1350 Å)/ Compound E (350 Å)/EML [90 wt% of host (Compound A-1: Compound B-186 = 4:6 w/w): 10 wt% of PhGD] (330 Å)/ Compound F (50 Å)/ Compound G:LiQ (300 Å)/LiQ (15 Å)/Al (1200 Å).
Compound A: N-(biphenyl-4-yl)-9,9-dimethyl-N-(4-(9-phenyl-9H-carbazol-3-yl)phenyl)-9H-fluoren-2-amine
Compound E: N,N-bis(9,9-dimethyl-9H-fluoren-4-yl)-9,9-spirobi(fluorene)-2-amine
Compound F: 2-[3'-(9,9-dimethyl-9H-fluoren-2-yl)[1,1'-biphenyl]-3-yl]-4,6-diphenyl-1,3,5-triazine
Compound G: 2-[4-[4-(4'-cyano-1,1'-biphenyl-4-yl)-1-naphthyl]phenyl]-4,6-diphenyl-1,3,5-triazine

### Examples 3 to 8 and Comparative Examples 1 to 11

Diodes of Examples 3 to 8 and Comparative Examples 1 to 11 were manufactured in the same manner as in Example 1 or 2, except that the host and composition were changed as described in Tables 1 and 2.

### Evaluation

### (1) Measurement of Current Density Change Depending on Voltage Change

The obtained organic light emitting diodes were measured regarding a current value flowing in the unit device, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltage of the organic light emitting diodes was increased from 0v to 10V.

### (3) Measurement of Luminous Efficiency

Luminous efficiency (cd/A) at the same current density (10 mA/cm²) were calculated by using the luminance and current density from (1) and (2) above.

The relative values based on the luminous efficiency of Comparative Example 1 are shown in Table 1.

The relative values based on the luminous efficiency of Comparative Example 6 are shown in Table 2.

### (4) Measurement of Life-span

T95 life-spans of the organic light emitting diodes were measured were measured as a time when their luminance decreased down to 95% relative to the initial luminance after emitting light with 24,000 cd/m² as the initial luminance (cd/m²) and measuring their luminance decreases depending on a time with a Polanonix life-span measurement system.

The relative values based on the T95 life-span of Comparative Example 1 are shown in Table 1.

The relative values based on the T95 life-span of Comparative Example 6 are shown in Table 2.

**(Table 1)**

| | Host | Luminous efficiency (%) | Life-span T95 (%) |
|---|---|---|---|
| Example 1 | A-1 | 130% | 200% |
| Example 2 | A-15 | 128% | 180% |
| Example 3 | A-27 | 131% | 260% |
| Comparative Example 1 | C1 | 100% | 100% |
| Comparative Example 2 | C2 | 90% | 110% |
| Comparative Example 3 | C3 | 110% | 120% |
| Comparative Example 4 | C4 | 108% | 105% |
| Comparative Example 5 | C5 | 105% | 103% |

**(Table 2)**

| | First host | Second host | Luminous efficiency (%) | Life-span T95 (%) |
|---|---|---|---|---|
| Example 4 | A-1 | B-186 | 120% | 120% |
| Example 5 | A-15 | B-186 | 121% | 115% |
| Example 6 | A-27 | B-186 | 120% | 130% |
| Example 7 | A-1 | C-5 | 115% | 140% |
| Example 8 | A-27 | C-5 | 115% | 155% |
| Comparative Example 6 | C1 | B-186 | 100% | 100% |
| Comparative Example 7 | C1 | C-5 | 85% | 110% |
| Comparative Example 8 | C2 | B-186 | 90% | 105% |
| Comparative Example 9 | C3 | B-186 | 110% | 110% |
| Comparative Example 10 | C4 | B-186 | 107% | 105% |
| Comparative Example 11 | C5 | B-186 | 102% | 103% |

Referring to Table 1, the organic light emitting diodes to which the compounds according to Examples of the present invention are applied have significantly improved efficiency and life-span characteristic compared to the organic light emitting diode according to Comparative Example, and
in particular, referring to Table 2, the efficiency and life-span characteristics of organic light emitting diodes to which compositions including compounds according to Examples of the present invention are applied are also improved.

While this invention has been described in connection with what is presently considered to be practical example embodiments, it is to be understood that the invention is not limited to the disclosed embodiments, but, on the contrary, is intended to cover various modifications and equivalent arrangements included within the spirit and scope of the appended claims.

## Claims

1. A compound for an organic optoelectronic device represented by Chemical Formula 1: 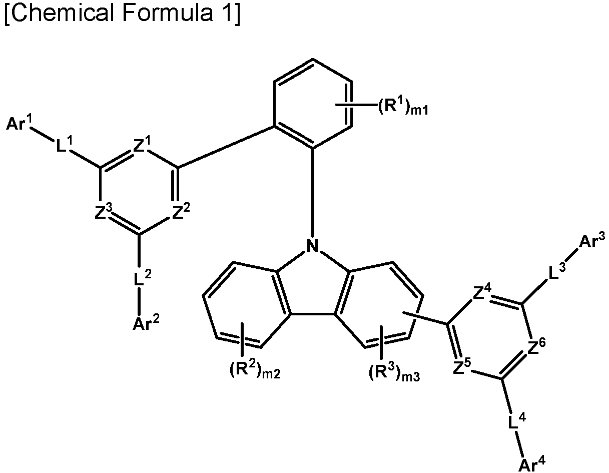
wherein, in Chemical Formula 1,
R¹ is hydrogen, deuterium, a substituted or unsubstituted C1 to C10 alkyl group, a substituted or unsubstituted C6 to C20 aryl group, or a substituted or unsubstituted C6 to C20 heterocyclic group,
Z¹ to Z³ are each independently N or CR^{a},
at least two of Z¹ to Z³ are N,
Z⁴ to Z⁶ are each independently N or CR^{b},
at least two of Z⁴ to Z⁶ are N,
R², R^{b}, R², and R³ are each independently hydrogen, deuterium, a cyano group, a halogen, a substituted or unsubstituted C1 to C30 alkyl group, a substituted or unsubstituted C6 to C30 aryl group, or a substituted or unsubstituted C2 to C30 heterocyclic group,
L¹ to L⁴ are each independently a single bond, a substituted or unsubstituted C6 to C20 arylene group, or a substituted or unsubstituted C2 to C20 heteroarylene group,
Ar¹ to Ar⁴ are each independently a substituted or unsubstituted C6 to C20 aryl group or a substituted or unsubstituted C2 to C30 heterocyclic group,
m1 and m2 are each independently one of integers of 1 to 4, and
m3 is one of integers of 1 to 3.

2. The compound for the organic optoelectronic device as claimed in claim 1, wherein
Chemical Formula 1 is represented by any one of Chemical Formula 1A to Chemical Formula 1D: 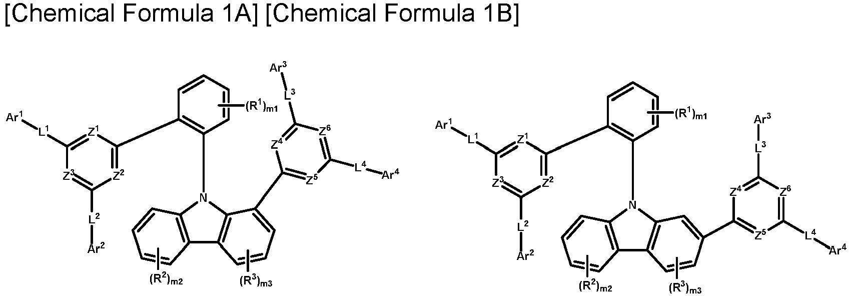 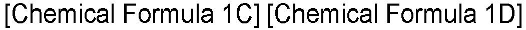 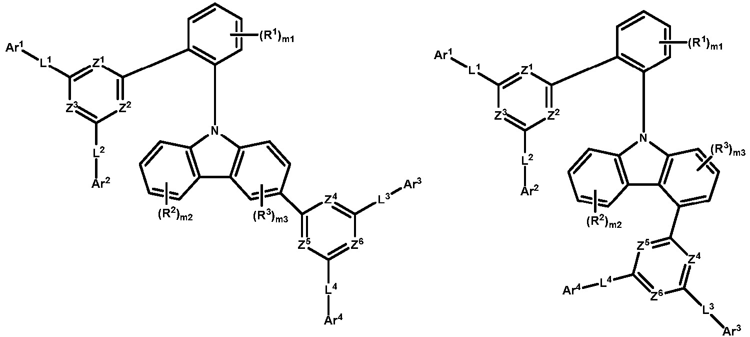
wherein, in Chemical Formula 1A to Chemical Formula 1D,
Z¹ to Z⁶, R¹ to R³, L¹ to L⁴, Ar¹ to Ar⁴, and m1 to m3 are the same as defined in claim 1.

3. The compound for the organic optoelectronic device as claimed in claim 1, wherein
Chemical Formula 1 is represented by any one of Chemical Formula 1-1 to Chemical Formula 1-9: 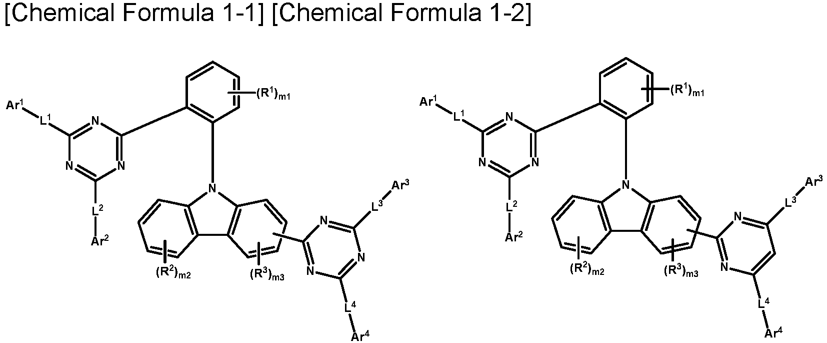 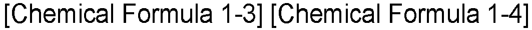 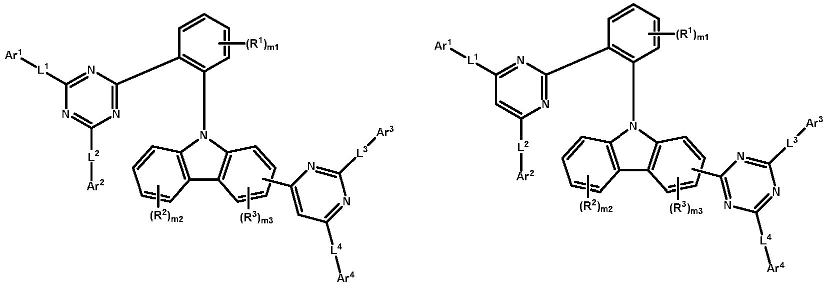 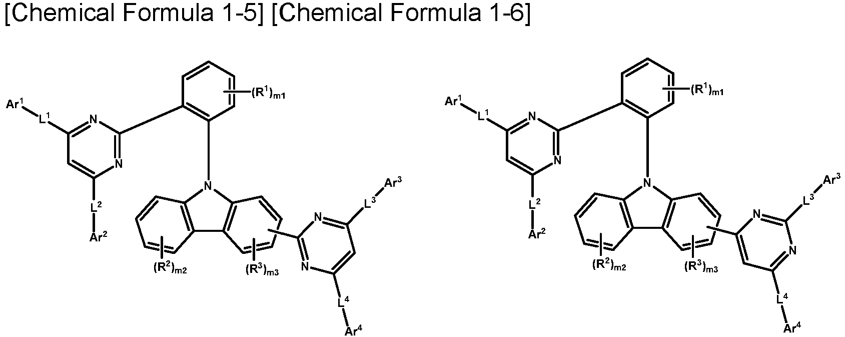 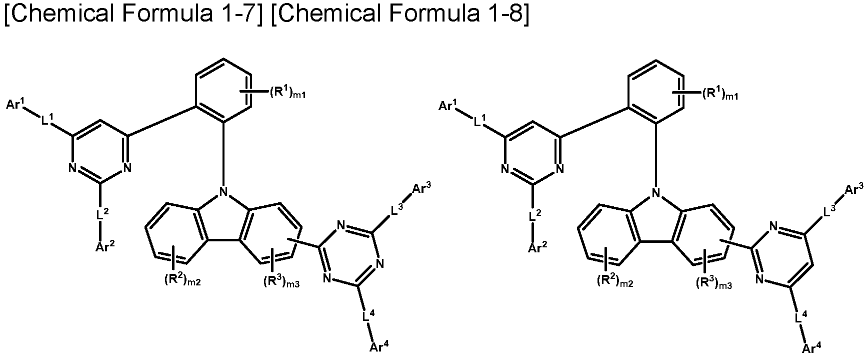 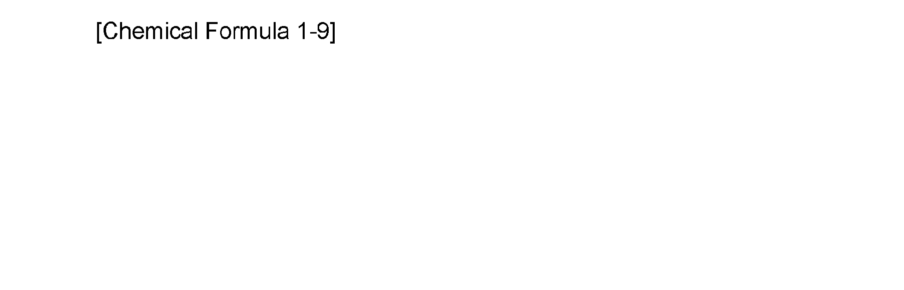 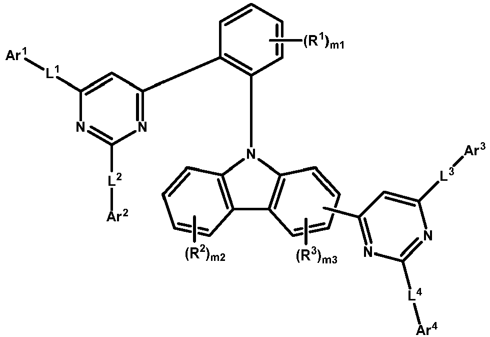
wherein, in Chemical Formula 1-1 to Chemical Formula 1-9,
R¹ to R³, L¹ to L⁴, Ar¹ to Ar⁴, and m1 to m3 are the same as defined in claim 1.

4. The compound for the organic optoelectronic device as claimed in claim 1, wherein
Chemical Formula 1 is represented by Chemical Formula 1-B-1 or Chemical Formula 1-D-1: 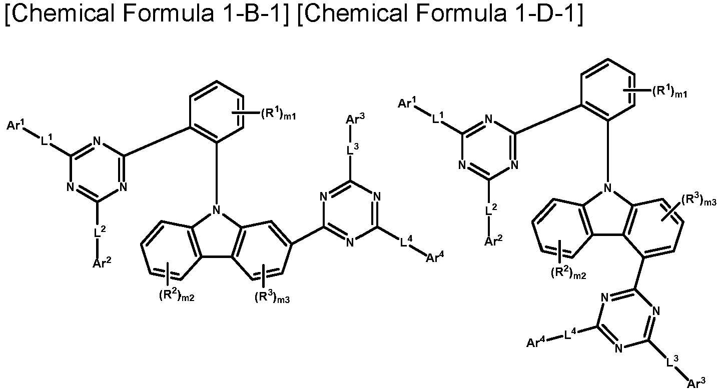
wherein, in Chemical Formula 1-B-1 and Chemical Formula 1-D-1,
R¹ to R³, L¹ to L⁴, Ar¹ to Ar⁴, and m1 to m3 are the same as defined in claim 1.

5. The compound for the organic optoelectronic device as claimed in claim 1, wherein
Ar¹ to Ar⁴ are each independently a substituted or unsubstituted phenyl group, a substituted or unsubstituted biphenyl group, a substituted or unsubstituted fluorenyl group, a substituted or unsubstituted dibenzofuranyl group, or a substituted or unsubstituted dibenzothiophenyl group.

6. A compound for an organic optoelectronic device selected from the compounds listed in Group 1:

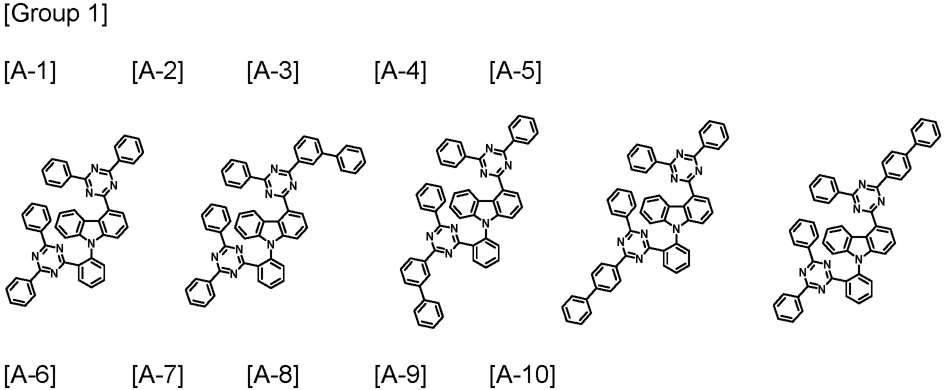

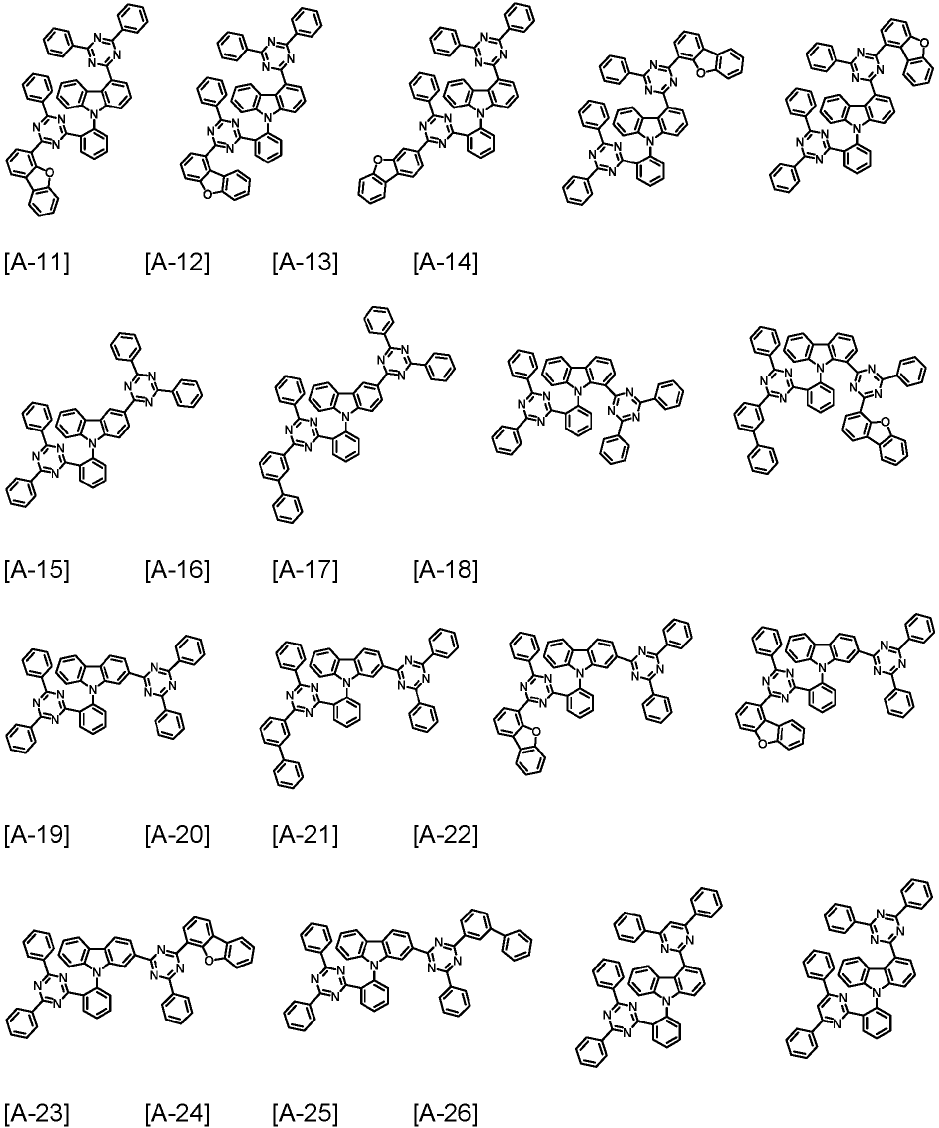

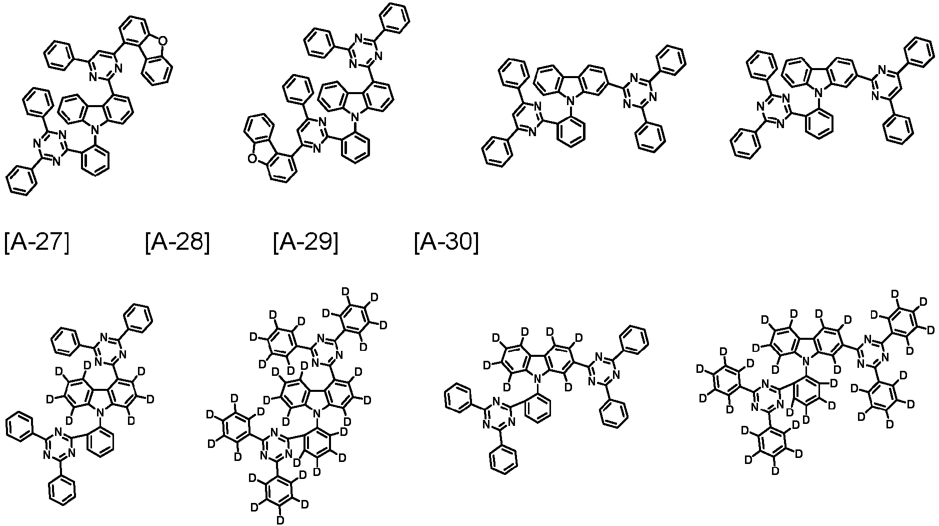

7. A composition for an organic optoelectronic device, comprising
a first compound and a second compound,
wherein the first compound is the compound for an organic optoelectronic device as claimed in claim 1, and
the second compound is represented by Chemical Formula 2; a combination of Chemical Formula 3 and Chemical Formula 4; or Chemical Formula 5: 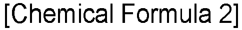 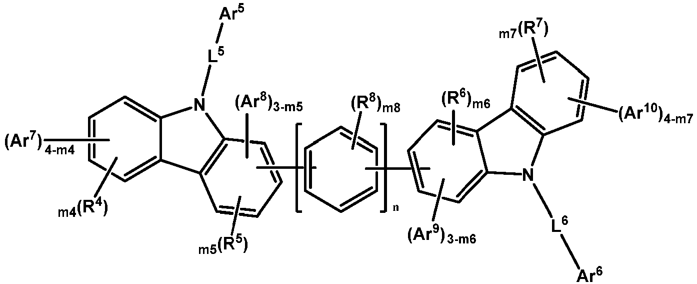
wherein, in Chemical Formula 2,
R⁴ to R⁸ and Ar⁷ to Ar¹⁰ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group,
L⁵ and L⁶ are each independently a single bond or a substituted or unsubstituted C6 to C20 arylene group,
m4, m7, and m8 are each independently one of integers of 1 to 4,
m5 and m6 are each independently one of integers of 1 to 3, and
n is one of integers of 0 to 2; 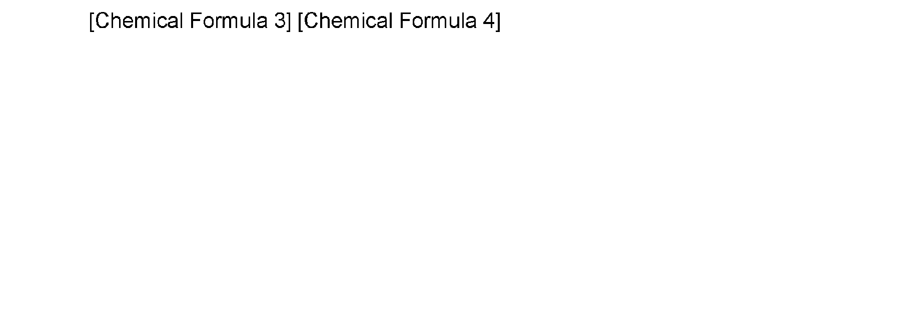 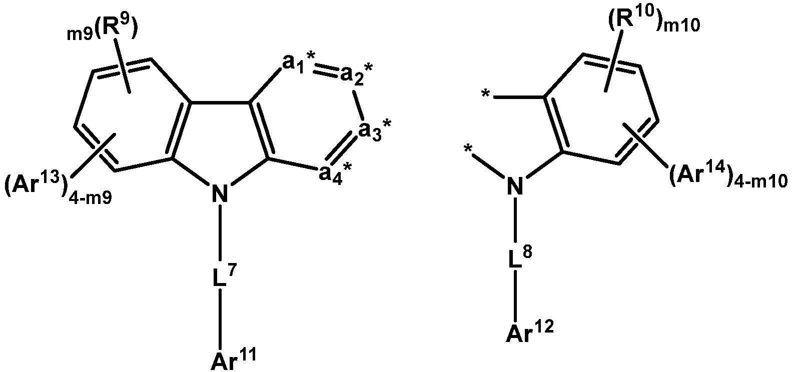
wherein, in Chemical Formula 3 and Chemical Formula 4,
a₁* to a₄* in Chemical Formula 3 are each independently a linking carbon (C) or C-L^{a}-R^{c},
among a₁* to a₄* in Chemical Formula 3, two adjacent ones are each linked to * in Chemical Formula 4,
L^{a}, L⁷, and L⁸ are each independently a single bond or a substituted or unsubstituted C6 to C20 arylene group,
R^{c}, Ar¹³, Ar¹⁴, R⁹, and R¹⁰ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar¹¹ and Ar¹² are each independently a substituted or unsubstituted C6 to C20 aryl group or a substituted or unsubstituted C2 to C30 heterocyclic group, and
m9 and m10 are each independently one of integers of 1 to 4; 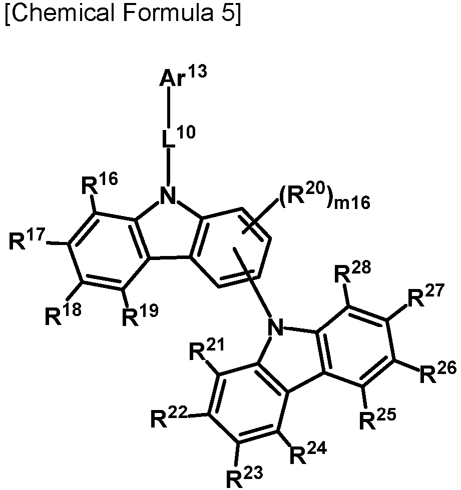
wherein, in Chemical Formula 5,
L¹⁰s are each independently a single bond or a substituted or unsubstituted C6 to C20 arylene group,
R¹⁶ to R²⁸ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar¹³ is a substituted or unsubstituted C6 to C20 aryl group or a substituted or unsubstituted C2 to C30 heterocyclic group, and
m16 is one of integers of 1 to 3.

8. The composition for the organic optoelectronic device as claimed in claim 7, wherein
Chemical Formula 2 is represented by Chemical Formula 2-8: 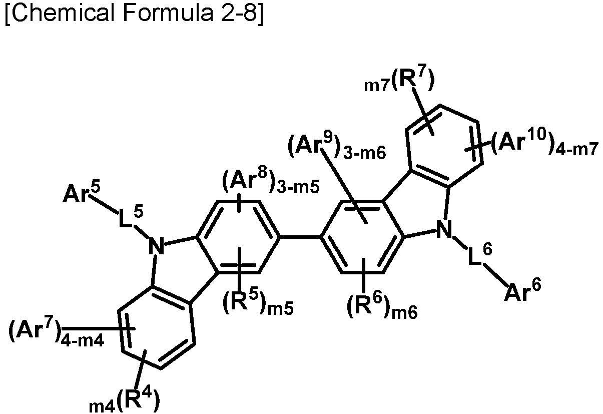
wherein, in Chemical Formula 2-8,
Ar⁷ to Ar¹⁰ and R⁴ to R⁷ are each independently hydrogen, deuterium or a substituted or unsubstituted C6 to C12 aryl group,
m4 and m7 are each independently one of integers of 1 to 4,
m5 and m6 are each independently one of integers of 1 to 3, and
*-L⁵-Ar⁵ and *-L⁶-Ar⁶ are each independently one of substituents listed in Group I , 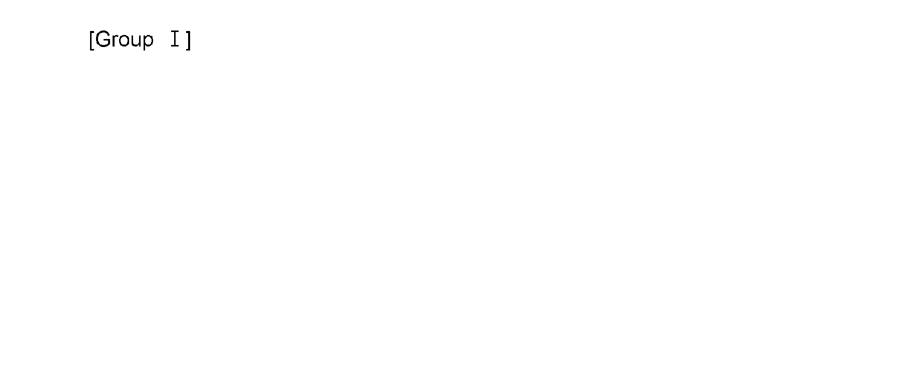 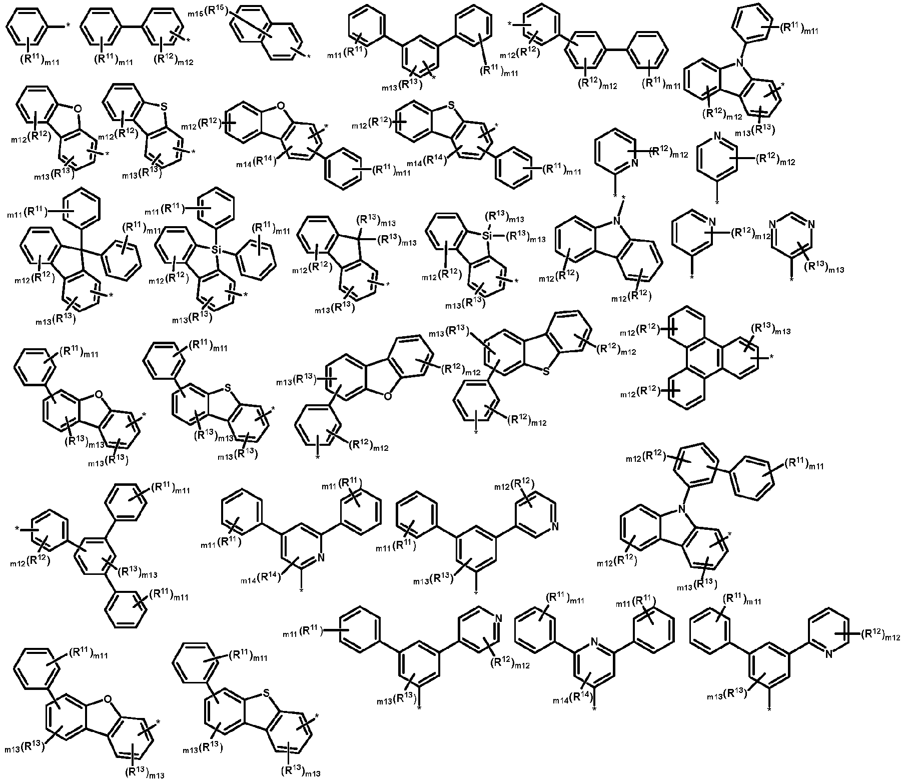
wherein, in Group I,
R¹¹ to R¹⁵ are each independently hydrogen, deuterium, a cyano group, a C1 to C10 alkyl group, or a C6 to C12 aryl group,
m11 is one of integers of 1 to 5,
m12 is one of integers of 1 to 4,
m13 is one of integers of 1 to 3,
m14 is an integer of 1 or 2,
m15 is one of integers of 1 to 7, and
* is a linking point.

9. The composition for the organic optoelectronic device as claimed in claim 7, wherein
the combination of Chemical Formula 3 and Chemical Formula 4 is represented by Chemical Formula 3C: 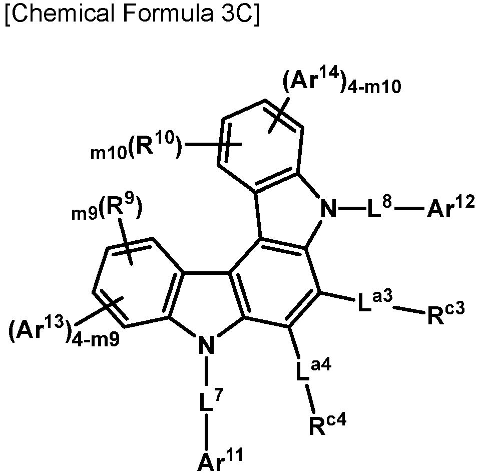
wherein, in Chemical Formula 3C,
L^{c3} and L^{c4} are a single bond,
Ar¹³, Ar¹⁴, R⁹, R¹⁰, R^{c3}, and R^{c4} are each independently hydrogen, deuterium or a C6 to C12 aryl group,
m9 and m10 are each independently one of integers of 1 to 4, and
*-L⁷-Ar¹¹ and *-L⁸-Ar¹² are each independently one of substituents listed in Group I , 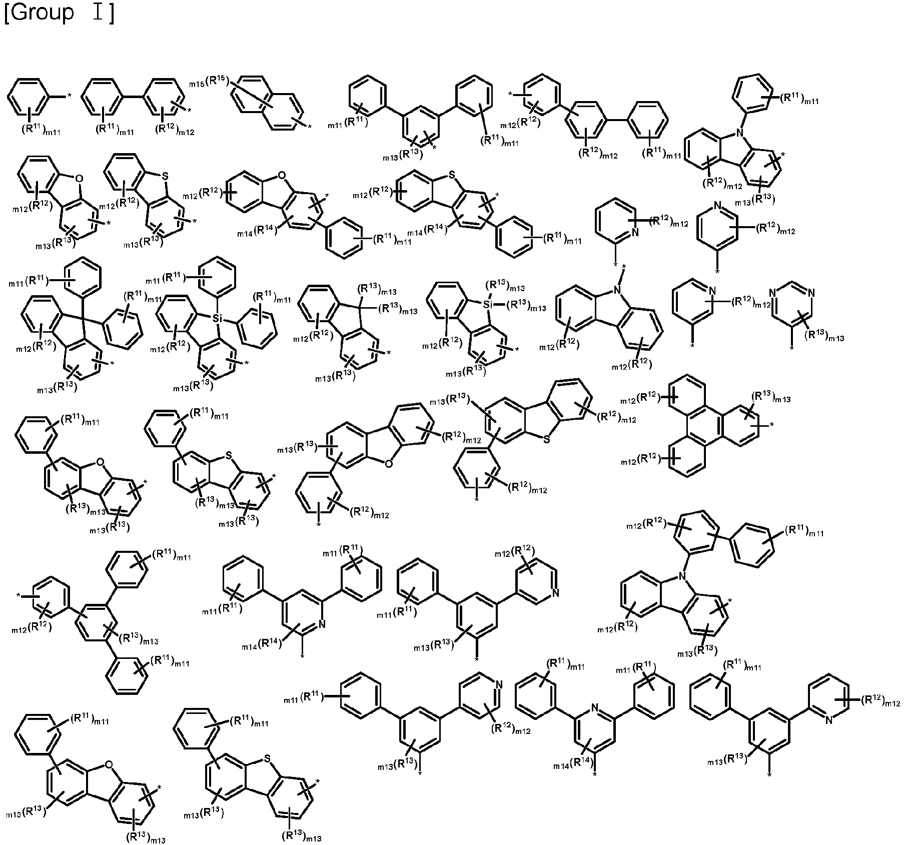
wherein, in Group I ,
R¹¹ to R¹⁵ are each independently hydrogen, deuterium, a cyano group, a C1 to C10 alkyl group, or a C6 to C12 aryl group,
m11 is one of integers of 1 to 5,
m12 is one of integers of 1 to 4,
m13 is one of integers of 1 to 3,
m14 is an integer of 1 or 2,
m15 is one of integers of 1 to 7, and
* is a linking point.

10. The composition for the organic optoelectronic device as claimed in claim 7, wherein
Chemical Formula 5 is represented by Chemical Formula 5-2 or Chemical Formula 5-3: 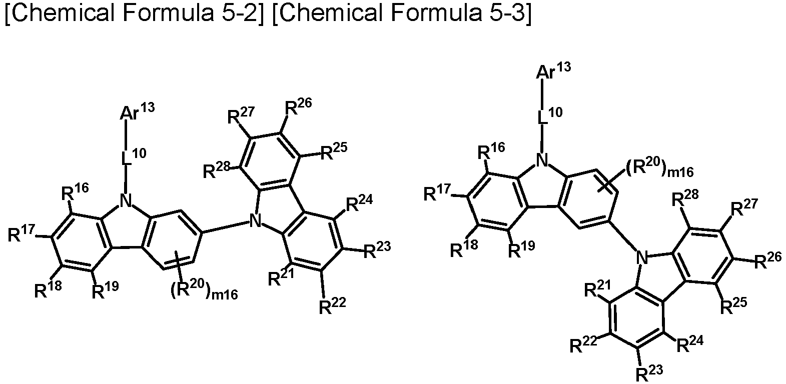
wherein, in Chemical Formula 5-2 and Chemical Formula 5-3,
L¹⁰, R¹⁶ to R²⁸, Ar¹³ and m16 are the same as defined in claim 7.

11. An organic optoelectronic device, comprising
an anode and a cathode facing each other, and
at least one organic layer between the anode and the cathode,
wherein the organic layer includes
the compound for an organic optoelectronic device as claimed in any one of claim 1 to claim 6; or
the composition for an organic optoelectronic device as claimed in any one of claim 7 to claim 10.

12. The organic optoelectronic device as claimed in claim 11, wherein
the organic layer includes a light emitting layer, and
the light emitting layer includes the compound for an organic optoelectronic device or the composition for an organic optoelectronic device.

13. A display device comprising the organic optoelectronic device as claimed in claim 11.
